# EUROPÄISCHE PATENTANMELDUNG

(11) **EP 4 822 351 A2**
(43) Veröffentlichungstag der Anmeldung: **14.10.2026**
(21) Anmeldenummer: 26171400.0
(22) Anmeldetag: 09.04.2026
(51) Int. Cl.: G01N 33/18, C02F 3/00, G01N 27/406, G01N 27/04, G01N 27/22

(54) **MESSTECHNISCHE VORRICHTUNG MIT MESSTECHNISCH WIRKSAMER KERAMIK SOWIE MESSTECHNISCHES SYSTEM ZUR MESSDATENAUSWERTUNG SOWIE VERFAHREN ZUM GENERIEREN VON ENERGETISCHEN/ELEKTROCHEMISCHEN PARAMETERN AUS MESSDATEN WENIGSTENS EINER MIKROBIELLEN ELEKTROCHEMISCHEN TECHNOLOGIEEINHEIT SOWIE COMPUTERPROGRAMMPRODUKT UND VERWENDUNG**

(30) Priorität: 11.04.2025 DE 102025114467; 11.04.2025 DE 202025102025 U
(71) Anmelder: Okeanos Smart Sensor Solutions GmbH, 44787 Bochum NRW (DE)
(72) Erfinder: Littfinski, Tobias, 44892 Bochum (DE); Oppel, Henning, 44892 Bochum (DE); Mewes, Benjamin, 44795 Bochum (DE); Grimmeisen, Felix, 84405 Dorfen (DE); Neumann, Philipp, 47647 Kerken (DE)
(74) Vertreter: Tanner, Lukas

(57) **Zusammenfassung**

Die Erfindung betrifft eine Messtechnische Vorrichtung (10) zum Generieren von wenigstens einem Parameter aus Messdaten basierend auf bioelektrochemischen Prozessen, aufweisend wenigstens eine messtechnische mikrobielle elektrochemische Technologieeinheit (11) mit einem kathodischen Bereich (11.1) abgeschottet von zu untersuchendem Fluid, und aufweisend eine Logikeinheit (16) mit wenigstens einem Mikrocontroller (16.1) zum Verarbeiten von über einen Auswertungszeitraum erhobenen Messdaten, wobei die messtechnische Vorrichtung (10) eingerichtet ist zum Korrelieren wenigstens eines Messwerts mit wenigstens einem Prozessparameter (P_{SB}) basierend auf den verarbeiteten Messdaten; wobei die messtechnische Vorrichtung wenigstens eine zwischen kathodischem Bereich (11.1) und zu untersuchendem Fluid angeordnete Keramik als Bestandteil der messtechnischen mikrobiellen elektrochemischen Technologieeinheit (11) aufweist, welche den kathodischen Bereich vom zu untersuchenden Fluid separiert, wobei die messtechnische Vorrichtung (10) mittels der Keramik eingerichtet ist zum Generieren des wenigstens einen Parameters aus über einen Auswertungszeitraum von mindestens 30 Tagen erhobenen Messdaten, insbesondere Messdaten aus zellinternen Widerstandskomponenten und/oder Spannungs-, Kapazitäts-, Strom-, Leistungs-, und/oder elektrischen Ladungsmessdaten.

## Beschreibung

### TECHNISCHES GEBIET

Die vorliegende Erfindung betrifft eine messtechnische Vorrichtung zum Generieren von wenigstens einem energetischen/elektrochemischen (Zell-)Parameter aus Messdaten (bzw. elektrischen Signalen) basierend auf bioelektrochemischen Prozessen, aufweisend wenigstens eine messtechnische mikrobielle elektrochemische Technologieeinheit mit einem kathodischen Bereich abgeschottet bzw. separiert von zu untersuchendem Fluid, und aufweisend eine Logikeinheit mit wenigstens einem Mikrocontroller eingerichtet zum Verarbeiten von über einen Auswertungszeitraum erhobenen Messdaten, wobei die messtechnische Vorrichtung eingerichtet ist zum Korrelieren wenigstens eines Messwerts mit wenigstens einem Prozessparameter basierend auf den verarbeiteten Messdaten, insbesondere mit einem Prozessparameter für den chemischen/biologischen Sauerstoffbedarf eines zu optimierenden Prozesses. Ferner betrifft die vorliegende Erfindung ein messtechnisches System zur Messdatenauswertung umfassend wenigstens eine solche messtechnische Vorrichtung, insbesondere im Zusammenhang mit Wasseraufbereitungsprozessen. Dabei steht die vorliegende Erfindung auch im Zusammenhang mit der Implementierung eines Verfahrens zum Generieren von wenigstens einem energetischen/elektrochemischen Parameter aus Messdaten wenigstens einer messtechnischen mikrobiellen elektrochemischen Technologieeinheit basierend auf bioelektrochemischen Prozessen, wobei wenigstens ein Messwert mit wenigstens einem Prozessparameter korreliert wird, insbesondere bei Langzeitmessreihen über eine Dauer von mindestens 30 Tagen, bevorzugt mindestens zwei oder drei Monaten. Nicht zuletzt betrifft die vorliegende Erfindung auch ein Computerprogrammprodukt zum Ausführen von Schritten eines messtechnischen Verfahrens durchgeführt mittels wenigstens einer solchen messtechnischen Vorrichtung sowie die Verwendung einer Membran-Elektroden-Baugruppe mit Keramik in einer solchen messtechnischen Vorrichtung. Insbesondere betrifft die Erfindung Vorrichtungen und Verfahren sowie Verwendungen jeweils gemäß dem Oberbegriff des jeweiligen unabhängigen Anspruchs.

### HINTERGRUND DER ERFINDUNG

Bei der Implementierung von mikrobiellen messtechnischen Brennstoffzellen (MBZ) für diverse messtechnische Anwendungen bestehen bisher insbesondere kostentechnische Herausforderungen und technische Hürden hinsichtlich Langzeitstabilität. Bei vielen messtechnischen Anwendungen muss ein Verhalten des Messobjekts über mindestens mehrere Wochen oder Monate untersucht werden. Die sich bei bisherigen MBZ stark in Abhängigkeit von der Zeit ändernden messtechnischen Randbedingungen können bisher nicht ohne weiteres berücksichtigt werden und demnach nicht fehlerfrei bzw. nicht bei guter Messsicherheit herausgerechnet werden. Dies erschwert einerseits die Schaffung einer belastbaren Datengrundlage, andererseits auch deren Auswertung. Zudem können die vergleichsweise hohen Kosten je Messpunkt/-stelle eine zu große Hürde sein, MBZ zu implementieren. Beispielsweise das Kathodenmaterial bleibt bisher ein starker Kostentreiber. Gleichwohl wäre es wünschenswert, das Potential speziell von MBZ weiter ausschöpfen zu können, beispielsweise im Zusammenhang mit der Bestimmung des chemischen Sauerstoffbedarfs (CSB) bei (Ab-)Wasseraufbereitungsprozessen über einen vergleichsweise langen Zeitraum von vielen Wochen oder Monaten, beispielsweise auch im Zusammenhang mit der Bestimmung des biologischen Sauerstoffbedarfs (BSB) bei (Ab-)Wasseraufbereitungsprozessen, vorteilhaft auch redundant an mehreren Messpunkten/-stelle.

Gemäß dem Stand der Technik wird insbesondere auch auf dem Gebiet der Wasseranalytik versucht, MBZ und/oder Elektrolysezellen z.B. für die Bestimmung der Sauerstoffkonzentration zu implementieren, insbesondere ausgehend vom Stromfluss bzw. einem entsprechenden elektrischen Signal. Problematisch bleibt dabei bisher der hohe Einfluss von performancerelevanten Signalinterferenzen und Umweltfaktoren. Nicht zuletzt sind bisher keine vielversprechenden Maßnahmen bekannt, die zu einer Überwindung des so genannten "Scaling"-Problems beitragen, also dem (Salz-)Ablagerungsproblem mit schleichendem Verlust der sensorischen bzw. analytischen Fähigkeiten. Davon ausgehend besteht speziell auch auf dem Gebiet der Wasseranalytik Interesse an einer verbesserten Langzeitstabilität in Kombination mit einer belastbareren Korrelation von Messwerten und momentanem Sauerstoffbedarf.

Beispielhaft kann die Veröffentlichung US 11,352,272 B2 genannt werden, welche einen auf einer Elektrolysezelle basierenden bioelektrochemischen strombetriebenen Sensor insbesondere für eine Verwendung im Rahmen von Prozessoptimierungsmaßnahmen bei der Abwasserbehandlung beschreibt.

Ausgehend vom Stand der Technik ist ein Bedarf an langzeitstabiler Analytik mit hoher Messwert-/Messdatengüte bzw. hoher Korrelations-Aussagekraft auch über einen langen Messzeitraum zu spüren. Nicht zuletzt besteht insbesondere hinsichtlich bisher vergleichsweise hoher Kosten je Messpunkt auch Interesse an einer sehr kostenoptimierten Implementierung von Wasseranalytik bzw. Gewässersensorik derart, dass eine Mengenskalierung für mehrere oder gar sehr viele Messpunkte potentiell realisierbar wird, insbesondere auch vor dem Hintergrund, die Güte der generierbaren Datengrundlage zu verbessern.

Aufgabe ist, eine messtechnische Vorrichtung und ein messtechnisches Verfahren bereitzustellen, womit energetische/elektrochemische Parameter aus Messdaten basierend auf bioelektrochemischen Prozessen bei besonders guter Langzeitstabilität und möglichst geringem vorrichtungstechnischen Aufwand generiert und bei hoher Datengüte ausgewertet werden können. Auch ist es Aufgabe, ein/das messtechnische/s Verfahren dabei derart auszugestalten, dass einerseits eine besonders belastbare und möglichst auch umfangreiche Datengrundlage auf vorteilhafte Weise geschaffen und andererseits diese geschaffene Datengrundlage auch auf vorteilhafte Weise ausgewertet bzw. analysiert werden kann, insbesondere im Rahmen der Generierung von wenigstens Einer Ziel- bzw. Stellgröße zwecks (Prozess-)Optimierungsmaßnahmen über längere Zeiträume von mehreren Monaten, insbesondere bei der (Ab-)Wasserbehandlung. Nicht zuletzt ist es Aufgabe, ein mit wenigstens einer solchen messtechnischen Vorrichtung ausgestattetes messtechnisches System derart zu konzipieren, dass eine vergleichsweise exakte und umfangreiche Korrelation unterschiedlicher Typen von Messdaten bzw. Prozessparametern hinsichtlich möglichst variabler anwendungsspezifischer Implementierung sichergestellt werden kann, wahlweise bei einer Vielzahl von Messpunkten.

### ZUSAMMENFASSUNG DER ERFINDUNG

Diese Aufgabe wird durch eine messtechnische Vorrichtung gemäß Anspruch 1 sowie durch ein damit ausgestattetes messtechnisches System sowie durch ein entsprechendes messtechnisches Verfahren, insbesondere in Kombination mit einer prozessualen Steuerung/Regelung, sowie durch Verwendungen gemäß dem entsprechenden nebengeordneten Verwendungsanspruch gelöst. Vorteilhafte Weiterbildungen der Erfindung werden in den jeweiligen Unteransprüchen erläutert. Die Merkmale der im Folgenden beschriebenen Ausführungsbeispiele sind miteinander kombinierbar, sofern dies nicht explizit verneint ist.

Bereitgestellt wird eine messtechnische Vorrichtung eingerichtet zum Generieren von wenigstens einem energetischen/elektrochemischen (Zell-)Parameter aus Messdaten (bzw. elektrischen Signalen) basierend auf bioelektrochemischen Prozessen, aufweisend wenigstens eine messtechnische mikrobielle elektrochemische Technologieeinheit, MET, mit einem kathodischen Bereich abgeschottet von zu untersuchendem Fluid, und aufweisend eine Logikeinheit mit wenigstens einem Mikrocontroller eingerichtet zum Verarbeiten von über einen Auswertungszeitraum erhobenen Messdaten, wobei die messtechnische Vorrichtung eingerichtet ist zum Korrelieren wenigstens eines Messwerts mit wenigstens einem (organischen/anorganischen) Prozessparameter, P_{SB}, basierend auf den verarbeiteten Messdaten, insbesondere mit einem Prozessparameter für den chemischen/biologischen Sauerstoffbedarf (CSB/BSB) und/oder für eine Kohlenstoffverbrauchsrate und/oder für Stofffraktionen flüchtiger Fettsäuren, insbesondere eines zu optimierenden (Ab-)Wasseraufbereitungsprozesses oder Energiegewinnungsprozesses;

Erfindungsgemäß wird vorgeschlagen, dass die messtechnische Vorrichtung wenigstens eine zwischen kathodischem Bereich und zu untersuchendem Fluid angeordnete und wirksame Keramik, insbesondere Silikatkeramik, insbesondere Ton-Keramik (insbesondere Terrakotta) als Bestandteil der messtechnischen mikrobiellen elektrochemischen Technologieeinheit aufweist, welche den kathodischen Bereich vom zu untersuchenden Fluid separiert, wobei die messtechnische Vorrichtung mittels der wenigstens einen Keramik eingerichtet ist zum Generieren des wenigstens einen energetischen/elektrochemischen (Zell-)Parameters aus über einen (systembedingt unbeeinträchtigten) Auswertungszeitraum von mindestens 30 Tagen erhobenen Messdaten betreffend das zu untersuchende Fluid, insbesondere Messdaten aus zellinternen Widerstandskomponenten und/oder Spannungs-, Kapazitäts-, Strom-, Leistungs-, und/oder elektrischen Ladungsmessdaten. Dies liefert ein vergleichsweise breit einsetzbares messtechnisches Konzept, basierend auf vergleichsweise kostengünstigen Komponenten, bei vergleichsweise schneller messtechnischer Reaktionszeit, bei besonders guten bzw. variablen Korrelationsmöglichkeiten zwischen einzelnen Messdaten bzw. Parametern. Beispielsweise die (Ab-)Wasseranalytik in einer prozessualen Phase vor einer/der Wasseraufbereitung ist als interessantes Anwendungsfeld zur Implementierung der vorliegenden Erfindung anzusehen. Vorteilhaft wird als Keramik Silikatkeramik gewählt, beispielsweise Terrakotta bzw. Ton-Keramik, insbesondere mit den hier näher beschriebenen Eigenschaften insbesondere hinsichtlich Porosität, insbesondere in Abhängigkeit von einer jeweils gewählten (radialen) Dicke, insbesondere in Abhängigkeit von einem optional vorgebbaren Silikatgehalt (SiO₂).

Sofern gemäß der vorliegenden Offenbarung von messtechnischer mikrobieller elektrochemischer Technologieeinheit, MET, gesprochen wird, so ist darunter im Kontext der vorliegenden Erfindung synonym eine MBZ bzw. eine entsprechend messtechnische Anordnung zu verstehen, wahlweise mit oder ohne mikrobieller Elektrolysezelle (MEZ). Anders ausgedrückt: Das hier beschriebene messtechnische Konzept kann mit einer auf MEZ basierenden Analyse kombiniert werden. Erfindungsgemäß ist jedoch die Implementierung einer MEZ nicht erforderlich, so dass auch auf das Anlegen eines Potentials (bzw. einer zusätzlichen Spannung) verzichtet werden kann, wodurch die Systemautarkie und die Verwendbarkeit auch an schwer zugänglichen Messpunkten verbessert werden kann.

Sofern gemäß der vorliegenden Offenbarung von Prozessparameter, P_{SB}, gesprochen wird, ist darunter je nach sensorischem Kontext synonym auch ein Parameter allgemein zu verstehen, d.h., es muss nicht notwendigerweise eine Korrelation im Kontext eines zu steuernden/regelnden Prozesses erfolgen (z.B. bei Bodensensorik), wenngleich dies die erfindungsgemäß vorrangig im Interesse stehende Art und Weise der Implementierung darstellen mag.

Es hat sich im Rahmen der vorliegenden Erfindung gezeigt, dass die hier beschriebene vorrichtungstechnische Ausgestaltung die darauf aufbauenden Datengenerierungs- und Datenauswerteschritte erleichtert bzw. erst ermöglicht, insbesondere bei Langzeitmessungen bzw. Langzeitanalytik über mehrere Wochen: Die erfindungsgemäß erzielte Langzeitstabilität der MET ermöglicht insbesondere auf dem Gebiet der Wasseranalytik ein bevorzugt Modulations-basiertes, insbesondere Pulsmodulations-basiertes Generieren von elektrochemischen Parametern, die maßgeblich von der (Ab-)Wassermatrix beeinflusst werden und nicht bzw. nicht signifikant durch Salzablagerungen an der Kathode gestört werden. Die erfindungsgemäß sichergestellte Langzeitstabilität ermöglich dabei auch die hier beschriebene Art und Weise der Datenauswertung aus der gleichen Grundgesamtheit; zudem kann ein größerer, möglichst unbeeinträchtigter Datensatz ausgewertet werden, wodurch die Güte der generierten Parameter bzw. der CSB- und/oder BSB-Daten (bzw. entsprechender Schätzungen) signifikant verbessert werden kann. Dabei liefert die hier beschriebene Modulations-Analyse, insbesondere Pulsmodulations-Analyse auch vorteilhaft auswertbare Eingangsgrößen für wenigstens einen ML-Algorithmus (Algorithmen maschinellen Lernens), z.B. für ein ANN (artificial neural network), wodurch die Belastbarkeit der Analytik weiter verbessert werden kann. Beispielsweise ermöglichen die ermittelten elektrochemischen Parameter eine Isolation von Signalinterferenzen (was bei alleiniger Messung der Stromdichte üblicherweise bisher nicht möglich ist/war). Zudem können durch ML-Algorithmen bzw. daraus hervorgehende KI-Modelle komplexe nicht-lineare Zusammenhänge identifiziert und ausgewertet bzw. zumindest berücksichtigt werden, insbesondere auch in Hinblick auf eine bessere Datengüte dank hoher Langzeitstabilität im Kontext der Erhebung der Messdaten.

Es ist zu verstehen, dass die hier beschriebene Analytik in Bezugnahme auf Prozessparameter für den chemischen/biologischen Sauerstoffbedarf (CSB/BSB) allgemein zu sehen ist, insbesondere da BSB und CSB erwartungsgemäß für die meisten Anwendungen in vergleichbar eindeutig konkretisierbarem Kontext, insbesondere Umrechnungskorrelation ca. Faktor 2, miteinander stehen dürften (so auch die BSB5-, BSB10-, ..., BSB21-Werte, welche jeweils für eine Messwertabnahme ab fünf, zehn, ..., 21 Analysetagen stehen). Insbesondere ist zu verstehen, dass eine eher spezifische Korrelation (BSB) bisher weniger fehlerbehaftet war als eine eher allgemeine Korrelation (CSB), speziell da der Gesamt-CSB auch inerte Fraktionen, also nicht biodegradierbare lösliche und nicht-lösliche Fraktionen umfasst (also nicht biologisch abbaubare aber lösliche Fraktionen sowie partikuläre Fraktionen), die strenggenommen insbesondere im Rahmen prozessualer Verbesserungsmaßnahmen eher nicht interessieren. Die vorliegende Erfindung ermöglicht eine vergleichsweise schnelle schlanke Messung und Analyse bereits nach kurzer Zeit, mit dem Ausblick, beispielsweise den bisher üblicherweise genutzten BSB5 bereits nach deutlich kürzerer Zeit als fünf Tagen verfügbar zu machen (insofern könnte sinngemäß basierend auf der vorliegenden Erfindung auch z.B. von einem "BSB3" oder "BSB2" gesprochen werden). Gleiches gilt für die hier beschriebene Korrelation von Stofffraktionen flüchtiger Fettsäuren (VFAs) und/oder der Kohlenstoffverbrauchsrate (CCR), wie weiter unten detaillierter beschrieben. Auch insofern liefert die vorliegende Erfindung ein auf breiter Basis vorteilhaft implementierbares analytisches Konzept, mittels welchem sich bisherige messtechnische Maßnahmen hinsichtlich zahlreicher Parameter und Anwendungen spürbar vereinfachen und auch in der Genauigkeit verbessern lassen.

Die vorliegende Erfindung kann auch in folgendem Kontext beschrieben werden: Mikrobielle Brennstoffzellen (MBZs) stellen eine innovative Biotechnologie dar, mit deren Hilfe die im Abwasser enthaltene chemische Energie (insbesondere über einen Bereich von 1,5 bis 3,5 kWh/m³) in elektrische Energie umgewandelt werden kann. Hauptakteure dafür sind anaeroben Mikroorganismen, so genannte elektrochemisch aktive Bakterien. Dieser Prozess wird insbesondere am biologisch aktiven Biofilm der Anode der MBZ initiiert, an welcher Bakterien dieser speziellen Bakteriengruppe organische Verbindungen zu CO2, Protonen und Elektronen oxidieren. Die freigesetzten Elektronen werden über intra- und extrazelluläre Transportmechanismen an die Anode übertragen (terminaler Elektronenakzeptor). Getrieben von einem Potentialgradienten wandern die Elektronen spontan über einen externen Stromkreis zur Kathode, wo sie zusammen mit Protonen und Sauerstoff zu Wasser reduziert werden. MBZs können in diesem Kontext als Biosensoren genutzt werden, insbesondere basierend auf der Konvertierung biologischer Systemantworten in messbare Signale, welche als Bioindikationsgrundlage für organische und anorganische Substanzen genutzt werden kann. Üblicherweise korrelieren MBZ-basierte Biosensoren das generierte elektrische Stromsignal (oder die Leistungsdichte) mit der aktuellen Konzentration organischer Wasserinhaltsstoffe, vernachlässigen dabei allerdings performancerelevante Signalinterferenzen. Dazu zählen u.a. Umweltfaktoren, wie die elektrische Leitfähigkeit, die Temperatur, toxische Stoffe, der pH-Wert aber auch performancelimitierende Faktoren wie z.B. das so genannte "Scaling" (Ablagerungen, insbesondere vergleichsweise harte mineralische Ablagerungen wie z.B. CaCO3 an messtechnisch relevanten Komponenten der MBZs, mit schleichendem Verlust der messtechnischen Fähigkeiten bzw. Möglichkeiten). Diese Faktoren können jeweils auch entscheidenden Einfluss auf die Messgenauigkeit und Sensitivität des Biosensors haben. Die vorliegende Erfindung ermöglicht eine Überwindung zahlreicher aus dem Stand der Technik bekannter Nachteile, bei vorteilhaft vergleichsweise einfachen vorrichtungstechnischen Maßnahmen an der Sensorik. Im Rahmen der vorliegenden Erfindung konnten insbesondere ausgehend von den durch verbesserte Langzeitstabilität sichergestellten messtechnischen und datentechnischen Eigenschaften auch äquivalente elektrische Schaltkreismodelle entwickelt werden, welche zur Isolation der angesprochenen Signalinterferenzen verwendet werden können, wobei sich gezeigt hat, dass sich die Modellparameter (entsprechend internen Zellparametern) vorteilhaft durch moduliertes, insbesondere pulsmoduliertes Trennen, speziell auch pulsweitenmoduliertes Trennen und Verbinden des externen elektrischen Lastwiderstandes bestimmen lassen. Die Analyse der Zellparameter konnte hinlänglich zeigen, dass diese insbesondere mit der Zusammensetzung des Anolyten (Abwasser) korreliert werden können. Beispielsweise konnte ein ausgeprägter Zusammenhang zwischen der elektrischen Leitfähigkeit und dem ohmschen Widerstand des mikrobiellen MBZ- bzw. MET-Systems nachgewiesen werden. Insbesondere im Vergleich zu einer alleinigen interferenzüberlagerten Messung des elektrischen Stromes verbessert die hier beschriebene Art und Weise der Analyse der einzelnen Zellparameter insbesondere über einen Messezeitraum von mindestens einigen Wochen oder Monaten (z.B. mit täglicher Erhebung von Messdaten) die Sensitivität, verringert die Ansprechzeit und reduziert die Anzahl von Fehlmeldungen. Dabei hat sich gezeigt, dass in Abgrenzung zu einer einfachen linearen Korrelation die zum Teil komplexen Zusammenhänge insbesondere betreffend den vergleichsweise allgemeinen CSB- oder (bereits etwas spezifischer) BSB-Prozessparameter vergleichsweise akkurat bzw. aussagekräftig abgebildet werden können, also vergleichsweise komplexe nichtlineare Beziehungen zwischen MBZspezifischen Parametern und Zielgrößen (CSB, BSB5, etc.), insbesondere auch indem das Auswerten/Analysieren mittels künstlicher neuronaler Netzwerke (eng. artificial neuronal networks, ANNs) weiterführend implementiert wird. Beispielweise kann der Einsatz von ANNs erfolgreich angewandt werden, um aus dem substratspezifischen Spannungsprofil einer MBZ innerhalb eines Batch-Zyklus (Substrat-Tausch/-Zugabe bei Laborbetrieb für möglichst standardisierte Versuchsbedingungen) die Konzentration und Art des verwendeten Substrates zu ermitteln. Die vorliegende Erfindung ermöglicht dabei auch, die Messreaktionszeit beträchtlich herabzusetzen, insbesondere auf unter eine Minute, so dass bei Langzeitbetrachtung (mehrere Wochen/Monate/Jahre) nahezu eine Echtzeitmessung von bisher sehr kosten-, labor-, personal- und zeitintensiven Qualitätsparametern (insbesondere im Kontext von CSB, BSB5, etc.) ermöglicht werden kann, insbesondere auch auf dem Gebiet der (Ab-)Wasseranalytik/-aufbereitung. In diesem Kontext werden im Rahmen der vorliegenden Erfindung insbesondere die vorrichtungstechnische mikrobielle MBZ-Technologie und die Messdatenauswertung, vorteilhaft umfassend Modulation (insbesondere Pulsmodulation, speziell Pulsweitenmodulation) und Softsensorik bestehend aus wenigstens einem äquivalenten elektrischen Schaltkreismodell und ANN, auf neue Art und Weise fusioniert, einerseits zum (vorrichtungstechnischen) Bereitstellen einer langzeitstabilen und vorteilhaft auch energetisch zumindest weitgehend unabhängigen messtechnischen Vorrichtung, andererseits zum Generieren und Auswerten von Messdaten hoher Güte, insbesondere auch in Hinblick auf einen seitens Anwendern bzw. Anlagenbetreibern sensordatengetriebenen prozessualen Steuerungs-/Regelungsvorgang. Dabei ermöglicht der hier beschriebene vorrichtungstechnische Aufbau eine besonders kostengünstige ("lowcost") und wartungsarme und messtechnisch langzeitstabile Reaktorkonfiguration, die auch eine redundante Implementierung für zahlreiche Messpunkte/-stellen begünstigt bzw. überhaupt erst ermöglicht (Raster, Schwarm, Netzwerk), wodurch die Messsicherheit bzw. Belastbarkeit der Analyse weiter gesteigert werden kann, sei es je Messpunkt, sei es über eine vergleichsweise große zu messende Fläche oder ein großes zu messendes Volumen.

Die messtechnische Vorrichtung kann dabei auch Temperatursensorik umfassen (z.B. auch implementiert als integrierte Temperaturkompensation insbesondere im Rahmen einer Leitfähigkeitsmessung), mittels welcher die Temperatur des zu untersuchenden Fluids erfasst werden kann, wobei die Korrelation von Messwert (betreffend die Spannung, Leitfähigkeit oder dergleichen) und Prozessparameter (insbesondere CSB/BSB) temperaturabhängig realisierbar ist. Dabei kann eine Genauigkeit von z.B. 0,1°C als ausreichend erachtet werden, insbesondere in Hinblick auf die als Richtwert anzunehmende Verdoppelung der temperaturabhängigen Aktivität von Mikroorganismen je 10° Temperaturanstieg (van't Hoff-Arrhenius Gleichung).

Es ist zu verstehen, dass das messtechnische Konzept der vorliegenden Erfindung auch als auf drei Aspekten basierend beschrieben werden kann:
- Biosensor bzw. mikrobielle elektrochemische Technologieeinheit (MET);
- Mikroelektronik (insbesondere mit Mosfet bzw. Feldeffekttransistoren) zum Generieren eines Spannungsprofils basierend auf Modulation, insbesondere Pulsmodulation, speziell auch Pulsweitenmodulation des externen elektrischen Lastwiderstandes;
- Datenauswertung durch Aufbereiten elektrochemischer Parameter mittels Mikrocontroller;

Das generierte Spannungsprofil kann insbesondere über eine Parameterbestimmungsroutine, abgeleitet aus einem mathematischen Modell ausgewertet werden (erste Softsensorik bzw. äquivalenter elektrischer Schaltkreis). Die Parameter des Modells können dabei (den) elektrochemischen Parametern entsprechen. Die Steuerung der Mikroelektronik sowie die Parameterbestimmungsroutine können auf einem/dem Mikrocontroller implementiert werden. Die darauffolgenden bzw. darauf basierende Datenauswertung erfolgt bevorzugt mittels zweiter Softsensorik insbesondere umfassend Machine Learning, z.B. ein neuronales Netz, insbesondere da komplexe nicht-lineare Beziehungen zwischen der oder den Zielgrößen (CSB, BSB, etc.) und den elektrochemischen Parametern berücksichtigt werden müssen. Als Output kann im Rahmen der Datenauswertung die gesuchte Zielgröße ausgegeben werden.

Es ist zu verstehen, dass die erfindungsgemäße Messtechnik und Datenanalysetechnik insbesondere auch im folgenden Kontext vorteilhaft implementierbar sind:
i) messtechnische Vorrichtungen für die Prozessoptimierung bei (Ab-)Wasseraufbereitungsprozessen und Energiegewinnungsprozessen;
ii) messtechnische Vorrichtungen eingerichtet zum Generieren von energetischen und/oder elektrochemischen Parameter basierend auf Modulation, insbesondere basierend auf Modulation des elektrischen Lastwiderstandes;
iii) messtechnische Vorrichtungen umfassend wenigstens eine mikrobielle elektrochemische Messeinrichtung, eingerichtet für wenigstens eine messtechnische Anwendung aus der folgenden Gruppe: Messtechnik für Abwasserströme, Industrieabwässer (z.B. aus Papier-/Fleisch-/Süßwarenindustrie), Prozesswässer, Grauwasser, Schwarzwasser, Gelbwasser, Sickerwässer, medizinische Abwässer, Landwirtschaft, Böden, Pflanzenkläranlagen, Deponien, Wasser/Gewässer im Allgemeinen, Trinkwasser, Grundwasser, Niederschlagswasserbehandlung, Substrate, Faultürme (insbesondere anaerobe Faulung);
iv) messtechnische Vorrichtungen umfassend wenigstens eine mikrobielle elektrochemische Messeinrichtung, eingerichtet zum messtechnischen Nachweis von Substanzen beim Bestimmen von Wasserqualitätsparametern, beispielsweise beim Bestimmen wenigstens eines der folgenden Parameter: CSB, BSB5, Stickstoff, Stickstoff-Verbindungen, Phosphor, Phosphor-Verbindungen (insbesondere Ortho-Phosphate), gelöster Sauerstoff, flüchtige organische Säuren;
v) messtechnische Vorrichtungen umfassend wenigstens eine mikrobielle elektrochemische Messeinrichtung, eingerichtet als messtechnische Frühwarnvorrichtungen insbesondere hinsichtlich toxischer Substanzen wie Schwermetallen, Nitraten, Antibiotika;
vi) energieautarke messtechnische Vorrichtungen umfassend wenigstens eine mikrobielle elektrochemische Messeinrichtung;
vii) messtechnische Vorrichtungen umfassend wenigstens eine mikrobielle elektrochemische Messeinrichtung, eingerichtet zur Erfassung wenigstens eines Photosynthese-Parameters insbesondere bei/von Bäumen.

Dabei kann die entsprechende messtechnische Vorrichtung wahlweise sowohl Firmware als auch Softsensorik umfassen, also all jene Komponenten, die für die Generierung der gewünschten Messgrößen genutzt werden. Insbesondere können dank des kosteneffizient und energieautark implementierbaren erfindungsgemäßen Konzeptes sowohl Firmware als auch Softsensorik im jeweiligen Sensor bzw. am jeweiligen Messpunkt integriert werden. Hierdurch kann auch eine anwendungsspezifische Anpassung der Messtechnik auf sehr spezifische Weise ermöglicht werden. Je Messpunkt kann demnach z.B. der (bio)chemische Sauerstoffbedarf direkt sichtbar gemacht werden (z.B. basierend auf der vordefinierten Abhängigkeit 1mA=7,2 mgCOD/d, vergleiche dazu auch die Abhängigkeit über die Faradaykonstante: ein (1) Elektronenäquivalent entsprechend acht (8) Gramm CSB), und dabei werden für den messtechnischen Aufbau auch keine toxischen Chemikalien erfordert (insbesondere kein Kaliumdichromat).

Das erfindungsgemäße messtechnische Konzept ermöglicht auch, weitere Substanzen nachzuweisen (insbesondere im Sinne von Abwasserqualitätsparametern), z.B. Parameter betreffend CSB, BSB5, Stickstoff, Stickstoff-Verbindungen, Phosphor, Phosphor-Verbindungen, gelösten Sauerstoff, flüchtige organische Säuren. Ferner kann insbesondere dank des auf lebenden Mikroorganismen basierenden messtechnischen Prinzips eine Implementierung als Frühwarnsystem bzw. Warnvorrichtung auf einfache Weise realisiert werden (z.B. hinsichtlich toxischer Substanzen, z.B. Schwermetalle, Nitrate, Antibiotika). Die energieautark realisierbare Implementierung ermöglicht zudem eine (je Messpunkt) energetisch unabhängige Messtechnik, die auch in nur auf schwierige Weise erschließbaren Bereichen bzw. in so genannten "remote zones" genutzt werden kann. Auch eine Implementierung zur Erfassung bzw. Auswertung von Photosynthese-Prozessen kann realisiert werden, beispielsweise im Zusammenhang mit dem Monitoring des (Gesundheits-)Zustands besonders wertvoller Pflanzen bzw. Bäume.

Es ist zu verstehen, dass "Keramik" hier im Sinne einer technischen Keramik mit einer offenen porenartigen Struktur zu verstehen ist und glasiertes Steinzeug begrifflich ausschließt. Die vorliegende Erfindung basiert auf dem Konzept, mittels der (technischen) Keramik einen Austausch bzw. eine Diffusion und gegebenenfalls auch zu gewissem Grad eine unschädliche die Langzeitstabilität nicht beeinflussende Ablagerung oder dergleichen Effekt zuzulassen. Gemäß einer Ausgestaltung ist die Keramik durch Silikatkeramik, insbesondere Ton-Keramik bzw. Terrakotta gebildet oder umfasst wenigstens eine Silikatkeramik, insbesondere Ton-Keramik.

Vorteilhaft ist die Keramik als Silikatkeramik ausgestaltet, gegebenenfalls weiter konfektioniert durch wenigstens ein Additiv, beispielsweise Aktivkohle, pyrolysierte Reishülsen vorteilhaft mit hohem SiO2-Anteil, Zirkon. Es hat sich gezeigt, dass mittels Silikatkeramik im hier beschriebenen Anwendungskontext insbesondere die folgenden vorteilhaften Effekte sichergestellt werden können:
- noch effektivere Reduzierung von Membran-Biofouling (weitere Erhöhung der Langzeitstabilität);
- gesteigerte Protonendiffusion, erhöhter Protonen-Massentransferkoeffizient;
- Verbesserung des Wasserrückhaltevermögens dank hygroskopischem Oxid wie SiO2;
- verbesserte Protonenleitfähigkeit dank Formation gebundenen Wassers innerhalb der Membran;

Beispielsweise kann SiO2 in Partikelform der Keramik beigemischt bzw. zugesetzt sein. Hierdurch kann auch die Hydrophilie verbessert/verstärkt werden. Ablagerungen bzw. Verschmutzungen auf der Membranoberfläche können dann leichter gelöst werden bzw. können sich leichter wieder ablösen.

Der SiO2-Anteil kann beträchtlich sein. Für einige Anwendungsfälle mag es vorteilhaft sein, den SiO2-Anteil deutlich über 50% zu wählen, gegebenenfalls sogar über 70% (Gewichtsprozent). Dies kann auch zu guter Leistungscharakteristik beitragen.

In messtechnischer Hinsicht ist auch zu verstehen, dass die so genannte Kohlenstoffverbrauchsrate (carbon consumption rate, CCR) mittels der hier beschriebenen messtechnischen Vorrichtung vergleichsweise einfach und bei guter Datengüte erfasst bzw. ausgewertet werden kann (einfacher Umrechnungsfaktor) und daher auch z.B. im Rahmen einer Plausibilitätsprüfung und/oder in Hinblick auf eine leichtere Datenanalyse/-verwendung durch Anwender bzw. anwendende Einrichtungen (z.B. Wasseraufbereitungs-Unternehmen) in Form ausgewerteter Daten bereitgestellt werden kann. Insbesondere kann im Kontext der Erfassung der Kohlenstoffverbrauchsrate auch eine Kalibrierung auf vergleichsweise einfache Weise durchgeführt werden bzw. mag auch komplett entbehrlich werden (insbesondere die Messung der Aktivität von Mikroorganismen mag auf ausreichend genaue Art und Weise ohne eine bestimmte Art und Weise einer Kalibrierung implementiert sein/werden). Auch kann die Kohlenstoffverbrauchsrate als Indiz für den CSB (bzw. als Größe im Kontext der Ermittlung des CSB) ausgewertet werden, wobei auch Substratlimitierungen aufgezeigt werden können, wobei auch ein (Gesundheits-)Zustand der involvierten Bakterien abgeschätzt werden kann. Somit kann die Datenanalyse unter Bezugnahme auf die Kohlenstoffverbrauchsrate bzw. durch deren Einbezug noch weiter verbessert werden.

Auch ist zu verstehen, dass die Stofffraktion flüchtige Fettsäuren (volatile fatty acids, VFA) mittels der hier beschriebenen messtechnischen Vorrichtung vergleichsweise einfach erfasst werden kann, wodurch weitere Erkenntnisse in Hinblick auf die ablaufenden biologischen Prozesse sowie anaerobe Faulung gewonnen werden können.

Somit wird durch Bezugnahme auf die hier explizit erwähnten drei Prozessparametergruppen für CSB/BSB, Kohlenstoffverbrauchsrate, flüchtige Fettsäuren aufgezeigt, in welchem Kontext die hier beschriebene Messtechnologie implementiert werden kann, sei es jeweils spezifisch für nur eine dieser Gruppen, sei es in Kombination von zwei oder allen drei dieser Gruppen (insbesondere einschließlich Ausgabe einer Korrelation unter diesen drei Gruppen bzw. dieser drei Gruppen untereinander).

Als kathodischer Bereich ist dabei insbesondere die Kathode als solche (katholytische Schicht, Katalysatorschicht) in Verbindung mit einem Stromkollektor zu verstehen, wahlweise auch in Kombination mit einer Gasdiffusionsschicht (Luftkathode), vorteilhaft jedoch nur zweischichtig, also ohne Gasdiffusionsschicht. Eine Gasdiffusionsschicht (z.B. aus PTFE) ermöglicht eine gezielte Einflussnahme auf den Sauerstofftransfer. Vorteilhaft ist jedoch bereits die Keramik derart funktional ausgelegt, dass die (Sauerstoff-)Diffusion auch ohne zusätzliche Gasdiffusionsschicht optimiert eingestellt werden kann, insbesondere auch bei guter Langzeitstabilität. Insofern ermöglicht die erfindungsgemäße Konfiguration auch den Verzicht auf eine zusätzliche Gasdiffusionsschicht.

Es ist zu verstehen, dass der kathodische Bereich einen Zugang zur Luftatmosphäre aufweist, z.B. realisiert mittels eines vor Umwelteinflüssen schützenden Deckels, oder dass eine technische (aktive) Belüftung bzw. O2-Zufuhr implementiert ist (Sauerstoffreduktionsreaktion O2 + 4e- + 4H⁺ → 2H2O). Insofern ist die Begrifflichkeit "abgeschottet" im Kontext des kathodischen Bereichs eng hinsichtlich des zu untersuchenden Fluids zu verstehen, insbesondere da der kathodische Bereich auch mit Katholyt(akkumulation) geflutet sein kann. Auch hierzu ist jedoch zu ergänzen, dass die vorliegend beschriebene Messtechnik vom Messprinzip her auch umgekehrt betrieben werden kann in Bezug auf Anoden-/Kathodenfunktion, d.h., die vorliegend beschriebene MET ist wahlweise auch für eine Bestimmung des O2-Gehalts implementierbar, z.B. bei redundanter Anordnung mehrerer MBZ in örtlicher Nähe, bei gegenseitiger Überprüfung und messtechnischer Ergänzung.

Personifizierte Begriffe, soweit sie hier nicht im Neutrum formuliert sind, können im Rahmen der vorliegenden Offenbarung alle Geschlechter betreffen. Etwaige hier verwendete fremdsprachige Ausdrücke oder Abkürzungen sind jeweils branchenübliche Fachausdrücke und sind dem Fachmann in der entsprechenden Sprache geläufig. Etwaige dazu synonym verwendete/verwendbare deutschsprachige Begriffe oder Abkürzungen können hier der Vollständigkeit halber redundant angegeben werden, oder vice versa, beispielsweise bezüglich des Begriffs chemischer Sauerstoffbedarf, CSB (chemical oxygen demand COD), oder bezüglich des Begriffs Kohlenstoffverbrauchsrate (CCR).

Gemäß einem Ausführungsbeispiel definiert die Keramik wenigstens eine Membrankomponente der messtechnischen mikrobiellen elektrochemischen Technologieeinheit. Dies begünstigt auch eine vorrichtungstechnische Implementierung, bei welcher mittels der Keramik eine Brücke bzw. Abschottung bzw. Barriere zwischen kathodischem Bereich/Kompartiment und zu untersuchendem Fluid bereitgestellt wird, insbesondere in der Art einer Separatorfunktion.

Als Membran bzw. Membrankomponente ist hier bei Bezugnahme auf die vorliegende Erfindung insbesondere diejenige vorrichtungstechnisch zwischen den Elektroden einer/der Membran-Elektroden-Baugruppe der MET eingebundene Komponente zu verstehen, welche für ein Separieren von Anode und Kathode bzw. anodischem Bereich und kathodischem Kompartiment Sorge trägt, also nicht notwendigerweise nur eine Membrankomponente im engeren Sinne, sondern in funktionaler Hinsicht auch jene Komponente(n), die in einer MBZ bzw. in der hier beschriebenen MET üblicherweise als Separator fungiert bzw. erfindungsgemäß vorgesehen ist/sind.

Gemäß einem Ausführungsbeispiel ist die Keramik Bestandteil einer Membran-Elektroden-Baugruppe der wenigstens einen messtechnischen mikrobiellen elektrochemischen Technologieeinheit, insbesondere in der Art einer vollflächigen Zwischenschicht oder Brücke zwischen einem anodischen Kompartiment/Abschnitt/Bereich (z.B. einem Abwasserbecken) und einem/dem durch die Membran-Elektroden-Baugruppe definierten kathodischen Kompartiment. Eine solche insbesondere zylindrische Einfassung des kathodischen Kompartiments durch die Keramik kann auch eine besonders gute Wirkung der Keramik sicherstellen, bei vergleichsweise geringem vorrichtungstechnischem Aufwand.

Als Membran-Elektroden-Baugruppe ist dabei gemäß einer Ausgestaltung der vorliegenden Erfindung insbesondere eine Keramik-Elektroden-Baugruppe zu verstehen.

Gemäß einem Ausführungsbeispiel bildet die wenigstens eine Keramik zusammen mit den Elektroden (Anode, Kathode) jeweils in vollumfänglicher mantelflächiger Anlage, bevorzugt radial zug-/druckkraftvorgespannt, eine/die bevorzugt dreikomponentige Membran-Elektroden-Baugruppe der messtechnischen mikrobiellen elektrochemischen Technologieeinheit, MET. Dies liefert auch einen vergleichsweise einfachen und kostengünstigen und langlebigen Aufbau einer der wichtigsten, wenn nicht sogar der wichtigsten Baugruppen der MET.

Beispielsweise wird die Membran-Elektroden-Baugruppe oder die gesamte messtechnische Vorrichtung in einer Gesamthöhe von 20-30cm ausgeführt, für eine bestimmungsgemäße Eintauchtiefe im Bereich von z.B. mindestens 5cm bis 10cm. Anders ausgedrückt: Es können vorteilhaft 10-20cm der Gesamthöhe in einem Bereich tiefer als 10cm eingetaucht sein, also z.B. in einer Tiefe von 10cm bis 30cm.

Bezüglich einer im Kontext der vorliegenden Erfindung insbesondere hinsichtlich Langzeitstabilität und Kostenaufwand besonders vorteilhaften Konfektionierung der Keramik ist zu verstehen, dass insbesondere die radiale Dicke und die Porosität der Keramik als effektive physikalische Auslegungsgrößen heranzuziehen sind, insbesondere zwecks Sicherstellen guter Langzeitstabilität und minimalen Drifts, und auch zwecks Optimierung der Leistung der MET, insbesondere in Hinblick auf die folgenden Eigenschaften:
i) Permeabilität für gasförmigen Luftsauerstoff;
ii) ohmscher Widerstand;
iii) ionische Leitfähigkeit;
iv) mechanische Stabilität;

Dabei kann die Dicke insbesondere auch als Funktion der Porosität vorgegeben werden, bzw. umgekehrt. Dazu im Einzelnen: Bei zu starker O2-Diffusion aufgrund zu hoher Permeabilität mag das Risiko bestehen, dass sich im anodischen Biofilm eine aerobe Milieubedingung einstellt, welche die anaeroben exoelektrogenen Bakterien hemmt. Anders ausgedrückt: Sauerstoff kann die Aktivität von exoelektrogenen Mikroorganismen hemmen, mit dem Effekt, dass die Bioenergieausbeute sinkt, insbesondere mit dem Risiko, dass aeroben Bakterien die exoelektrogenen Bakterien überwachsen. Wird die Keramik hingegen zu dick gewählt oder die Porosität zu gering gewählt, steigt der ohmsche Widerstand, mit dem Effekt, dass die Biostromproduktion sinkt.

Die ionische Leitfähigkeit kann insbesondere durch die Partikelgröße und die Art des verwendeten Tons vorgegeben werden.

Die mechanische Stabilität ist stark anwendungs- und einsatzspezifisch zu definieren: Die Keramik sollte nicht zu dünn sein, insbesondere nicht dünner als 2mm, bevorzugt nicht dünner als 3mm. Als obere Grenze für die Dicke mag wohl 20mm anzusehen sein, bevorzugt sogar nur 10mm, je nach Anwendung.

Die Porosität liegt vorteilhaft im Bereich von ca. 10 bis 35%, bevorzugt 20 bis 30%. Beispielsweise liefert eine radiale Dicke im Bereich von 6mm bis 9mm einen guten Kompromiss insbesondere hinsichtlich guter Leistungsdichte.

Somit kann beispielsweise eine (radiale) Keramikdicke zwischen den Elektroden im Bereich von 5 bis 10mm insbesondere in Kombination mit einer Porosität im Bereich von ca. 23% bis 30% (Mittelwert) einen vorteilhaften Kompromiss insbesondere zwischen Sauerstoffpermeabilität, ohmschem Widerstand, ionischer Leitfähigkeit und mechanischer Stabilität sicherstellen, bei guter bzw. maximierter Bioenergieproduktion. Der Fachmann kann die anwendungsspezifisch optimierbaren Materialeigenschaften ausgehend von der vorliegenden Offenbarung insbesondere durch Wahl der Art des verwendeten Tons und wahlweise auch Zugabe von Additiven weiter optimieren.

Was einen Zusammenhang zwischen Porosität und (radialer) Dicke betrifft, so ist auch zu verstehen, dass eine anwendungsspezifisch vordefinierbare Abhängigkeit dieser physikalisch-strukturellen Parameter untereinander auch durch materialspezifische (Werkstoff-)Besonderheiten beeinflusst sein/werden kann. Anders ausgedrückt: Neben diesen physikalischen Parametern kann für die jeweils gewünschte Funktionsweise/Funktionalität ferner auch wenigstens ein chemischer Werkstoff-Parameter beachtenswert sein/werden, insbesondere im Kontext einer Unterteilung in Silikatkeramiken, Oxidkeramiken, Nichtoxidkeramiken, bzw. im Kontext einer Abgrenzung der hier beschriebenen Keramik von Oxidkeramiken und Nichtoxidkeramiken. Es hat sich gezeigt, dass es vorteilhaft ist, den Silikat (SiO₂)-Anteil zu beachten und vorteilhaft auch derart vorzugeben, dass die Keramik eine Silikatkeramik ist oder zumindest wenigstens eine Silikatkeramik umfasst, insbesondere Silikatkeramik mit SiO2 als Hauptbestandteil >70% und wahlweise Bestandteilen wie z.B. Aluminium und dergleichen. Wahlweise kann die Keramik Zirkon zumindest als Additiv umfassen. Basierend auf der vorliegenden Offenbarung versteht der Fachmann, welche Charakteristik die Keramik in Hinblick auf Separatorfunktion, mechanische Stabilität und Langzeitstabilität vorteilhaft aufweisen sollte, so dass der Fachmann davon ausgehend auch weitere Varianten der Keramik ersinnen kann, beispielsweise umfassend oder basierend auf ZrO2-Keramikbestandteilen.

Es hat sich gezeigt, dass neben der Porosität auch wenigstens ein (Massen-)Transferparameter als Auslegungsgröße herangezogen werden kann, zwecks noch gezielterer Abstimmung der Eigenschaften der Keramik. Vorteilhaft ist die Keramik insbesondere auch durch wenigstens einen Transferparameter aus der folgenden Gruppe charakterisiert oder zumindest charakterisierbar:
Sauerstofftransferkoeffizient, Wassertransferkoeffizient, Wasseraufnahmekoeffizient. Dies begünstigt ein noch exakteres Einstellen der MET oder ein noch gezielteres Auslegen der MET für bestimmte Anwendungen. Basierend auf dem Sauerstofftransferkoeffizienten kann insbesondere eine Optimierung der MET hinsichtlich Aktivität exoelektrogener Bakterien einerseits und ausreichendem Ionen- bzw. Wassertransport andererseits erfolgen. Basierend auf dem Wassertransferkoeffizient kann insbesondere eine noch weitergehende Optimierung des Langzeitverhaltens und der Robustheit insbesondere gegen so genannte Scaling-Effekte erfolgen. Ferner kann dank eines ausreichend hohen Wassertransferkoeffizients auch einer Austrocknung der Kathodenkammern insbesondere in den Sommermonaten entgegengewirkt werden, insbesondere indem über einen ausreichend hohen Wassertransferkoeffizienten sichergestellt werden kann, dass ein Evaporationseffekt ausgeglichen oder überkompensiert wird.

Für eine noch gezieltere Charakterisierung der Keramik kann der Fachmann auch auf die Porengröße bzw. auf das Spektrum der Porengrößen eingehen, zumindest im Rahmen von experimentellen Untersuchungen. Die Porengröße kann insbesondere auf die folgenden Eigenschaften Einfluss haben: tatsächliche Auswirkungen von Scaling-Effekten, Massentransfer, Anwendungsspektrum der MET. Ausgehend von einem als vorteilhaft erkannten Bereich für die Porosität kann der Fachmann demnach anwendungsspezifisch, z.B. je nach Verunreinigungsgrad des zu untersuchenden Fluids, Vorgaben für eine bevorzugte Porengröße vornehmen.

Ferner ist zu verstehen, dass guter flächiger Kontakt zwischen den Elektroden (ins. der Kathode) und der Keramik ausschlaggebend für die erzielbare Leistungsausbeute ist. Bevorzugt wird für die Keramik eine Maßtoleranzen deutlich unter 2mm vorgeben, vorteilhaft maximal ±1,5mm. Maßtoleranzen der Elektroden können noch genauer/kleiner sein. Vorteilhaft wird insbesondere zwecks Ausgleich etwaiger Maß-Toleranzen seitens der Keramik die Verwendung von Hydrogelen vorgesehen, zwecks Optimierung der flächigen Kontaktierung zwischen Keramik-Membrankomponente und Elektroden (insbesondere Kathode), mit dem Effekt höherer Stromausbeuten. Auch kann mittels Hydrogelen in der Art eines positiven Nebeneffekts ein Feuchthalten der Kathode, sofern anwendungsspezifisch erforderlich oder zumindest zweckdienlich, erleichtert werden, so dass das Haushalten bezüglich Katholyt vereinfacht wird (vorteilhaft hydratisierte Kathode, vorteilhaft minimierte Evaporation). Eine Menge von nur einigen wenigen Gramm Hydrogel mag bereits bis zu 1 Liter Wasser speichern können. Beispiel: bei 10mL/d Katholyt mögen ca. 3g Hydrogel ausreichen, um den gebildeten Katholyt über eine Dauer von ca. 100 Tagen zu binden. Auch diese Maßnahme kann demnach förderlich sein im Kontext des Ziels hoher Langzeitstabilität. Nicht zuletzt mag die Verwendung von Hydrogel, z.B. in Granulatform, auch die energetischen Anforderungen abmildern und die Implementierung einer energetischen Autarkie über einen möglichst langen Zeitraum erleichtern (z.B. danke Verzicht auf Pumpentechnologie bzw. Energieverbrauch durch Abpumpmaßnahmen). Dabei ist zu verstehen, dass das Katholyt-Aufkommen für die meisten Anwendungen biostromabhängig sein dürfte, d.h., bei hoher Biostromproduktion ist auch mit relativ hoher Katholyt-Menge zu rechnen. Da die Biostromproduktion auch von der momentanen Zusammensetzung des zu untersuchenden Fluids abhängen kann bzw. damit schwanken kann, mag es für bestimmten Anwendungen zweckdienlich sein, zusätzliche Vorkehrungen zur Optimierung des Katholythaushalts bzw. der Feuchtebedingungen für die Kathode zu treffen, insbesondere die Verwendung von Hydrogel. Beispielsweise wird Hydrogel schichtweise bzw. als wenigstens eine Schicht vorgesehen (z.B. pulverförmig appliziert), insbesondere zwischen einer entsprechenden Elektrode und der Keramik, und wahlweise auch an der Innenseite der Kathode.

Somit wird deutlich, dass eine insbesondere hinsichtlich Langzeitstabilität und Kostenaufwand besonders vorteilhafte Konzipierung der messtechnischen Vorrichtung ausgehend von der gewählten Ausgestaltung der Keramik mit darauf abgestimmter Ausgestaltung der Elektroden zu einer anwendungsspezifisch optimierbaren Membran-Elektroden-Baugruppe führen kann, bei welcher z.B. anwendungsspezifisch eine Optimierung der messtechnischen Reaktionszeit implementiert ist (insbesondere dank möglichst dünner Anode), und/oder eine kostentechnisch besonders effektive Ausgestaltung insbesondere unter Berücksichtigung der Ausgestaltung der Kathode. Die Dicke der Kathode liegt dabei z.B. bei ca. 0,5mm, kann jedoch auch anwendungsspezifisch von der Anzahl der für die Sauerstoffreduktionsreaktion nutzbaren aktiven Zentren abhängig sein.

Es ist zu verstehen, dass es je nach Art der Implementierung von Vorteil sein kann, eine gewisse Menge an Katholyt(akkumulation) im messtechnischen System zu belassen, insbesondere um eine gegebenenfalls hemmende Wirkung von zu hohen Sauerstoffkonzentrationen unterbinden zu können. Dies kann z.B. auch auf konstruktive Weise besorgt werden, z.B. durch eine vergleichsweise lange schmale Zylindergeometrie. Insbesondere sind auch solche Implementierungen realisierbar, bei welchen bereits der gelöste Sauerstoff eine ausreichend hohe Konzentration aufweist, um den Betrieb der MBZ sicherstellen zu können (8 bis 9 mg/L in der Flüssigphase). Dabei kann auch die gewählte Dicke der Anode zum Sauerstoffkonzentrationsgleichgewicht beitragen, insbesondere hinsichtlich des für die gewünschte Geschwindigkeit des diffusiven Substrat-Transports vordefinierbaren Konzentrationsbereichs bzw. hinsichtlich der Dicke des sich im Betrieb bildenden Biofilms.

Auch ist zu verstehen, dass die hier beschriebene Membran-Elektroden-Baugruppe der MET insbesondere im Kontext der folgenden Materialauswahl vorteilhaft implementierbar ist: Anodenmaterial bestehend aus oder zumindest umfassend kohlenstoffbasierten Schleier bzw. Kohlenstoffschleier (sinngemäß: CFK-Matte) oder Graphitfilz oder Aktivkohlefaserfilz, Keramikmembran(komponente) bzw. Keramikseparator, Kathodenmaterial umfassend ein Aktivkohlegemisch als Katalysator, vorteilhaft in Kombination mit einem (Edel-)Stahlgewebe für eine Stromkollektorfunktion. Vorteilhaft umfasst die Membran-Elektroden-Baugruppe für wenigstens eine der Elektroden ein Aktivkohlefaserfilz. Es hat sich gezeigt, dass Aktivkohlefaserfilz in stabiler Form als Karbonschleier bereitgestellt bzw. genutzt werden kann. Die Anode kann z.B. auch durch einen mehrlagigen Kohlenstoff-/Karbonschleier gebildet sein, wobei die Anodendicke anwendungsspezifisch adaptierbar ist/bleibt.

Vorteilhaft ist/wird die Materialauswahl für die Elektroden der hier beschriebenen Membran-Elektroden-Baugruppe unter Materialien aus der folgenden Gruppe getroffen: kohlenstoffbasierte Schleier bzw. Kohlenstoffschleier, Graphitfilz, Aktivkohlefaserfilz (Anode) und/oder Aktivkohlegemisch, Aktivkohlefaserfilz, Aktivkohle auf Kohlenstoffbasis (Kathode).

Die vorliegende Erfindung beruht dabei auch auf der Erkenntnis, dass bei der Auswahl des Anodenmaterials eine große Oberfläche und Porosität in Kombination mit guter elektrischer Leitfähigkeit, Biokompatibilität sowie Langlebigkeit und Stabilität als vorherrschende Eigenschaften sichergestellt sein sollen. Es hat sich im Rahmen der vorliegenden Erfindung gezeigt, dass kohlenstoffbasierter Schleier bzw. Kohlenstoffschleier oder Graphitfilz einen guten Kompromiss zwischen Leistungsausbeute und vorrichtungstechnischem Aufwand liefert (insbesondere auch mit großen kostentechnischen Vorteilen). Dabei kann auch ein vordefinierter Biegeradius bzw. eine diesbezügliche Toleranzangabe vorteilhaft sein, insbesondere in Hinblick auf die Verwendung von rundgebogenem Edelstahlgewebe/-gitter für eine Stromkollektor-Funktion in Kombination mit wenigstens einer der Elektroden. Auch ausgehend von planaren Elektroden/Edelstahlgeweben kann eine zylindrische Geometrie erzielt werden, insbesondere durch Biegen, zur Konfektionierung der Keramik bzw. eines Keramikzylinder-Grundkörpers. Durch Vorgabe einen Biegeradius kann eine optimale Passform vordefiniert werden (insbesondere eines Edelstahlgewebes), was auch den Zusammenbau erleichtert und die Funktionsweise optimieren kann.

Auch beruht die vorliegende Erfindung dabei auf der Erkenntnis, dass bei der Auswahl des Kathodenmaterials gute katalytische Eigenschaften, eine hohe Oberfläche und hohe elektrische Leitfähigkeit als vorherrschende Eigenschaften sichergestellt sein sollen. Die hier beschriebenen Kathoden (bzw. der entsprechende kathodische Bereich) bestehen vorteilhaft aus zwei Schichten, wahlweise auch aus drei Schichten: Katalysatorschicht, Stromkollektor(schicht), und im Falle einer Luftkathode eine zusätzliche Gasdiffusionsschicht. Die hier beschriebenen Kathoden können insbesondere hinsichtlich guter Kosten-Nutzen-Abwägung auch vergleichsweise dünn ausgestaltet sein/werden, insbesondere in Abhängigkeit von der Anzahl der für die Sauerstoffreduktionsreaktion nutzbaren aktiven Zentren. Vorteilhaft ist auch die Ausgestaltung eines Stromkollektors, insbesondere mit Metallgewebe, anwendungsspezifisch adaptiert, beispielsweise indem Drahtstärke, Biegeradius, Metallmaterialzusammensetzung und dergleichen Randbedingungen vorgegeben werden. Der Stromkollektor kann in die Elektrode eingearbeitet sein (zum Erhalt einer integral-einstückigen Ausgestaltung).

Dabei ist zu verstehen, dass Bakterien und Enzyme zu den biotischen Katalysatoren gehören, während die abiotischen Katalysatoren in Platingruppenmetall-Katalysatoren (PGM-Katalysatoren), platingruppenmetallfreie Katalysatoren (PGM-freie Katalysatoren) und kohlenstoffhaltige Materialien eingeteilt werden können. Diese Katalysatoren können auf kohlenstoffhaltigen oder metallischen Stromabnehmern eingesetzt werden, und können z.B. dieselben Materialeigenschaften aufweisen, die für das Anodenmaterial vorteilhaft sind. Es hat sich im Rahmen der vorliegenden Erfindung gezeigt, dass Aktivkohle bzw. das entsprechend für die hier beschriebene Elektroden konfektionierte kohlenstoffhaltige Material als ein vorteilhafter Katalysator in den hier beschriebenen MET verwendet werden kann, insbesondere da Aktivkohle ORR-aktiv ist (Sauerstoffreduktionsreaktion ORR), eine große Oberfläche aufweist und zu vergleichsweise geringen Kosten bereitgestellt bzw. konfektioniert werden kann. Bisher jedoch wurde der Anwender gemäß dem Stand der Technik bei einer Verwendung von Aktivkohle speziell in stark verschmutzten Abwässern mit sehr schnellem Leistungsabfall insbesondere aufgrund von Salzansammlungen (bzw. mineralische Ablagerungen, Fouling) und Biofilmbildung (relative weiche Ablagerungen, Fouling) auf dem Katalysator und einer/der Gasdiffusionsschicht konfrontiert. Diesen Nachteil überwindet die vorliegende Erfindung, insbesondere auch basierend auf einem Katalysator (bzw. Katalysatorschicht) bestehend aus einem oder zumindest umfassend ein Aktivkohle-Graphit-Leitruss-Gemisch, ferner bevorzugt mit einem Metallgewebe als Stromkollektor. Die Katalysatorschicht kontaktiert möglichst vollflächig die Innenmantelfläche bzw. Innenseite des Keramikseparators. Die Aktivkohle-Materialbasis kann auch als Anode bzw. für die Anode verwendet werden. Das Metallgewebe des Stromkollektors kann z.B. eine Maschenweite im Bereich von 0,1mm bis 0,5mm aufweisen, und/oder eine Drahtstärke kleiner gleich 0,5mm. Dies begünstigt auch eine vorteilhafte Umformbarkeit, insbesondere Biege-/Rollbarkeit zwecks Erhalt einer zylindrischen Geometrie. Beispielsweise besteht das Elektrodenmaterial aus (vergleichsweise steifem) Edelstahl V4A und weist eine Maschenweite im Bereich von 0,1mm und/oder eine Drahtstärke im Bereich von 0,15mm auf. Beispielsweise ist das Metallgewebe in das Elektrodenmaterial eingearbeitet, bzw. umgekehrt, z.B. durch Umformen und/oder Einbetten. Insofern kann auch eine anwendungsspezifische Auslegung im Kontext der Permeabilitäts- und Konfektionierungsanforderungen erfolgen.

Auch ist zu verstehen, dass die Ausgestaltung einer/der Membran-Elektroden-Baugruppe in der Art eines Aufbaus gemäß Anode (außen) - Separator-/Membrankomponente - Kathode (innen) im folgenden Kontext einzuordnen ist und dabei dank erfindungsgemäßer vorrichtungstechnischer Optimierung zu großem Anteil zu der hier beschriebenen langzeitstabilen MET zum Erheben von Messdaten hoher Güte über einen langen Zeitraum beiträgt. Mikrobielle MBZs können in zwei grundlegende Bauformen unterteilt werden: Einkammer-MBZs, bei denen sich beide Elektroden in derselben Arbeitskammer befinden, und Zweikammer-MBZs, bei denen sich Anode und Kathode in einer separaten Arbeitskammer befinden. Die Anoden- und Kathodenkammern sind bei Zweikammer-MBZs häufig durch eine selektive Membran (bzw. Separator) physisch voneinander getrennt, was die Substratverluste verringern und anaerobe Bedingungen in der Anodenkammer aufrechterhalten soll. Ferner sind MBZs in hohem Maße auf den Ionenaustausch zwischen Anode und Kathode angewiesen, da dieser die primäre Antriebskraft für die Elektronen im äußeren Kreislauf darstellt. Ein Separator stellt in funktionaler Hinsicht dementsprechend auch eine wichtige Zwischenphase für den Ionenfluss dar, welcher durch den elektroosmotischen Effekt des elektrischen Feldes der MBZ ausgelöst wird. Eine möglichst kompakte Anoden-Kathoden-Anordnung kann die Gesamtabmessungen verringern, den Innenwiderstand senken und die Leistung der MBZ verbessern.

Typischerweise eingesetzte Kathodenaustauschmembranen (cathode exchange membrane CEM) als Separator/Membran sind jedoch vergleichsweise kostenintensiv (bis zu 60% der Materialkosten der MBZ) und bleiben dabei leider nach wie vor sehr anfällig für Biofouling- und Scaling-Effekte. Ebenso leidet auch die Luftkathode üblicherweise unter Biofouling und Scaling. Es hat sich im Rahmen der vorliegenden Erfindung gezeigt, dass die vorrichtungstechnische Konzipierung der hier beschriebenen Baugruppe Anode - Separator-/Membrankomponente - Kathode eine gute "Produktion" von Katholyt sicherstellen kann, insbesondere bei weiterer Optimierung basierend auf Feuchtigkeitskontrolle, bei Berücksichtigung von Effekten durch elektroosmotischen Sog, welcher auch hinsichtlich Abwaschen von Ablagerungen von der Kathodenoberfläche und Produktion antimikrobieller Wirkstoffe von Bedeutung ist. Flüssiger Katholyt trägt zu guter Langlebigkeit und zur Verhinderung von Biofouling, Scaling und etwaiger Verblockung der Kathode bei. Vorteilhaft wird ein Feuchthalten der Kathode durch gebildeten Katholyt auf kontinuierliche Weise sichergestellt, zum Vorbeugen eines Austrocknens und zum Abtransport von Salzen sowie zur Verhinderung von Biofouling an der Luftkathode (insbesondere dank antimikrobieller Eigenschaft des Katholyt).

Insbesondere hinsichtlich optimaler Betriebsbedingungen ist zu verstehen, dass ein Feuchthalten des Innenraums von Keramik und Kathode einerseits einen vorteilhaften Wasserhaushalt bezüglich der Kathode sicherstellt (H+-Ionen für Sauerstoffreduktionsreaktion, O2 + e⁻ + H⁺ → H2O), andererseits den Vorgang des Abtransports von Salz optimieren kann, zur effektiven Vermeidung ungewollter mineralischer Ausfällungen. Stöchiometrisch und ladungsmäßig ausgeglichen lautet die Reaktion: O2 + 4H⁺ + 4e⁻ → 2H2O Dabei weist gebildeter Katholyt antibakterielle Eigenschaften auf (hoher pH-Wert, zum Teil >10, hohe Leitfähigkeit), was auch das Ausbilden eines Biofilms auf der Kathode effektiv unterbinden kann, wodurch wiederum die Langzeitstabilität verbessert werden kann. Das Feuchthalten der Kathode durch gebildeten Katholyt liefert demnach vorteilhafte Langzeitbetriebsbedingungen. Dabei ist ein ständiges Feuchthalten nicht notwendigerweise erforderlich, sondern kann bereits auf konstruktive Weise und/oder die betriebsbedingte Bildung von Katholyt begünstigt oder sogar sichergestellt werden, gegebenenfalls unterstützt durch zusätzliche Maßnahmen wie Feuchtigkeitsspeicher (siehe dazu die hier erwähnten Maßnahmen bezüglich Hydrogelen). Die erfindungsgemäße Ausgestaltung kann demnach auch sicherstellen, dass der gebildete Katholyt den Sauerstoff in gelöster Form bereitstellt, so dass vergleichsweise wenig Sauerstoff an die Anode gelangen kann (im Vergleich zu Luftsauerstoff-Konditionen), so dass auch ein anaerobes Milieu im anodischen Biofilm aufrechterhalten werden kann.

Weiterführende Untersuchungen zur vorliegenden Erfindung haben dabei auch aufgedeckt, dass eine vergleichsweise weiche nachgiebige Elektrodenmaterial-Konfektionierung, vorteilhaft auch hydrophile Konfektionierung, insbesondere in Ausgestaltung als Filz, zu vergleichsweise guten Ergebnissen führt. Insbesondere kann bevorzugt mittels Filzelektroden (sowohl an Anode als auch an Kathode) sichergestellt werden, dass ein besonders umfangreicher flächiger Kontakt mit der entsprechenden Keramikseparatormantelfläche geschaffen ist/wird. Eine derartige vergleichsweise weiche nachgiebige Elektrodenmaterial-Konfektionierung, insbesondere Filzkonfektionierung, liefert auch in feuchtem Milieu vergleichsweise gute Kontaktierungseigenschaften. Auch kann der vorrichtungstechnische Aufbau basierend auf wenigstens einer derart beschaffenen Elektrode weiter vereinfacht werden. Nicht zuletzt werden nochmals spürbarere Kostenvorteile realisierbar.

Gemäß einem Ausführungsbeispiel definiert die Keramik zumindest teilweise auch einen/den Reaktorkörper (Chassis) der messtechnischen mikrobiellen elektrochemischen Technologieeinheit. Dies kann auch den konstruktiven Aufbau weiter vereinfachen und eine besonders robuste und dichte Struktur bereitstellen, bei guter Stabilität selbst dann, wenn die Keramik vergleichsweise dünn ausgestaltet ist. Auch mag die Ausgestaltung zumindest von Teilen des Chassis aus Keramik dazu beitragen, dass der Feuchtehaushalt im Bereich der Kathode optimiert werden kann. Dabei ist zu verstehen, dass auch eine flächig-planare Ausgestaltung der Keramik realisierbar ist, jedoch im Vergleich zu einer zylindrischen Ausgestaltung Nachteile wie z.B. das Abdichten/Verbinden an winkeligen Abschnitten mit sich bringt. Zumindest insofern dürfte die zylindrische Ausgestaltung als vorteilhafter angesehen werden können. Auch ist zu verstehen, dass ein Chassis nicht notwendigerweise vorliegen muss, d.h., die Keramik kann auch lediglich im Wesentlichen die Membran(en) bilden. Gleichwohl sind Ausgestaltungen möglich, bei welchen die Keramik topfartig oder sacklochartig nicht nur die Membran(en) bildet, sondern auch einen Bodenbereich, an welchem jedoch nicht notwendigerweise eine Membranfunktion erfüllt wird. Somit kann die Keramik auch eine Art Chassis oder eher rein strukturelle Komponente bilden bzw. ergänzend bereitstellen. Ein/das Chassis oder zumindest Teile eines/des Chassis müssen nicht notwendigerweise aus Keramik ausgestaltet sein, jedoch liefert chemische/biologische Stabilität von Keramik auch im Kontext eines/des Chassis insbesondere bei Langzeiteinsatz gute Vorteile.

Gemäß einem Ausführungsbeispiel definiert die Keramik wenigstens einen zwischen kathodischem und anodischen Bereich wirksamen Bestandteil oder eine entsprechende Komponente einer/der Membran-Elektroden-Baugruppe der messtechnischen mikrobiellen elektrochemischen Technologieeinheit. Dies begünstigt auch die gewünschte vorrichtungstechnische flächige Implementierung der Keramik zwischen zu untersuchendem Fluid und kathodischem Bereich/Kompartiment.

Gemäß einem Ausführungsbeispiel sind die Materialeigenschaften der (Ton-)Keramik zumindest hinsichtlich Porosität definiert, insbesondere in einem Bereich von 23% bis 30% (Mittelwert), und wahlweise auch hinsichtlich Materialstärke (Dicke, insbesondere radiale Dicke), z.B. 7mm oder 8mm. Die Porosität kann als besonders relevanter Parameter der Keramik erachtet werden, insbesondere auch hinsichtlich stabiler Langzeit-Betriebsbedingungen. Der Fachmann kann basierend auf der vorliegenden Offenbarung auch anwendungsspezifisch Porositätsbereiche definieren, wahlweise auch in Abhängigkeit von der Keramik-Materialstärke. Dabei können auch weitere Konfektionierungsvorgaben angegeben oder Toleranzangaben vorgegeben werden, z.B. betreffend eine zylindrische Rundheit.

Beispielsweise liegt die Porosität der Keramikmembran im Bereich von 21% bis 25%, insbesondere die offene Porosität ermittelt mittels Wasserabsorptionsmethode. Beispielsweise liegt die Wasseraufnahme der Keramik im Bereich von 11% bis 15% (Mittelwert), insbesondere 11% bis ca. 14% (Mittelwert) oder 11% bis 13%.

Gemäß einem Ausführungsbeispiel ist die Keramik unglasiert. Dies kann insbesondere im Kontext mit der Funktionsweise von MET vorteilhafte messtechnische Eigenschaften sicherstellen und einem aus dem Stand der Technik bekannten potentiell auftretenden Scaling-Effekt besonders effektiv vorbeugen.

Gemäß einem Ausführungsbeispiel ist die wenigstens eine Keramik bei geometrisch zumindest annähernd zylindrischem Aufbau zwischen Anode und Kathode einer/der Membran-Elektroden-Baugruppe der wenigstens einen messtechnischen mikrobiellen elektrochemischen Technologieeinheit angeordnet, beispielsweise vollumfänglich in flächiger Anlage (insbesondere mechanisch vorgespannt) sowohl an Kathode als auch an Anode. Dies liefert nicht zuletzt auch einen besonders einfachen, robusten und auch dichten vorrichtungstechnischen Aufbau, wobei die Keramik auch zumindest teilweise ein/das Chassis (Reaktorkörper) der MET bilden kann. Die großflächige Kontaktierung begünstigt dabei auch einen vollständigen Ersatz von gemäß Stand der Technik recht kostspieligem Material bzw. Werkstoffen durch die hier beschriebene Keramik, insbesondere auch hinsichtlich Elektrodenmaterial.

Es ist zu verstehen, dass die messtechnische Vorrichtung ohne räumlich vordefinierten messtechnisch wirksamen Bereich zur zumindest zeitweisen Aufnahme oder zum Durchleiten von zu untersuchendem Fluid auskommt. Anders ausgedrückt: die messtechnische Vorrichtung muss nicht notwendigerweise ein Kompartiment für das zu untersuchende Fluid definieren, sondern kann z.B. in das zu untersuchende Wasser/Abwasser/Gewässer (Fluid) eingetaucht werden, und das zu untersuchende Fluid liegt von außen um das anodische Kompartiment an (bzw. liegt in einem anodischen Bereich vor), wahlweise bei umgekehrtem/umkehrbarem Messprinzip. Sofern sich das zu untersuchende Fluid in einem vordefinierten Gefäß, Becken, Rohr oder dergleichen befindet oder dort hindurchfließt, lässt sich zwar auch von einem räumlich vordefinierten anodischen Kompartiment sprechen, andernfalls eher nur von einem anodischen Bereich oder anodischen Abschnitt. Wahlweise kann auch insoweit ein anodisches Kompartiment gebildet werden, als die Keramik in radialem Abstand von einer Art Gitter oder dergleichen Schutzhüllkurve umgeben ist, z.B. um harte Gegenstände (z.B. Äste oder dergleichen Treibgut, insbesondere bei Anwendung in Fließgewässern) davon abzuhalten, die Anode oder deren etwaige Einhausung zu kontaktieren. Auch in diesem Kontext ist erwähnenswert, dass das hier beschriebene messtechnische Prinzip auch umgekehrt betrieben bzw. genutzt werden kann, d.h., die vorliegend beschriebene MET ist wahlweise auch für eine Bestimmung des O2-Gehalts implementierbar.

Gemäß einem Ausführungsbeispiel umfassen die Elektroden einer/der Membran-Elektroden-Baugruppe der wenigstens einen messtechnischen mikrobiellen elektrochemischen Technologieeinheit wenigstens eines der folgenden Materialien: kohlenstoffbasierter Kohlefaservlies/-schleier, Graphitfilz, Aktivkohlefaserfilz, jeweils für die Anode, und/oder Aktivkohle-Graphit-Leitruss-Gemisch, Aktivkohlefaserfilz, Graphitfilz, granulierte Aktivkohle, jeweils für die Kathode. Es ist zu verstehen, dass Leitruss in der vorwiegend englischsprachigen Fachliteratur auch als Carbon Black bezeichnet wird.

Vorteilhaft weist wenigstens eine der Elektroden eine weiche nachgiebige Konfektionierung auf, vorteilhaft auch eine hydrophile Konfektionierung. Dies begünstigt auch ein vorteilhaft konstantes Langzeitverhalten der Membran-Elektroden-Baugruppe insbesondere in Hinblick auf die Stromaufnahme- bzw. Stromweiterleitungs-Charakteristik.

Dabei kann wahlweise ein Stromkollektor (z.B. Edelstahlgewebe oder Kohlefaservlies) und/oder optional auch eine Gasdiffusionsschicht (z.B. PTFE) für die Kathode vorgesehen sein/werden.

Der Fachmann kann basierend auf der vorliegenden Offenbarung anwendungsspezifisch erkennen, inwiefern oder ob die jeweilige Elektrode vorteilhaft eine bestimmte Konfektionierung aufweisen soll und/oder durch Zugabe von Additiven anwendungsspezifisch optimiert werden kann. Insbesondere für die Kathodenkonzipierung ist es vorteilhaft, auf am Markt verfügbare "Rohlinge" zurückzugreifen und diese anwendungsspezifisch weiterzuentwickeln, insbesondere in Hinblick auf die hier beschriebene Baugruppe umfassend den Keramikseparator bzw. die Keramikmembran.

Gemäß einem Ausführungsbeispiel stellt die messtechnische Vorrichtung eine messtechnische Langzeitstabilität (insbesondere langzeitstabil hinsichtlich Drift eines Leistungsdichte-Messwerts bzw. energetischer Kenngrößen wie Leistung, Strom, Zellspannung bzw. entsprechender Messdaten) von mindestens zwei Monaten sicher, bevorzugt von mindestens einem Jahr, insbesondere indem die wenigstens eine Keramik auch wirksam ist gegen (Salz- und/oder Biofilm-)Ablagerung insbesondere an der Kathode. Diese besonders lange Langzeitstabilität begünstigt ein sehr genaues, datentechnisch belastbares Analysieren von eher langwierigen Prozessen wie z.B. der Abwasseraufbereitung und eröffnet auch neue Möglichkeiten insbesondere hinsichtlich selbstlernender Algorithmen zur Datenauswertung. Beispielsweise können die langzeitstabil über einen Zeitraum von z.B. mindestens drei Monaten erhobenen Daten durch ein KI-Modell bzw. durch wenigstens einen ML-Algorithmus im Kontext von oder basierend auf einem Multi-Layer-Perzeptron bzw. MLP-Regressor (auch als künstliches neuronales Netz) ausgewertet werden, insbesondere um die ausgewerteten Daten auf exaktere Weise mit Prozessparametern korrelieren zu können, insbesondere im Kontext mit der Ermittlung eines vergleichsweise allgemeinen CSB-Parameters. Die Implementierung eines MLP-Regressors kann dabei insbesondere im Kontext der hier beschriebenen messtechnischen Aufgabenstellungen auch den folgenden technischen Effekt liefern: besonders effektive Behebung deterministischer Mängel zur Ermöglichung einer Parameterkorrelation insbesondere auch bei Messdatenerhebung über mehrere Monate oder Jahre. Dabei ist die Keramik insbesondere im Verbund mit den Elektroden und einem bevorzugt rein vorrichtungstechnisch sichergestellten Feuchthalten wirksam gegen Ablagerungen (insbesondere Ausfällung von Salzen durch Evaporation) und Fouling, insbesondere über viele Monate langzeitstabil, so dass die Daten für eine bevorzugt KI-gestützte Auswertung und gegebenenfalls für ein KI-Training bzw. eine ML-Maßnahme mit besonders hoher Güte bereitgestellt werden können. Dies kann insbesondere auch durch Abstimmung von Materialparametern wie Dicke, Porosität und/oder Materialauswahl und Konfektionierung der Elektroden und/oder etwaiger Zwischenschichtwirkmaterialien wie z.B. Hydrogelen optimiert werden.

Somit kann das vorliegende erfinderische Konzept ausgehend von vorrichtungstechnischen Optimierungen im Kontext der Messdatenerhebung auch noch vorteilhafter im Zusammenhang mit KI-gestützter Auswertung und ML-basiertem Training implementiert sein/werden, so dass z.B. auch eine stabile Langzeit-Überwachung von Anlagen und Prozessen auch im industriellen Maßstab vorteilhaft umgesetzt werden kann, wahlweise auch in Kombination mit einer Prozessteuerung/- regelung basierend auf den wie hier beschrieben ausgewerteten Messdaten.

Es ist zu verstehen, dass die hier beschriebenen Ansätze zur Implementierung von ML-Algorithmen bevorzugt auf Regressor-Modelle abzielen, weniger auf Klassifikationsmodelle. Gleichwohl kann z.B. auch im Kontext einer Klassifikation von (Ab-)Wasser-Typen wenigstens ein KI-Klassifikationsmodell implementiert sein/werden, z.B. zwecks leichterer Zuordnung von vordefinierten/vordefinierbaren Parametersets zu einzelnen Klassen oder Oberbegriffen von zu untersuchenden (Ab-)Wässern, sinngemäß zwecks leichterer Zuordnung von Grundkalibrierungs-Varianten zu spezifischen Anwendungsfällen. Bei sehr grober Betrachtungsweise ist z.B. eine Klassifizierung zumindest in die Typen kommunales Abwasser und industrielles Abwasser zweckdienlich, wobei jeder Typ freilich weiter unterteilt werden kann. Dabei kann offenbleiben, ob basierend auf mehreren Inputs zu einem Output gelangt wird, oder vice versa.

Eine Klassifizierung kann auch mittels bzw. basierend auf ML-Zuständen definiert sein/werden, beispielsweise in der Art einer Ampel (grün: alles in Ordnung, gelb: möglicherweise Betriebsstörungen, rot: Betriebsstörungen erwartet).

Auch ist zu verstehen, dass die hier beschriebenen Ansätze zur Implementierung von ML-Algorithmen jeweils spezifisch hinsichtlich einer jeweiligen Stofffraktion implementiert sein/werden können, insbesondere jeweils umfassend ein künstliches neuronales Netz (NN) oder beruhend auf einem NN.

Es ist zu verstehen, dass die Implementierung von KI-Modellen im Rahmen der vorliegenden Erfindung eine computertechnische Infrastruktur bzw. Datenverarbeitungs-Architektur insbesondere auch im Kern wenigstens einer Recheneinheit umfassen kann, welche die Ausführung von ML-Algorithmen auch zentral direkt in/auf der Hardware erleichtert, leistungsfähiger macht bzw. beschleunigt (bis hin zu Echtzeit-Verarbeitung) und/oder energieeffizienter macht oder zumindest teilweise überhaupt auch erst ermöglicht. Beispielsweise umfassen die hier beschriebenen computertechnischen und chipbasierten Mittel wenigstens eine der folgenden Komponenten: photonische KI-Chips insbesondere mit Silizium-Photonik-Strukturen (Kombination elektronischer und optischer Datenverarbeitungsprozesse), optische Wellenleiter zumindest teilweise anstelle von oder zumindest in Ergänzung zu elektronischer Halbleiter, sowie Multiplexing-Komponenten, Photonenmodulatoren, Fotodetektoren, Ringresonatoren, wenigstens eine Dense Wavelength Division Multiplexing (DWDM)-Komponente zur simultanen Abarbeitung mehrerer Datenkanäle, wenigstens einen optischen Schaltkreis integriert in wenigstens ein neuronales Netz (NN oder DNN, deep neural network), wenigstens einen photonischen Prozessor. Beispielsweise wird wenigstens ein NN und/oder DNN direkt auf Hardwareebene ausgeführt. Im Effekt können insbesondere auch besonders große Datenmengen besonders schnell analysiert werden. Beispielsweise liegt dabei wenigstens ein photonisches Bauteil in direkt auf einen Wafer gedruckter Form vor, insbesondere wenigstens eines der folgenden photonischen Bauteile: optische Verstärker, photonische Schaltungen (PICs), Polarisationswandler, Splitter, Lichtwellenleiter, Splitter, Phasenmodulatoren.

Es ist zu verstehen, dass gemäß dem Stand der Technik potentiell sämtliche für die messtechnische Auswertung relevante Parameter von Salz-/Biofilmablagerung betroffen bzw. beeinflusst sein können, vermutlich an erster Stelle die Leistungsdichte bzw. energetische Kenngrößen im Allgemeinen (Leistung, Strom, Zellspannung). Auch die Leerlaufspannung (OCV) wird bei vorbekannten Vorrichtungen sukzessiv durch Scaling beeinträchtigt. Die hier beschriebenen erfindungsgemäßen Maßnahmen insbesondere im Kontext der Implementierung der Keramik ermöglichen hingegen einen langzeitstabilen Betrieb über viele Monate, wohingegen gemäß dem Stand der Technik mit einer Abnahme z.B. der Leistungsdichte bereits nach ca. einem Monat im Bereich von ca. 50-75% gerechnet werden muss.

Gemäß einem Ausführungsbeispiel ist die wenigstens eine messtechnische mikrobielle elektrochemische Technologieeinheit als rohrförmiger Sensor ausgestaltet, welcher einen über einen Deckel zugänglichen kathodischen Bereich (bzw. kathodisches Kompartiment) aufweist, wobei der Deckel bevorzugt in eine Einhausung übergeht, welche die wenigstens eine Keramik und die Anode und Kathode bzw. das kathodische Kompartiment umschließt. Diese Ausgestaltung liefert Vorteile insbesondere auch für Abwasseranalytik-Anwendungen, insbesondere auch in Fließgewässern. Eine solche Einhausung ragt z.B. längs der Keramik bis zur Anode.

Gemäß einem Ausführungsbeispiel fungiert die Keramik als Separator zwischen Anode und Kathode. Dies liefert nicht zuletzt auch einen vergleichsweise einfachen und robusten Aufbau, der auch auf einfache Art und Weise (größen-)skaliert werden kann.

Gemäß einem Ausführungsbeispiel weist die messtechnische mikrobielle elektrochemische Technologieeinheit eine Membran-Elektroden-Baugruppe auf, bei welcher die Keramik als Membran in radial zugkraftvorgespannter Anordnung mit der Anode vorliegt und/oder bei welcher die Keramik als Membran in radial druckkraftvorgespannter Anordnung mit der Kathode vorliegt, insbesondere derart dass eine Relativbewegung zwischen entsprechender Elektrode und Keramik unterbunden ist, bei vordefiniert gleichbleibendem Mantelflächenkontaktanteil. Dies erleichtert auch ein Sicherstellen gleichbleibender Betriebsrandbedingungen und damit auch den Feuchtehaushalt über eine lange Betriebsdauer von mindestens einigen Monaten.

Gemäß einem Ausführungsbeispiel ist die messtechnische Vorrichtung eingerichtet zum Erfassen und wahlweise auch Dokumentieren (Monitoring) der mikrobiellen Aktivität, insbesondere über die Kohlenstoffverbrauchsrate. Dies kann insbesondere auch die Prozesssteuerung bei Wasseraufbereitung erleichtern.

Gemäß einem Ausführungsbeispiel ist die messtechnische Vorrichtung eingerichtet zum Generieren des wenigstens einen energetischen/elektrochemischen (Zell-)Parameters basierend auf Modulation, insbesondere Pulsmodulation, speziell auch Pulsdauermodulation des externen elektrischen Lastwiderstandes. Die Modulation kann nicht zuletzt auch das Bereitstellen eines vorteilhaft auswertbaren Messdatenverlaufs sicherstellen und eine gute bzw. noch besser belastbare Datengrundlage für etwaige nachgelagerte Datenauswerteschritte liefern.

Insbesondere im Vergleich zu gängigen elektrochemischen Methoden (elektrochemische Impedanz-Spektroskopie, zyklische Voltametrie), bei welchen ein Wechselstrom auf die Zelle angelegt wird (mit entsprechender Einwirkung auf den elektroaktiven Biofilm), ermöglicht die hier beschriebene Modulation eine nicht-disruptive modellgestützte Parameterbestimmungsroutine, wobei für die hier beschriebene Parameterbestimmung bzw. Korrelation kein Potentiostat benötigt wird, demnach auch keine Eingriffe in den Strom-/Spannungsverlauf erfolgen, und auch eine gute messtechnische Reaktionszeit sichergestellt werden kann (sowohl was die sensorische Ansprechzeit als auch die Messdatenauswertezeit betrifft). Anders ausgedrückt: es wird bei der bzw. durch die Messung kein Einfluss auf die Mikrobiologie genommen, und eine Vor-/Nachbereitungszeit oder spürbare Auswertezeitdauer braucht nicht eingeplant werden. Beispielsweise kann die Analyse auch in zeitlicher Hinsicht sehr schnell zum insbesondere für einen Steuerungs-/Regelungsvorgang verwendbaren Messergebnis führen, z.B. im Kontext einer biologischen Reinigungsstufe (insbesondere Belebungsbecken)..

Gemäß einem Ausführungsbeispiel ist der wenigstens eine Mikrocontroller der messtechnischen Vorrichtung eingerichtet, eine Modulation, insbesondere Pulsmodulation, speziell auch Pulsweitenmodulation ohne PC-Anbindung zu realisieren. Dies begünstigt auch eine sensorpunktspezifische Implementierung mit sensorpunktspezifisch bereitstellbaren Messdaten hoher Güte. Als Sensorpunkt ist hier ein Messpunkt in örtlicher Hinsicht zu verstehen. Standardmäßig mag die hier beschriebene messtechnische Vorrichtung einen einzigen Sensorpunkt definieren bzw. für einen einzigen Sensorpunkt vorgesehen/ausgestaltet sein, wahlweise jedoch können auch mehrere Sensorpunkte durch eine einzige messtechnische Vorrichtung abgegriffen werden.

Gemäß einem Ausführungsbeispiel weist die messtechnische Vorrichtung ein Kommunikationsmodul eingerichtet zur drahtlosen Übermittlung von generierten Zellparametern auf. Die drahtlose Ausgestaltung einer Datenübermittlung, vorteilhaft in Kombination mit energetischer Autarkie, eröffnet weitere Optionen zur Anordnung/Implementierung von Sensorpunkten sowie zur Ausgestaltung von vergleichsweise komplexen messtechnischen Systemen umfassend eine Vielzahl von Sensorpunkten bzw. messtechnischen Vorrichtungen und etwaiger ergänzender Sensorik.

Gemäß einem Ausführungsbeispiel ist die messtechnische Vorrichtung eingerichtet, die aus den erhobenen Messdaten, insbesondere Messdaten aus zellinternen Widerstandskomponenten und/oder Spannungs-, Kapazitäts-, Strom-, Leistungs-, und/oder elektrischen Ladungsmessdaten, basierend auf Modulation, insbesondere Pulsmodulation, speziell auch Pulsweitenmodulation des externen elektrischen Lastwiderstandes generierten energetischen/elektrischen (Zell-)Parameter basierend auf wenigstens einem KI-Modell bzw. wenigstens einem ML-Algorithmus auszuwerten, insbesondere mittels eines/des in die messtechnische Vorrichtung integrierten Mikrocontrollers ohne PC-Anbindung. Dies begünstigt nicht zuletzt auch eine vergleichsweise einfache zahlenmäßige Skalierung von Sensorpunkten auch in vergleichsweise komplexen messtechnischen Systemen, bei maximaler Flexibilität hinsichtlich eines jeweiligen Sensorpunktes.

Es ist zu verstehen, dass die hier beschriebene Modulation das Generieren einer vergleichsweise hohen Anzahl von Parametern ermöglicht (insbesondere >= sechs Parameter), die wahlweise auch als Eingangsgrößen in ein ANN eingebracht werden können. Es hat sich im Rahmen der vorliegenden Erfindung gezeigt, dass hierdurch besonders effektiv etwaige Interferenzen aufgedeckt werden können, wodurch die Messung bzw. Messdatenauswertung präziser und sensitiver ausgestaltet werden kann.

Sofern gemäß der vorliegenden Offenbarung von Modulation, insbesondere Pulsmodulation, speziell auch Pulsweitenmodulation bzw. Pulsdauermodulation gesprochen wird, so ist darunter synonym eine entsprechend wirkende signaltechnische Maßnahme betreffend erfasste Messsignale insbesondere unter Bezugnahme auf ein Trägersignal zu verstehen. Speziell als Pulsmodulation ist dabei eine Umwandlung kontinuierlicher analoger Signale in zeitdiskrete Signalfolgen aus Einzelpulsen zu verstehen. In der englischsprachigen Fachliteratur ist auch der Begriff "transient response analysis" zu finden. Vorteilhaft werden zumindest die Spannungssignale moduliert (Auswerten der Widerstandsänderungen als Funktion der Frequenz), wahlweise auch der Stromfluss insbesondere zwecks Bestimmung der Ladung (Bezugnahme auf die Stromkurve).

Gemäß der vorliegenden Offenbarung wird bei Gleichungen und Kennzahlen sowohl die Schreibweise mit tiefgestellten Bezugnahmen als auch die Schreibweise mit Unterstrich genutzt; diese Schreibweisen werden hier als gleichbedeutend erachtet. Beispiel: CCRₜₒₜ bzw. CCR_tot. Ebenso wird hier bei Elementen oder Molekül-Bezeichnungen wie z.B. für Sauerstoff sowohl die hintenangestellte Schreibweise als auch die tiefgestellte Schreibweise gleichbedeutend verwendet. Beispiel: O2 bzw. O₂.

Gemäß einem Ausführungsbeispiel ist die messtechnische Vorrichtung eingerichtet zum Korrelieren der generierten Zellparameter mit dem wenigstens einen Prozessparameter (P_{SB}) unter Berücksichtigung wenigstens einer Randbedingung aus der folgenden Gruppe: Leitfähigkeit, Temperatur, pH-Wert; insbesondere in/für einem/n (Ab-)Wasseraufbereitungsprozess. Dies ermöglicht nicht zuletzt auch eine Steigerung der Genauigkeit bzw. Belastbarkeit der Messdaten.

Es ist zu verstehen, dass bei der Prozessparameterbestimmung auch Randbedingungen insbesondere aufgrund von Umwelteinflüssen oder externen Effekten Berücksichtigung finden können, insbesondere die Leitfähigkeit, die Temperatur, der pH-Wert. Derartige Größen können auch bei der Bestimmung zellinterner Parameter berücksichtigt werden. Vorteilhaft werden zumindest einige dieser Größen, je nach Anwendung, auch in einem für die Datenanalyse im Rahmen der vorliegenden Erfindung implementierbaren KI-Modell berücksichtigt, insbesondere um deren (potentiellen) Einfluss basierend auf selbstlernenden Algorithmen zu berücksichtigen. Beispielweise kann der Ohm'sche Widerstand der Zelle nach dem Prinzip eines Leitfähigkeitssensors über die Zellkonstante mit der elektrischen Leitfähigkeit korreliert werden. Dabei ist zu verstehen, dass ein/das implementierbare/s KI-Modell auch als ein bereits vortrainiertes KI-Modell ausgestaltet sein kann, insbesondere indem wenigstens ein ML-Algorithmus im Rahmen einer (Grund-)Kalibrierung angewandt ist/wird.

Gemäß einem Ausführungsbeispiel ist die messtechnische Vorrichtung eingerichtet zum Generieren des wenigstens einen Zellparameters binnen einer Zeitspanne von maximal fünf Minuten, bevorzugt maximal zwei Minuten. Eine derart kurze Reaktionszeit liefert auch den Vorteil hoher Reaktivität z.B. bei Anwendungen in Fließgewässern.

Es ist zu verstehen, dass die erreichbare (möglichst kleine) Zeitspanne insbesondere von der Zellkapazität und einem Pause-Zeitfenster der MET zum Zurückkehren wieder in einen Ausgangszustand abhängt. Insbesondere ist die vergleichsweise kurze Reaktionszeit auf die hier beschriebene Art und Weise der Ermittlung der elektrochemischen Parameter zurückzuführen, in Abgrenzung zu typischerweise leicht messbaren Spannungs-/Strommessgrößen (bei welcher Messung gemäß dem Stand der Technik jedoch kapazitive Einflüsse auftreten: die Kapazität reagiert exponentiell, also recht langsam, auf Änderungen, mit entsprechenden Verzögerungen in den Signaländerungen, so dass bisher eher mit einer Mess-/Auswertedauer im Bereich von Stunden bis zum Erreichen eines messtechnischen Plateaus bzw. belastbaren Endwerts gearbeitet werden musste). Im Rahmen der vorliegenden Erfindung konnte eine bereits sehr kurze Zeitspanne realisiert werden, wobei erwartungsgemäß weiteres zeitliches Optimierungspotential ausgeschöpft werden kann. Insbesondere im Kontext des (Ab-)Wassermonitorings (Analyse) ist die erfindungsgemäß erzielbare Zeitspanne bereits als ausreichend kurz zu erachten, denn eine Abwassermatrix (Zusammensetzung) ändert sich erwartungsgemäß deutlich langsamer. So kann bereits von einer quasi-kontinuierlichen Messung gesprochen werden, welche derart reaktiv ist, dass auch vergleichsweise schnelle Änderungen des zu untersuchenden Fluids erfasst und als prozessuale Steuerungsvorgabe genutzt werden können.

Gemäß einem Ausführungsbeispiel ist die messtechnische Vorrichtung eingerichtet zum Korrelieren wenigstens eines Messwerts mit einem Prozessparameter (P_{SB}) für einen/den chemischen Sauerstoffbedarf (CSB), insbesondere eines zu optimierenden (Ab-)Wasseraufbereitungsprozesses, insbesondere indem eine Korrelation mit dem momentan durch die MET erzeugten Biostrom über ein Elektronenäquivalent und/oder ein Stromäquivalent erfolgt. Insbesondere können der Aktivierungswiderstand, die Zellkapazität, die Leerlaufspannung, die Stromdichte, die Leistungsdichte, die elektrische Ladung und/oder der ohmsche Widerstand insbesondere bei organischen Ionen (insbesondere Acetaten) jeweils mit dem CSB korreliert werden, wahlweise auch unter Einbezug der Kohlenstoffverbrauchsrate (CCR). Hierdurch kann einerseits eine für weitere Auswerteschritte wertvolle Datengrundlage geschaffen werden, andererseits kann auch die Bereitstellung von vergleichsweise leicht zu handhabbaren Parametern für z.B. Anlagenbetreiber erfolgen.

Gemäß einem Ausführungsbeispiel ist die messtechnische Vorrichtung eingerichtet zum Korrelieren wenigstens eines Messwerts mit einem Prozessparameter (P_{SB}), insbesondere CSB, z.B. BCOD, unter Bezugnahme auf den biologischen Sauerstoffbedarf (BSB), insbesondere eines zu optimierenden (Ab-)Wasseraufbereitungsprozesses, insbesondere indem von BSB auf CSB rückgeschlossen wird, bevorzugt nach einer messtechnischen Auswertezeit von deutlich weniger als fünf Tagen, bevorzugt maximal drei Tagen. Dabei kann auch eine Analyse vom Speziellen ins Allgemeine implementiert werden. Eine bisher gemäß Stand der Technik über eine Zeitdauer von fünf Tagen durchgeführte BSB5-Messung kann demnach deutlich schneller abgebildet werden, wodurch auch eine wertvolle (Mess)Datengrundlage für Steuerungs-/Regelungsprozesse geschaffen werden kann. Anders ausgedrückt: Über ein Zeitfenster von z.B. einem Monat können deutlich mehr zeitpunktbezogene Messwerte ermittelt werden als bisher, z.B. 15 bis 30 mal. Eine Steuerung/Regelung kann demnach auch deutlich reaktiver ausgestaltet werden, was prozessuale Optimierungsmaßnahmen vereinfacht bzw. effizienter werden lässt.

Es ist zu verstehen, dass eine Korrelation oben genannter Messwerte mit dem vergleichsweise allgemeinen CSB auf komplexere Weise realisiert werden kann (bzw. muss) als mit dem BSB, insbesondere da beim BSB lediglich der biologisch abbaubare Anteil des CSB erfasst wird. Gleichwohl ist zu verstehen, dass die hier beschriebene Analytik gleichermaßen im Kontext von BSB, CSB, Kohlenstoffverbrauchsrate und Stofffraktionen flüchtiger Fettsäuren stehen mag, insbesondere da im Rahmen der vorliegenden Erfindung erkannt wurde, dass jeweils vergleichsweise leicht konkretisierbare Abhängigkeiten genutzt werden können, um sowohl eine erste Gruppe (z.B. BSB) als auch eine andere/weitere Gruppe (z.B. CSB) von Prozessparametern zu bestimmen, beispielsweise auch auf bilanzielle Weise den so genannten biodegradierbaren chemischen Sauerstoffbedarf (BCOD bzw. "biodegradable COD"), entsprechend dem CSB bezüglich allein der nicht-inerten Anteile des CSB. Anders ausgedrückt: Nur gelösten Fraktionen können die Zellmembran der Bakterien durchdringen; inerte, biologisch-nicht abbaubare Fraktionen können üblicherweise nicht bestimmt werden; somit werden die gelösten Stofffraktionen des CSB gemessen, beispielsweise vornehmlich VFAs, insbesondere Acetat(e). Die vorliegende Erfindung basiert daher auch auf dem Konzept, die Datengüte dadurch zu maximieren, dass der CSB möglichst detailliert aufgeschlüsselt wird. Inerte, biologisch-nicht abbaubare Fraktionen können über Bilanzen ermittelt werden. Beispiel:
COD (total, gemessen) = 500mg/L; COD (biodegradierbar) = 300mg/L
Bilanz: COD (nicht-biodegradierbar): 500 - 300 = 200 [mg/L]

Gemäß einem Ausführungsbeispiel ist die messtechnische Vorrichtung eingerichtet zur CSB-Fraktionierung, insbesondere zur messtechnisch basierten Durchführung einer Echtzeit-Fraktionierung des CSB.

Soweit gemäß der vorliegenden Offenbarung von Echtzeit gesprochen wird, so ist darunter auch eine Zeitspanne von ein bis drei Minuten zu verstehen, welche insbesondere im Kontext großtechnisch skalierter Wasseraufbereitungsprozesse mit deren entsprechender Trägheit als eine Nahezu-Echtzeit-Analytik betrachtet werden kann. Begrifflich ist der Ausdruck "Echtzeit" dabei kontextbezogen zu verstehen: eine kontinuierliche Messung (z.B. für die CCR-Bestimmung, insbesondere unter kontinuierlicher Bezugnahme auf momentane Stromwerte) mittels der hier beschriebenen messtechnischen Ausstattung ist zumindest theoretisch innerhalb von Millisekunden realisierbar, und die Auswertung von modulierten Messdaten zwecks Parameterbestimmung bei z.B. CSB-/BSB-Analysen mag aufgrund von Systemträgheit eine Dauer im Bereich von einigen wenigen Sekunden bis zu einer halben Minute erfordern, dürfte jedoch im Kontext von z.B. Prozesssteuerung/-regelung vergleichsweise träger Wasseraufbereitungsprozesse ebenfalls noch als "in Echtzeit" bezeichnet werden können.

Gemäß einem Ausführungsbeispiel ist die messtechnische Vorrichtung eingerichtet zum Korrelieren wenigstens eines Messwerts mit einem Prozessparameter (P_{SB}), insbesondere CSB, z.B. BCOD, unter Bezugnahme auf Stofffraktionen flüchtiger Fettsäuren (volatile fatty acids, VFA, bei Raumtemperatur flüssig und flüchtig; Acetate, Propionate, Butyrate, Isobutyrate, Valerate, Isovalerate, Capronate), insbesondere indem wenigstens ein Typ VFA (insbesondere Acetate) direkt über den Stromfluss gemessen bzw. bestimmt wird und bei der Auswertung bzw. Korrelation weiterer Prozessparameter berücksichtigt wird. Dies erweitert nicht zuletzt die Nutzbarkeit der ermittelten Daten und kann auch die Belastbarkeit der Analyse verbessern, insbesondere auch in Hinblick auf eine Plausibilitätsprüfung. Vorzugsweise wird dabei zumindest die Fraktion Acetate gemessen/erfasst, insbesondere da diese stark von exoelektrogenen Bakterien verstoffwechselt wird. Dabei können VFAs, insbesondere Acetate, über den Stromfluss gemessen werden, wohingegen eine direkte Konzentrationsmessung ist ohne weiteres implementierbar ist. Da jedoch die exoelektrogenen Bakterien bevorzugt Acetat verstoffwechseln, kann die CCR(-Rate) als Bezugsgröße für das Vorhandensein von Acetaten herangezogen werden. Je höher die CCR, desto mehr Acetate liegen vor (1mA=7,2mgCOD/d, wobei sich der chemische Sauerstoffbedarf COD auf den COD von Acetate bezieht).

Es ist zu verstehen, dass eine Korrelation oben genannter Messwerte unter Bezugnahme auf VFAs dank besonders starker Interdependenz erwartungsgemäß auf vergleichsweise einfache Art und Weise implementierbar ist, so dass die Güte der ausgewerteten Messdaten weiter steigen mag, sobald VFAs mit einbezogen werden.

Gemäß einem Ausführungsbeispiel ist die messtechnische Vorrichtung eingerichtet zum Korrelieren wenigstens eines Messwerts mit einem Prozessparameter P_{SB} für die Kohlenstoffverbrauchsrate, insbesondere bei einem zu optimierenden (Ab-)Wasseraufbereitungsprozesse, insbesondere indem der erzeugte Biostrom mit der Kohlenstoffverbrauchsrate korreliert bzw. umgerechnet wird, insbesondere eingerichtet ist zum Ermitteln auch eines Prozessparameters P_{SB} für den chemischen Sauerstoffbedarf (CSB) oder des CSB selbst basierend auf der Kohlenstoffverbrauchsrate. Dies erweitert nicht zuletzt die Nutzbarkeit der ermittelten Daten (insbesondere auch durch Anwender, z.B. bei prozessualen Optimierungsmaßnahmen in Aufbereitungsanlagen) und kann auch die Belastbarkeit der Analyse verbessern, insbesondere auch in Hinblick auf eine Plausibilitätsprüfung.

Die vorliegende Erfindung beruht dabei auch auf der Erkenntnis, dass durch (bevorzugt kontinuierliche) Messung bzw. Bestimmung der Kohlenstoffverbrauchsrate (carbon consumption rate CCR) wichtige Erkenntnisse insbesondere in Hinblick auf den (Gesundheits-)Zustand der involvierten Bakterien gewonnen werden können (was für Anlagenbetreiber und Prozessteuerung relevant sein mag), insbesondere basierend auf einer vordefinierbaren Umrechnungsfaktor-Korrelation von generiertem Biostrom und CCR, so dass einem (Klär-)Anlagenbetreiber bzw. einem anderweitigen Anwender mittels des hier beschriebenen messtechnischen Ansatzes basierend auf ermitteltem CCR und ermitteltem CSB/BSB auch eine (proaktive) Anlagen-/Prozesssteuerung ermöglicht werden kann. Der CSB wird dabei bevorzugt als *Elektronenäquivalent* (eeq) dargestellt, nämlich: 8gCSB=1eeq. Dieser Zusammenhang kann auch als *Stromäquivalent* ausgedrückt werden: 1mA = 7,2mgCSB/d. Basierend auf diesem Zusammenhang kann eine direkte Korrelation von mittels der MBZ erzeugtem Biostrom und dem CSB geliefert werden. Insbesondere kann über Faktor 7,2 vom generierten Biostrom auf den CSB geschlossen werden. Anders ausgedrückt kann der gemessene Biostromfluss als ein Maß für die gemessene Kohlenstoffverbrauchsrate CCR_{gem} dargestellt werden. Dabei kann berücksichtigt werden, dass nur derjenige Anteil der Elektronen gemessen wird, welcher nicht zum Biomasseaufbau genutzt wird, d.h., nur derjenige Teil der Elektronen, welcher im Rahmen des Energiestoffwechsels auf den Elektronenakzeptor (Anode) übertragen wird. Um die gesamte/absolute Kohlenstoffverbrauchsrate CCRₜₒₜ (Anabolismus und Katabolismus zusammengenommen) zu erhalten, kann vorteilhaft die so genannte *Ertragsrate* (Y, engl.: yield) genutzt werden. Diese kann theoretisch zu 0,243 gCOD/gCOD angenommen werden (COD, engl.: chemical oxygen demand). Basierend auf einem Halbreaktionsansatz und einem "concept of yield"-Ansatz (Ertragsratenansatz) kann demnach die gesamte Kohlenstoffverbrauchsrate CCRₜₒₜ ermittelt werden: ${CCR}_{tot} = 1 / \left(1-Y\right) * 7 , 2 * {I}_{MBZ} = 1 / \left(1-Y\right) * {CCR}_{gem}$

In diesem Zusammenhang kann die Begrifflichkeit auch wie folgt definiert sein/werden:
CCRtot = CCRMet (Metabolismus)
CCRgem = CCRKat (Katabolismus) $CCRAna = Y / \left(1-Y\right) * CCRMet \left(Anabolismus\right)$

Dabei handelt es sich um den so genannten wahren Ertragskoeffizienten bzw. den "true yield". Im Gegensatz zum beobachteten Ertragskoeffizienten ("observed" Yield) berücksichtigt der "true" Yield kein Sterben der Bakterien, ist also auf theoretischer Basis hergeleitet (aus der Stöchiometrie).

Dabei werden nicht alle Bakterien im zu untersuchenden Fluid bzw. Abwasser erfasst, sondern nur die so genannten exoelektrogenen Bakterien/Mikroorganismen, d.h., nur diejenigen Spezies/Spezien, welche in der Lage sind, Elektronen an die Anode zu übertragen, wobei diese Bakterien als Indikatororganismen herangezogen werden können.

Randnotiz: In Hinblick darauf, dass die Summe aus Katabolismus und Anabolismus als Metabolismus bezeichnet wird, kann für die Begrifflichkeit CCR_tot alternativ auch CCR_Met gewählt werden.

Durch die (kontinuierliche) Messung der CCR lässt sich demnach insbesondere auch der "Gesundheitszustand" der Bakterien ableiten, also beispielsweise ermitteln, ob z.B. eine Nährstofflimitation vorliegt. Dies kann auch eine Prozesssteuerung erleichtern, insbesondere die Effizienz einer Abwasseraufbereitung steigern. Ergänzend können Abwassergüteparameter eine tiefere Diagnose ermöglichen, wie hier insbesondere unter Bezugnahme auf Ermittlungsschritte basierend auf Modulation und/oder maschinellem Lernen (ML) beschrieben.

Rein beispielhaft sei hier ein Anwendungsbeispiel aus Sicht eines Anlagenbetreibers skizziert:
Ein Industriebetrieb leitet einen hochbelasteten CSB-Abwasserstrom ein. Durch geänderte bzw. sich ändernde Zusammensetzung des Abwassers steigt die Aktivität der Bakterien und somit die CCR augenblicklich an. Der Kläranlagenbetreiber sollte also fokussierter auf mögliche Prozessstörungen achten. Basierend auf dem hier beschriebenen messtechnischen Ansatz, vorteilhaft auch umfassend Modulation und/oder ML-Maßnahmen, kann dabei erwartungsgemäß ermittelt werden, dass bzw. in welchem Maße die CSB-Konzentration angestiegen ist. Diese Informationen kann der Betreiber z.B. dafür nutzen, möglichen Prozessstörungen zu begegnen (z.B. Einhalten der Ablaufgrenzwerte) oder den Aufbereitungs-Prozess zu optimieren bzw. effizienter auszugestalten (z.B. zwecks Minimierung von Treibhausgas-Emissionen und/oder Minimierung des Energieverbrauches). Indem also der momentan generierte elektrische Biostrom und die momentane CCR messtechnisch ermittelt werden, kann z.B. ein Kläranlagenbetreiber die Aktivität der Bakterien aktuell mitverfolgen und durch die bekannte Kenngröße des CSB direkt interpretieren und vorausschauend z.B. eine Steuerungs-/Regelungsmaßnahme initiieren oder sogar unmittelbar basierend auf CCR und/oder CSB vorgeben.

Gemäß einem Ausführungsbeispiel ist die messtechnische Vorrichtung in energetischer Hinsicht autark, insbesondere indem die wenigstens eine messtechnische mikrobielle elektrochemische Technologieeinheit den Energieeigenbedarf selbstständig deckt und/oder indem die messtechnische Vorrichtung mit wenigstens einem Mittel zur Energiegewinnung ausgestattet ist, z.B. mit wenigstens einer Solarkollektoreinheit. Dies liefert nicht zuletzt noch höhere Flexibilität bzw. Variabilität hinsichtlich der Verwendung der hier beschriebenen messtechnischen Vorrichtungen insbesondere in komplexen messtechnischen Systemen und/oder an eher unzugänglichen Sensorpunkten. Nicht zuletzt kann auch ein im Kontext mit Wartung der Sensorik entstehender Aufwand weiter minimiert werden.

Gemäß einem Ausführungsbeispiel ist die messtechnische Vorrichtung energetisch autark für stromlose Messung, insbesondere indem die für den Betrieb der messtechnischen Vorrichtung erforderliche Energie durch die wenigstens eine messtechnische mikrobielle elektrochemische Technologieeinheit generiert wird. Die energetische Autarkie liefert nicht zuletzt auch die hier beschriebene hohe Flexibilität/Variabilität insbesondere hinsichtlich (auch wartungsarmer) Auswahl und Anordnung einer Vielzahl von Sensorpunkten, was speziell in vergleichsweise komplexen messtechnischen Systemen, z.B. bei einer Vielzahl von über eine weitläufige Aufbereitungsanlage oder über ein weitläufiges Gewässer verteilten messtechnischen Vorrichtungen, spürbare Vorteile mit sich bringt.

Es ist zu verstehen, dass die mittels einer oder mehrerer parallel geschalteter MBZs bzw. METs generierbare (Bio-)Energie betragsmäßig ausreichend groß/hoch ist, dass wenigstens eine Energiespeichereinheit (Batterie) der messtechnischen Vorrichtung geladen werden kann, mittels welcher ausreichend Energie für drahtlose Datenübertragung und/oder Modulation bereitgehalten werden kann, insbesondere basierend auf Ultra-Low Power Management System-Komponenten. So ließe sich z.B. mehr oder weniger kontinuierlich eine Batterie laden, um die benötigte Energie für drahtlose Datenübertragung und wahlweise auch Modulation, insbesondere Pulsmodulation, speziell auch Pulsweitenmodulation liefern zu können.

Beispielsweise kann durch eine Power Management System-Komponente eine 20mV-Eingangsspannung auf ca. 2 bis 5V angehoben werden (insbesondere mittels verschalteter Kondensatoren). Beispielsweise wird eine Start-Spannung von 330mV vorgesehen, bei minimaler Eingangsspannung von 100mV, bei dauerhafter Eingangsspannung im Bereich von 3,3 bis 5,5V. Beispielsweise werden die Messdaten bzw. Messwerte/Parameter (insbesondere CSB, BSB) mittels Modulation und gegebenenfalls auch künstlicher Intelligenz alle 5 bis 15min. ermittelt, z.B. in Abwasseraufbereitungssystemen. In den Zwischenphasen, in denen keine Parameterbestimmung erfolgt, kann die jeweilige MBZ bzw. das entsprechende MET (Bio-)Energie liefern, zur Akkumulation. Die generierte bzw. überschüssige Energie kann wahlweise auch anderweitig eingesetzt werden, z.B. für weitere Sensoren wie z.B. Temperatursensoren, Leitfähigkeitssensorik, pH-Wert-Sensorik. Zumindest ein Teil generierter (Bio-)Energie wird vorteilhaft für Mikroelektronik der messtechnischen Vorrichtung sowie für Datentransfer bereitgestellt.

Ferner ist zu verstehen, dass die hier beschriebenen Autarkie-Vorteile für zahlreiche unterschiedliche Konstellationen sichergestellt werden können. Zwar ist jeweils auch eine gewisse biologische/chemische Aktivität erforderlich, um überhaupt spürbar (Bio-)Energie aus dem Prozess der MET(s) generieren zu können, jedoch wäre es schwierig, einen unteren Schwellwert für den CSB anzugeben, insbesondere da viele Faktoren darauf einen Einfluss haben können (z.B. externer Widerstand, Substrat, Temperatur, Reaktordesign). Der Fachmann mag für die Implementierung einer Energie-Autarkie einen Richtwert über die so genannte Halbsättigungskonstante der exoelektrogenen Bakterien/Mikroorganismen berechnen können.

Gemäß einem Ausführungsbeispiel umfasst die messtechnische Vorrichtung wenigstens einen Sensor, welcher Messdaten betreffend wenigstens eine Fluidcharakteristik aus der folgenden Gruppe generiert und energetisch über die energetisch bevorzugt autarke messtechnische Vorrichtung versorgt ist/wird: Temperatur, pH-Wert, Leitfähigkeit; wobei diese Messdaten betreffend wenigstens eine Fluidcharakteristik bevorzugt für das Korrelieren des wenigstens einen Messwerts mit dem wenigstens einen Prozessparameter berücksichtigt werden. Dies kann auch eine noch exaktere Berücksichtigung etwaiger Abhängigkeiten von Parametern bzw. messtechnischen Randbedingungen erleichtern, wodurch die Datengrundlage weiter verbessert bzw. zusätzlich sicherer/belastbarer ausgestaltet werden kann.

Gemäß einem Ausführungsbeispiel umfasst die messtechnische Vorrichtung eine Mehrzahl von messtechnischen mikrobiellen elektrochemischen Technologieeinheiten, insbesondere in einer wenigstens einfach redundanten Anordnung an/für denselben Messpunkt, wobei die messtechnische Vorrichtung eingerichtet ist für eine Querschnittsmessung bzw. Mittelwertmessung über die Mehrzahl von messtechnischen mikrobiellen elektrochemischen Technologieeinheiten für den jeweiligen bzw. entsprechenden Messpunkt. Dies kann die Datengrundlage weiter verbessern und auch eine Plausibilitätsprüfung erleichtern, insbesondere bei den hier beschriebenen vergleichsweise niedrigen vorrichtungstechnisch begründeten Maßnahmen bzw. Kosten je Sensorpunkt.

Gemäß einem Ausführungsbeispiel ist die messtechnische Vorrichtung eingerichtet/implementiert zur Analyse oder zumindest zur Detektion photosynthetischer Prozesse oder von Bodenfeuchte. Dies erweitert nicht zuletzt auch das Anwendungsspektrum für die hier beschriebene Analytik, insbesondere basierend auf einer/der sehr kostengünstig realisierbaren vorrichtungstechnischen Ausgestaltung je Messpunkt.

Gemäß einem Ausführungsbeispiel ist die Logikeinheit eingerichtet zum Auswerten von Messdaten basierend auf maschinellem Lernen, wobei Zellparameter zum Bestimmen des chemischen und/oder biologischen Sauerstoffbedarfs und/oder der Kohlenstoffverbrauchsrate und/oder von Stofffraktionen flüchtiger Fettsäuren ausgewertet werden, welche bevorzugt basierend auf Modulation, insbesondere Pulsmodulation des externen elektrischen Lastwiderstandes von aus über einen (systembedingt unbeeinträchtigten) Auswertungszeitraum von mindestens 30 Tagen erhobenen Messdaten betreffend zellinterne Widerstandskomponenten und/oder Spannungs-, Kapazitäts-, Strom-, Leistungs-, und/oder elektrischen Ladungsmessdaten generiert sind/wurden. Dies kann auch das Spektrum der analysierbaren Daten erweitern, die Korrelation verbessern und auch das möglichst detaillierte Aufdecken eines Zusammenhangs zwischen biologischen/chemischen Vorgängen und daraus hervorgehenden Effekten im zu untersuchenden Fluid erleichtern.

Es ist zu verstehen, dass die Applikation von ML-Algorithmen bzw. ein KI-Training vorteilhaft lokal am jeweiligen Sensorpunkt bzw. Messpunkt erfolgen kann, insbesondere durch einen/den sensorpunktspezifischen Mikrocontroller. Wahlweise kann dieser Vorgang jedoch auch remote outgesourced werden (extern systemfremd vergeben werden), also indem die auszuwertenden Daten zunächst transmittiert werden und dann zentral weiterverarbeitet werden. Alternativ oder ergänzend kann auch ein bereits vortrainiertes KI-Modell bzw. ein ML-Algorithmus implementiert sein/werden, insbesondere im Kontext einer Grundkalibrierung, basierend auf welcher Ergebnisse produziert werden können, ohne notwendigerweise bereits Messdaten erhoben haben zu müssen. Dank einer Grundkalibrierung können Parameter eines wahlweise zu verwendenden KI-Modells bzw. ML-Algorithmus auch im Vorfeld bestimmt werden, beispielsweise indem eine Kalibrierlösung verwendet wird (z.B. synthetisches Abwasser) und/oder indem Parameter eines/des ANNs, die bereits für andere Abwässer bestimmt wurden, genutzt werden. Vorteilhaft wird dabei zwischen kommunalem und industriellen Abwasser unterschieden.

Eine derartige Grundkalibrierung kann mit der Zeit durch aktuelle Messdaten erweitert bzw. nachjustiert werden (Nachkalibrierung während des Betriebs bzw. zeitlich parallel zur Nutzung), insbesondere derart, dass das angewandte KI-Modell bzw. wenigstens ein angewandter ML-Algorithmus spezifischer auf die jeweilige Anwendung (z.B. spezifischer Kläranlagen-Typ) angepasst werden kann. Beispielsweise kann basierend auf einem aus wenigstens drei Schichten von Neuronen (Eingabeschicht, wenigstens eine verdeckte Schicht, Ausgabeschicht) bestehenden künstlichen neuronalen Netzwerk bzw. basierend auf einem gerichteten azyklischen Graph eine Kalibrierung dahingehend erfolgen, dass die messtechnische Vorrichtung einsatzbereit bereitgestellt werden kann, also messtechnische Ergebnisse direkt ohne Training im Feld liefern kann. Insbesondere erfolgt über die Eingabeschicht eine Verknüpfung elektrischer bzw. elektrochemischer Parameter mit wenigstens einer Zwischenschicht (welche z.B. spezifisch je zu untersuchendem Abwasser definiert wird), um aus der wenigstens einen Zwischenschicht zur Ausgabeschicht zu gelangen, welche z.B. den CSB ausgibt. Knotenpunkte und Parameter können z.B. im Rahmen einer globalen Kalibrierung ermittelt und angepasst werden.

Gemäß einem Ausführungsbeispiel weist die messtechnische Vorrichtung, insbesondere die messtechnische mikrobielle elektrochemische Technologieeinheit, eine Grundkalibrierung auf, insbesondere eine mittels wenigstens eines ML-Algorithmus eingestellte Grundkalibrierung. Dies begünstigt auch einen unmittelbaren Einsatz bzw. eine sofortige Funktionsfähigkeit im Feld, also ohne dass anwenderseitig vorab eine gewisse Datenmenge generiert werden müsste oder ein KI-Training stattfinden müsste.

Als Grundkalibrierung ist dabei hier eine entweder standardmäßige oder aber anwendungsspezifische Vorbereitung der Art und Weise der messtechnischen Auswertung durch die messtechnische Vorrichtung zu verstehen, insbesondere unter Anwendung wenigstens eines ML-Algorithmus. Insbesondere in der analytischen Chemie ist als Kalibrierung allgemein ein Prozess zu verstehen, welcher darauf abzielt, eine vordefinierte/vordefinierbare mathematische Beziehung zwischen einer gemessenen physikalischen Größe und der Konzentration des Analyten herzustellen (gemessene physikalische Größe = Ausgangssignal des Sensors bzw. Antwortsignal; bei der hier beschriebenen MET also die entsprechenden energetische und elektrochemische Kenngrößen). Aufgrund diverser Einflussfaktoren bei Einsatz in realem Abwasser treten komplexere nicht-lineare Beziehungen auf, die basierend auf der hier beschriebenen MET insbesondere mittels eines KI-Modells erfasst bzw. abgebildet werden können. Insbesondere im Labormaßstab (offline) können definierte Bedingungen geschaffen werden, die zur Grundkalibrierung der messtechnischen Vorrichtung bzw. zur Definition des KI-Modells genutzt werden können. Beispielsweise bei Verwendung synthetischen Abwassers kann eine eindeutige mathematische Beziehung abgeleitet werden. Bei optimalen Randbedingungen gilt die folgende Annahme: Der elektrische Stromfluss ist proportional zur Substratkonzentration bei ungesättigten Bedingungen (linearer Zusammenhang). Diese Annahme wird im Großteil in den Fachliteraturstudien gemacht/unterstellt, ist jedoch für z.B. kommunales Abwasser nicht zutreffend bzw. für die Analyse nicht tragbar (insbesondere da zu viele/starke Interferenzen auftreten, welche das Stromsignal beeinflussen, beispielsweise auch zu große Schwankungen von Temperatur, pH-Wert, Leitfähigkeit, anderen bioaktiven Stoffen). Somit besteht ein großer Nutzen an einer insbesondere mittels wenigstens eines ML-Algorithmus eingerichteten Grundkalibrierung in Verbindung mit einem vortrainierten KI-Modell, um damit im nächsten Schritt anwendungsspezifisch zu einem weiter anwendungsspezifisch trainierten KI-Modell gelangen zu können.

In Hinblick auf die hier beschriebenen Maßnahmen zur Implementierung von ML-Algorithmen ist zu erwähnen, dass sich im Rahmen der erfindungsgemäßen Untersuchungen gezeigt hat, dass daraus hervorgehende KI-Modelle (spezialisierte KI, in Abgrenzung zu einer generalisierten KI) grob zumindest hinsichtlich der folgenden drei Themenbereiche bzw. Aufgabenfelder unterteilt werden können, wobei für jeden Themenbereich ein spezifisches KI-Modell implementiert werden kann: Abbildung der Parameterkorrelation als solcher; Überwindung eines deterministischen Mangels zur Beschreibung von Interdependenzen; Bereitstellung wenigstens eines Kalibrierzustandes. Dabei ist die hier beschriebene KI-Implementierung bevorzugt bereits hinsichtlich der Kalibrierung zumindest zweistufig, nämlich einerseits hinsichtlich Vorgabe einer/der anwendungsspezifisch z.B. ausgehend von einer (Ab-)Wasser-Klassifikation optimierten Grundkalibrierung (Stichwort Warmstart bei Erstinbetriebnahme) und andererseits hinsichtlich Vorgabe einer selbstlernenden sich an spezifische messtechnische Randbedingungen im Feld angleichenden Kalibrierung für den Dauerbetrieb über viele Monate bzw. Jahre. Insoweit kann auch eine lernfähige (Selbst-)Kalibrierfunktion bereitgestellt werden, in Abgrenzung zu deterministischen/vordefinierten Kalibrierungen, die insbesondere im hier beschriebenen Kontext von Abwasser-Monitoring messtechnische Nachteile mit sich bringen würden.

Dabei wird hier basierend auf dem Oberbegriff Grundkalibrierung zwischen hier so genannter globaler Kalibrierung und hier so genannter Werkskalibrierung unterschieden: Letztere kann als eine Art Standardkalibrierung erachtet werden. Hingegen die globale Kalibrierung kann insbesondere anwendungsspezifisch implementiert sein/werden, z.B. indem ein Anwender (z.B. Industriekläranlagenbetreiber oder Betreiber kommunaler Kläranlagen) einen erwartungsgemäßen Messbereich für das zu analysierende Abwasser bereitstellt (z.B. erwartungsgemäße Mindestverschmutzung, mittlere Verschmutzung, maximale Verschmutzung), und indem die messtechnische Vorrichtung für diesen Messbereich voreingestellt wird, insbesondere durch Anwendung wenigstens eines ML-Algorithmus, zum Vordefinieren eines/des adäquaten KI-Modells. Im Nachgang können bei Bedarf z.B. nach bestimmten Zeitabständen der Betriebsdauer Plausibilitätsprüfungen erfolgen. Beide Kalibrierungsarten eignen sich für einen Auslieferungszustand der messtechnischen Vorrichtung derart, dass die messtechnische Vorrichtung sofort einsatzfähig ist und Messwerte generieren kann.

Auch ist zu verstehen, dass eine/die hier beschriebene Modulation, insbesondere Pulsmodulation, speziell auch Pulsweitenmodulation kein notwendiges Kriterium ist, um ein KI-Training durchführen zu können, sondern dass ML-Algorithmen auch dann vorteilhaft implementiert werden können, wenn keine Modulation vorgesehen ist, beispielsweise indem Spannungsprofile ausgewertet werden (insbesondere im Batch-Betrieb). Beispielsweise werden Profile, Verläufe, Kurven und dergleichen Charakteristika unter Bezugnahme auf Steigung, Fläche/Flächeninhalt, maximale Spannung oder dergleichen Kurvenparameter computergestützt ausgewertet.

Im Kontext der Implementierung maschinengestützter, insbesondere selbstlernender Datenanalyse ist zu verstehen, dass auch Randbedingungen insbesondere aufgrund von Umwelteinflüssen oder externen Effekten Berücksichtigung finden können, insbesondere die Leitfähigkeit, die Temperatur, der pH-Wert. Ein für die Datenanalyse im Rahmen der vorliegenden Erfindung implementierbares bzw. bereits vortrainiert bereitstellbares KI-Modell berücksichtigt vorteilhaft zumindest die drei hier genannten Randbedingungen (Leitfähigkeit, Temperatur, pH-Wert), insbesondere um deren (potentiellen) Einfluss bei der Auswertung zu berücksichtigen.

Somit kann durch die hier beschriebene Implementierung wenigstens eines KI-Modells wenigstens einer der folgenden technischen Effekte sichergestellt werden:
- Bereitstellung einer bereits anwendungsspezifisch implementierbaren Grundkalibrierungsfunktionalität, insbesondere für eine erste Funktionalität z.B. im Kontext von Installation und Inokulation (Inbetriebnahmephase zur Bildung eines elektroaktiven Biofilms), in Kombination mit einer anwendungsspezifisch selbstlernenden Kalibrierungsfunktion, insbesondere dank Klassifizierung von (Ab-)Wässern in Obergruppen (z.B. kommunale und industrielle Abwässer) für eine Art Warmstart basierend auf der Grundkalibrierung;
- verbesserte Korrelation bei Langzeitbetrieb insbesondere dank hoher Datengüte für das KI-Training auch bei mehrmonatigem Betrieb;
- höhere Datengüte insbesondere dank selbstlernendem Angleichen;
- Bereitstellung einer selbstlernenden Kalibrierfunktion für besonders hohe Datengüte im Feld auch über mehrere Monate Betriebsdauer;

Dabei kann durch eine selbstlernende Kalibierfunktion auch die Güte der Abwasserqualitätsparameter (CSB, BSB, etc.) gesteigert werden, insbesondere indem die Kalibierfunktion an das spezifische Abwasser angepasst wird.

Die messtechnische Vorrichtung kann als Warnvorrichtungen mit Warnfunktion ausgestaltet sein, insbesondere hinsichtlich toxischer Substanzen wie Schwermetallen, Nitraten, Antibiotika, insbesondere mit implementierter Warnsignalauslösung in Abhängigkeit vom Über-/Unterschreiten wenigstens eines vordefinierten Schwellwerts, beispielweise im Zusammenhang oder im Kontext von so genannten Spülstößen und/oder (Pumpen-)Betriebsausfällen z.B. von Pumpen oder dergleichen einen Pegel beeinflussender Geräte oder Ausrüstung.

Gemäß einem Ausführungsbeispiel ist die messtechnische Vorrichtung eingerichtet, basierend auf dem externen Lastwiderstand eine Kalibrierung der messtechnischen Vorrichtung vorzugeben, zu steuern und/oder einzustellen, insbesondere wenigstens eine Kalibrierungseinstellung der messtechnischen Vorrichtung durch Adjustieren des externen Lastwiderstandes vorzugeben, insbesondere eine/die Grundkalibrierungseinstellung, insbesondere einen linearen Bereich der Kalibrierung vorzugeben, insbesondere zu erweitern. Dies begünstigt auch das optimierte Einstellen der messtechnischen Vorrichtung für bestimmte Messbereiche, z.B. in Hinblick auf besonders belastetes Abwasser einerseits oder andererseits hinsichtlich Monitoring eines eher sauberen Gewässers. Als ein Adjustieren des externen Lastwiderstandes kann dabei auch ein Einstellen der Rahmenbedingungen bei Modulation, insbesondere Pulsmodulation zu verstehen sein, insbesondere hinsichtlich der erforderlichen/gewünschten betragsmäßigen Änderungen im Spannungssignal (Abstimmen des betragsmäßig wirksamen Widerstands hinsichtlich eines für den jeweiligen Anwendungsfall zweckdienlichen Spannungssignaländerungen zwecks Optimierung der Rahmenbedingungen für die Parameterbestimmung, beispielsweise in Abhängigkeit von einer anwendungsspezifischen Zusammensetzung eines untersuchten Abwassers). Hierdurch lassen sich auch negative Effekte wie ein Abknicken des Verlaufs oder ein so genanntes Überschießen vermeiden.

Es ist zu verstehen, dass die vorliegende Erfindung auch auf dem Konzept beruht, die messtechnische Vorrichtung bereits mit einer vorteilhaften Kalibrierung insbesondere anwendungsspezifisch auszustatten, insbesondere um die Inbetriebnahme im Feld zu erleichtern, bevor ausreichend Daten akkumuliert sind für die eigene KI-gestützte Anpassung der Kalibrierung und/oder weiterer Einstellungen. Dabei kann mittels Adjustierung des externen Lastwiderstandes auch der lineare Bereich der Kalibrierung eingestellt/erweitert werden, so dass ausgehend von einer Grundkalibrierung noch einfacher eine anwendungsspezifische Konfiguration gefunden bzw. vorgegeben werden kann, mittels welcher das Training mi Feld auf optimale Weise gestartet bzw. verfeinert werden kann. Damit kann auch das Antwortverhalten der MET basierend auf einer Vorgabe auf Basis des externen Lastwiderstandes eingestellt werden.

Die zuvor genannte Aufgabe wird auch gelöst durch eine (Ab-)Wasseraufbereitungsanlage, aufweisend wenigstens eine messtechnische Vorrichtung gemäß der vorliegenden Offenbarung, oder wobei die Wasseraufbereitungsanlage zumindest eingerichtet ist, in datenkommunikativer Verbindung mit einer solchen messtechnischen Vorrichtung zu stehen, und aufweisend eine Steuerungs-/Regelungseinheit eingerichtet zum Steuern/Regeln eines (Ab-)Wasseraufbereitungsprozesses in Abhängigkeit von mittels der messtechnischen Vorrichtung bevorzugt drahtlos über wenigstens ein Kommunikationsmodul bereitgestellten Messdaten bzw. Prozessparametern, insbesondere basierend auf einem messtechnischen Verfahren gemäß der vorliegenden Offenbarung. Hierdurch lassen sich zuvor genannte Vorteile realisieren, insbesondere in Hinblick auf die Implementierung besonders effizienter Prozessoptimierungsmaßnahmen.

Die zuvor genannte Aufgabe wird auch gelöst durch einen (Ab-)Wasseraufbereitungsprozess, welcher betrieben wird oder zumindest optimiert wird basierend auf mittels wenigstens einer messtechnischen Vorrichtung gemäß der vorliegenden Offenbarung bereitgestellten Messdaten bzw. Prozessparametern, insbesondere basierend auf einem messtechnischen Verfahren gemäß der vorliegenden Offenbarung. Hierdurch lassen sich zuvor genannte Vorteile realisieren, insbesondere in Hinblick auf eine sehr zielgerichtete prozessuale Steuerung/Regelung basierend auf durch die hier beschriebene Analytik bereitgestellten Zielgrößen, z.B. zwecks effizienterer Energieeinsparungsmaßnahmen.

Die zuvor genannte Aufgabe wird auch gelöst durch ein messtechnisches System umfassend wenigstens eine messtechnische Vorrichtung gemäß der vorliegenden Offenbarung, aufweisend wenigstens eine Datenbank und wenigstens eine Recheneinheit eingerichtet zum Auswerten von Messdaten basierend auf maschinellem Lernen, wobei Zellparameter zum Bestimmen des chemischen und/oder biologischen Sauerstoffbedarfs ausgewertet werden, welche basierend auf Modulation, insbesondere Pulsmodulation des externen elektrischen Lastwiderstandes von aus über einen (systembedingt unbeeinträchtigten) Auswertungszeitraum von mindestens 30 Tagen erhobenen Messdaten betreffend zellinterne Widerstandskomponenten und/oder Spannungs-, Kapazitäts-, Strom-, Leistungs-, und/oder elektrischen Ladungsmessdaten generiert sind/wurden, insbesondere mit dem messtechnischen System in einer Implementierung für ein messtechnisches Verfahren gemäß der vorliegenden Offenbarung. Hierdurch lassen sich zuvor genannte Vorteile realisieren, insbesondere in Hinblick auf die Auswertung vergleichsweise komplexer Korrelationen, insbesondere unter Bezugnahme auf zahlreiche Messpunkte in einer Art eines sensorischen Netzwerks insbesondere bei vergleichsweise komplexen Anlagen/Prozessen.

Gemäß einem Ausführungsbeispiel umfasst das messtechnische System wenigstens eine messtechnische Vorrichtung gemäß der vorliegenden Offenbarung, wobei das messtechnische System die jeweils wenigstens eine messtechnische Vorrichtung an einer Vielzahl von unterschiedlichen Messpunkten vorsieht und ein Netz oder einen Schwarm oder ein Raster aus in Kombination miteinander auswertbaren Messpunkten bereitstellt. Dies erleichtert eine möglichst akkurate Analyse auch sehr komplexer Anlagen/Prozesse und kann die Datengüte verbessern und eine Art Plausibilitätsprüfung bzw. Messdatenverifizierung erleichtern.

Gemäß einem Ausführungsbeispiel ist das messtechnische System eingerichtet, eine Vielzahl von unterschiedlichen Messpunkten auszuwerten, an welchen jeweils eine Mehrzahl von messtechnischen mikrobiellen elektrochemischen Technologieeinheiten vorgesehen sind. Eine zumindest teilweise redundante Implementierung von Sensorik je Messpunkt liefert auch eine noch größere Belastbarkeit der Messdaten.

Die zuvor genannte Aufgabe wird auch gelöst durch ein Verfahren zum Generieren wenigstens eines energetischen/elektrochemischen Parameters aus Messdaten erhoben mittels wenigstens einer messtechnischen mikrobiellen elektrochemischen Technologieeinheit basierend auf bioelektrochemischen Prozessen, wobei wenigstens ein Messwert mit wenigstens einem Prozessparameter (P_{SB}) korreliert wird, insbesondere mit einem Prozessparameter (P_{SB}) für den chemischen/biologischen Sauerstoffbedarf (CSB/BSB) und/oder für eine Kohlenstoffverbrauchsrate und/oder für Stofffraktionen flüchtiger Fettsäuren, insbesondere Acetate; insbesondere mittels einer messtechnischen Vorrichtung gemäß der vorliegenden Offenbarung; wobei die Messdaten mittels der wenigstens einer messtechnischen mikrobiellen elektrochemischen Technologieeinheit aufweisend wenigstens eine zwischen kathodischem Bereich und zu untersuchendem Fluid angeordnete und wirksame Keramik erhoben werden, wobei das Generieren des wenigstens einen energetischen/elektrochemischen Parameters aus Messdaten erhoben über einen Auswertungszeitraum von mindestens 30 Tagen erfolgt, insbesondere Messdaten aus zellinternen Widerstandskomponenten und/oder Spannungs-, Kapazitäts-, Strom-, Leistungs-, und/oder elektrischen Ladungsmessdaten. Dies ermöglicht insbesondere im Kontext der zuvor genannten Vorteilen auch eine vergleichsweise schnelle und umfassende Analyse, mit Ergebnissen, die für zahlreiche Zwecke nutzbar bereitgestellt werden können.

Gemäß einem Ausführungsbeispiel wird mittels der wenigstens einen Keramik ein/der Separator der messtechnischen mikrobiellen elektrochemischen Technologieeinheit definiert, welcher einen anodischen Bereich und ein/das kathodische Kompartiment der Membran-Elektroden-Baugruppe derart in der Art einer vollflächigen Zwischenschicht separiert, dass eine messtechnische Langzeitstabilität von mindestens zwei Monaten, vorteilhaft mindestens drei oder vier Monaten, sichergestellt ist und die Messdaten als Datengrundlage für ein KI-Training ohne spürbare Drift bereitgestellt und basierend auf wenigstens einem selbstlernenden Algorithmus ausgewertet werden. Die hier beschriebene vorrichtungstechnische Maßnahme an der Membran-Elektroden-Baugruppe (Implementierung einer keramischen Brücke zwischen den daran anliegenden Elektroden) begünstigt nicht nur den (konstruktiven) Aufbau der Vorrichtung als solchen, sondern wirkt sich unmittelbar auf die erzielbaren bzw. implementierbaren Messdatenauswertemaßnahmen aus.

Gemäß einem Ausführungsbeispiel umfasst das Generieren des wenigstens einen energetischen/elektrochemischen Parameters eine Modulation, insbesondere eine Pulsmodulation des externen elektrischen Lastwiderstandes. Dies liefert auch vorteilhaft auswertbare Eingangsgrößen, z.B. auch für einen ML-Algorithmus (z.B. für ein ANN), wodurch die Analyse weiter verbessert werden kann und Korrelationen deutlicher erkannt und z.B. auch als Grundlage für die Ausgabe von anwendungsspezifischen Steuerungs-/Regelungsparametern genutzt werden können.

Gemäß einem Ausführungsbeispiel wird eine/die Modulation, insbesondere Pulsmodulation ohne PC-Anbindung vorgenommen, nämlich lokal an einem/dem entsprechenden Sensorpunkt bzw. Messpunkt.

Gemäß einem Ausführungsbeispiel werden die erhobenen Messdaten, insbesondere Messdaten aus zellinternen Widerstandskomponenten und/oder Spannungs-, Kapazitäts-, Strom-, Leistungs-, und/oder elektrischen Ladungsmessdaten, basierend auf wenigstens einem KI-Modell bzw. ML-Algorithmus ausgewertet. Dies erleichtert nicht zuletzt auch eine Korrelationsanalyse, eine Mustererkennung und/oder eine Auswertung ganz bestimmter Messdatenverläufe z.B. hinsichtlich wiederkehrender Charakteristika der Kurvendiskussion, so dass anwendungsspezifische Zielgrößen einfacher und akkurater generiert und ausgegeben werden können.

Gemäß einem Ausführungsbeispiel wird zum Korrelieren der generierten Zellparameter mit dem wenigstens einen Prozessparameter P_{SB} wenigstens eine Randbedingung aus der folgenden Gruppe berücksichtigt, insbesondere bereitgestellt durch einen entsprechenden Sensor zum Erfassen von Messdaten betreffend wenigstens eine Fluidcharakteristik aus der folgenden Gruppe: Leitfähigkeit, Temperatur, pH-Wert. Dies verbessert nicht zuletzt auch die Auswertung von Messdaten betreffend Größen oder Parameter, welche von zumindest einer dieser Randbedingungen der Fluidcharakteristik abhängen mögen.

Gemäß einem Ausführungsbeispiel erfolgt das Korrelieren der generierten Zellparameter mit dem wenigstens einen Prozessparameter für wenigstens eine prozessuale Belebungsstufe eines Wasseraufbereitungsprozesses, insbesondere in Kombination mit einer Steuerung/Regelung des Wasseraufbereitungsprozesses z.B. in energetischer Hinsicht (Energieverbrauchsminimierung) basierend auf den ermittelten Messdaten. Dies vereinfacht nicht zuletzt die Implementierung prozessualer Optimierungsmaßnahmen z.B. für Anlagenbetreiber.

Gemäß einem Ausführungsbeispiel wird wenigstens ein Messwert, insbesondere betreffend den Aktivierungswiderstand, die Zellkapazität, die Leerlaufspannung, die Stromdichte, die Leistungsdichte, die elektrische Ladung und/oder der ohmsche Widerstand insbesondere bei organischen Ionen (insbesondere Acetaten) jeweils mit einem Prozessparameter P_{SB} für einen/den chemischen Sauerstoffbedarf (CSB) korreliert, insbesondere über ein Elektronenäquivalent und/oder ein Stromäquivalent basierend auf dem momentan durch die MET erzeugten Biostrom, wahlweise auch unter Einbezug der Kohlenstoffverbrauchsrate (CCR). Die ermittelte Korrelation bzw. der CSB können als wertvolle Ausgangsdaten z.B. für eine Prozesssteuerung ausgegeben werden.

Gemäß einem Ausführungsbeispiel wird wenigstens ein Messwert mit einem Prozessparameter P_{SB}, insbesondere CSB, basierend auf dem biologischen Sauerstoffbedarf (BSB) korreliert. Gemäß einem Ausführungsbeispiel wird ein Messwert mit einem Prozessparameter P_{SB}, insbesondere CSB, basierend auf Stofffraktionen flüchtiger Fettsäuren bzw. kurzkettiger Fettsäuren korreliert. Die jeweils gewählte Art der Korrelation zeigt die Variabilität der hier beschriebenen Analytik auf, was auch eine anwendungsspezifische Implementierung erleichtert.

Gemäß einem Ausführungsbeispiel wird die Kohlenstoffverbrauchsrate (CCR) ermittelt, wobei die Kohlenstoffverbrauchsrate als insbesondere im Rahmen einer Steuerung/Regelung nutzbarer Prozessparameter ausgegeben wird und/oder weiter ausgewertet wird zum Ermitteln eines Prozessparameters P_{SB} für den chemischen Sauerstoffbedarf (CSB) oder des CSB selbst. Die Analyse basierend auf einer Korrelation von CCR und CSB kann dabei zwei insbesondere im Kontext der Abwasseraufbereitung besonders wertvolle Prozessparameter für nachgelagerte Schritte, z.B. zur Steuerung/Regelung, nutzbar machen.

Gemäß einem Ausführungsbeispiel wird die Kohlenstoffverbrauchsrate (CCR) basierend auf mittels der messtechnischen mikrobiellen elektrochemischen Technologieeinheit der messtechnischen Vorrichtung generiertem Biostrom ermittelt wird, insbesondere unter Bezugnahme auf ein Elektronen-/Stromäquivalent des CSB (1mA = 7,16mgCSB/d) und der Ertragsrate 0,243 Gramm CSB Biomasse pro Gramm CSB Substrat. Dies liefert nicht zuletzt auch eine wertvolle Datengrundlage für weitere Analysen insbesondere betreffend Abwassergüteparameter, insbesondere basierend auf Modulation und/oder Algorithmen maschinellen Lernens.

Vorteilhaft werden zumindest Daten betreffend CCR und CSB (bzw. die entsprechenden Prozessparameter) an einen Anwender bzw. Anlagenbetreiber übergeben, insbesondere zwecks kontinuierlichem prozessualem Monitoring und wahlweise auch Initiierung von Steuerungs-/Regelungsmaßnahmen.

Gemäß einem Ausführungsbeispiel erfolgt das Auswerten von Messdaten basierend auf maschinellem Lernen, wobei Zellparameter zum Bestimmen des chemischen und/oder biologischen Sauerstoffbedarfs und/oder der Kohlenstoffverbrauchsrate und/oder von Stofffraktionen flüchtiger Fettsäuren ausgewertet werden, welche bevorzugt basierend auf Modulation, insbesondere Pulsmodulation des externen elektrischen Lastwiderstandes von aus über einen Auswertungszeitraum von mindestens 30 Tagen erhobenen Messdaten betreffend zellinterne Widerstandskomponenten und/oder Spannungs-, Kapazitäts-, Strom-, Leistungs-, und/oder elektrischen Ladungsmessdaten generiert sind/wurden. Dies kann auch die Korrelation bzw. gemeinsame Auswertung von bisher eher separat betrachteten Prozessparametern, insbesondere CSB und CCR und gegebenenfalls Stofffraktionen flüchtiger Fettsäuren (VFAs) erleichtern und Zusammenhänge zwischen biologischen/chemischen Vorgängen und daraus hervorgehende Effekte aufdecken, also zu besserem Verständnis von Abläufen z.B. bei Abwasseraufbereitungsprozessen beitragen.

Gemäß einem Ausführungsbeispiel ist die messtechnische mikrobielle elektrochemische Technologieeinheit als Drei-Elektroden-System mit einer/der dritten Elektrode in Ausgestaltung als Referenzelektrode ausgebildet, wobei mittels der Referenzelektrode das Potential insbesondere in unmittelbarer Nähe der Anode gemessen wird, insbesondere simultan zur mittels der Membran-Elektroden-Baugruppe der MET durchgeführten Messung. Dies erleichtert auch eine Optimierung der Genauigkeit und kann z.B. auch weitere Verbesserungen im Langzeitverhalten erleichtern. Insbesondere kann ein möglicher Einfluss der Kathode größtenteils ausgeblendet werden; die Messung kann auch die vornehmlich relevante Anode fokussiert werden. Dabei ist zu verstehen, dass die Referenzelektrode vorteilhaft in unmittelbarer Nähe zur Anode platziert ist/wird (Potentialbestimmung bezüglich Anode/Kathode; Ermittlung von Spannung/Potential zwischen Referenzelektrode und Anode bzw. Kathode).

Gemäß einem Ausführungsbeispiel weist die messtechnische Vorrichtung, insbesondere die messtechnische mikrobielle elektrochemische Technologieeinheit, eine Grundkalibrierung auf, insbesondere eine mittels wenigstens eines ML-Algorithmus eingestellte Grundkalibrierung, wobei das Generieren des wenigstens einen energetischen/elektrochemischen Parameters aus den erhobenen Messdaten basierend auf der Grundkalibrierung erfolgt. Dies begünstigt nicht zuletzt eine vordefinierte Einstellung insbesondere zu Beginn der Betriebsdauer, welche mit der Zeit durch Erfahrungswerte im Feld weiter angepasst werden kann. Auf diese Weise kann z.B. einem Anlagenbetreiber auch das Umstellen von bisher verwendetem Mess-Equipment auf die hier beschriebenen messtechnischen Systeme erleichtert werden, insbesondere auch im Sinne einer zeitweise parallelen Implementierung und Plausibilitäts-Prüfung bzw. gegenseitigem Überprüfen der generierten Messdaten. Basierend auf einer Grundkalibrierung kann auch ein Grundstock an Messdaten bereitgestellt werden, auf welche eine anwendungsspezifische Optimierung bzw. Implementierung aufbauen kann. Auch kann eine Art Ausgangszustand geboten werden, auf welchen ein ML-Modell zurückgreifen kann.

Dabei ist zu verstehen, dass insbesondere im Kontext mit den BSB1- oder BSB5-Messungen bisher eine üblicherweise vergleichsweise große Messunsicherheit zumindest betreffend den CSB bestand bzw. nicht auf einfache Weise überwunden werden konnte; die Fehlertoleranz lag erwartungsgemäß bei vielen Anwendungen sogar im Bereich von ±30%. Das Messungenauigkeitsintervall mag unter Berücksichtigung aller Faktoren (Probenahme, Lagerung und Labormessung) sogar im Bereich von ±20 bis 40% Gesamtabweichung liegen. Die vorliegende Erfindung ermöglicht insbesondere im Kontext einer/der exakteren BCOD-Bestimmung eine Überwindung dieser nachteilig großen Toleranz auf elegante Weise. Insbesondere wurde im Rahmen der vorliegenden Erfindung erkannt, dass die nun erzielbare hohe Langzeit-Datengüte zu einer besonders belastbaren Parameterkorrelation, Datenauswertung und/oder Datenaufbereitung führen kann, insbesondere auch im Kontext der hier beschriebenen ML- bzw. KI-Implementierungsmaßnahmen.

Es ist zu verstehen, dass die hier an mehreren Stellen erfolgende Bezugnahme auf einen "Auswertungszeitraum von mindestens 30 Tagen" als untere Schwellwertgrenze anzusehen ist, die mittels der hier beschriebenen Technologie und Implementierung problemlos auch um einen vergleichsweise großen Faktor überschritten werden kann, z.B. um den Faktor 3 auf mindestens 90 Tage. Dies gilt auch für Anwendungen, bei welchen die Inokulation (Bildung des elektroaktiven Biofilms) direkt im Fluid bzw. Abwasserstrom erfolgt, d.h., die Sensorik kann sehr pragmatisch direkt in ein Abwasser eingebunden bzw. gehangen werden, ohne dass vorab irgendeine Behandlung oder dergleichen erforderlich ist. Für die Inokulation bis hin zum stationären messtechnischen Betrieb sind dabei maximal nur einige wenige Tage im einstelligen Bereich anzusetzen, z.B. drei oder fünf Tage. Im direkten Vergleich mit konventioneller Online-Sensorik, die üblicherweise wöchentlich bzw. alle zwei Wochen gereinigt wird (werden muss), ergeben sich durch dieses vorteilhafte Langzeitverhalten auch in der täglichen Praxis große Vorteile.

Es hat sich gezeigt, dass mittels des hier beschriebenen messtechnologischen Konzeptes auch vergleichsweise eindeutig zwischen Messunsicherheiten und z.B. Ereignissen wie Spülstößen oder externen Verdünnungseffekten unterschieden werden kann, insbesondere dank vergleichsweise sensibler Ganglinie mikrobieller Aktivität (Metabolismus). Insbesondere kann ausgewertet werden, dass/wenn in der stationären Phase Änderungen im Metabolismus vornehmlich auf Änderungen in der Fluid- bzw. Abwasserzusammensetzung zurückzuführen sind, insbesondere auf Ereignisse wie Starkregen oder Schneeschmelze (bzw. Eintrag von Streusalz in die Kanalisation bzw. Kläranlage, mit dem Effekt einer Erhöhung der Leitfähigkeit), wobei kleinere Schwankungen auf tageszeitbedingte Schwankungen in der Schmutzfracht (z.B. Nutzerverhalten, z.B. in der Aufstehphase der Bevölkerung) zurückgeführt werden können. Ein diesbezügliches Korrelieren kann dabei auch basierend auf Onlinemesstechnik einer jeweiligen (Klär-)Anlage verfeinert werden, insbesondere betreffend pH-Wert, Temperatur, Leitfähigkeit, Volumenstrom.

Dabei kann mittels der hier beschriebenen Messtechnologie auch eine Identifikation und Auswertung von Ereignissen z.B. aus der folgenden Gruppe erfolgen:
- Tagesgänge (insbesondere an Trockenwettertagen), insbesondere durch Auswertung von Belastungsspitzen (zeitlich variabel insbesondere in Abhängigkeit vom Wochentagen, Feiertagen, Umwelteinflüssen, oder dergleichen);
- Regenevents bzw. Spülstöße, mit Verdünnungseffekten (insbesondere höherer Durchfluss bei verringerter Konzentration);
- Salzeinträge insbesondere aufgrund von abgespültem Streusalz von Straßen (insbesondere Erhöhungen der Leitfähigkeit);
- Einleiten von insbesondere toxischen (Industrie-) Abwässern, insbesondere mit Warnfunktionsmöglichkeit hinsichtlich möglicherweise rechtswidriger Handlungen (Schock-Beständigkeit);

Speziell die Kenntnis von Belastungsspitzen bzw. Tagesgängen kann auch als Randbedingung oder Parameter bei der Steuerung/Regelung einer Anlage bzw. eines Prozesses implementiert sein/werden.

Speziell was Spülstöße oder Eintrag von Streusalz betrifft, so ist zu verstehen, dass die theoretische Erwartungshaltung aufgrund Anstiegs der Durchflussrate eine ebenfalls abrupte Abnahme des Metabolismus (Verdünnungseffekte) prognostizieren würde, was jedoch nicht auftreten wird bzw. messtechnisch nicht erfasst wird, da bei Eintrag von Streusalz in das Kanalnetz die Leitfähigkeit kurzzeitig rapide ansteigt, wohingegen bei einem Spülstoß (Rücklösung von abgesetzten Schmutzstoffe im Kanal insbesondere bei Starkregen) die Leitfähigkeit nicht spürbar ansteigt. Vielmehr steigt bei letzterem Ereignis der Metabolismus kurzzeitig an. Aus diesen Analysen lässt sich mittels der hier beschriebenen Messtechnik auf die Art des jeweiligen Ereignisses schlussfolgern, ohne dass dabei die Messgenauigkeit bzw. die Belastbarkeit der Analyse leidet.

Speziell was Tagesgänge betrifft, so ist hinsichtlich deren Auswertung zu verstehen, dass ein jeweiliger Tagesgang unter anderem mit der Durchflussrate korreliert bzw. korreliert werden kann, wobei ein gegebenenfalls anwendungsspezifischer zeitlicher Verzug insbesondere auf die Messstelle bezüglich der Durchflussrate zurückführt werden kann (üblicherweise liegt diese stromauf von der prozessualen Phase einer Vorklärung bei Wasseraufbereitungsanlagen, wohingegen der Biosensor bzw. die MET bevorzugt am Ende der Vorklärung platziert ist). D.h., eine bestimmte hydraulische Aufenthaltszeit in der Vorklärung kann den leichten Versatz zwischen Metabolismus und Durchflussrate erklären.

Nicht zuletzt kann am folgenden Anwendungsbeispiel auch die hohe Robustheit der hier beschriebenen Messtechnologie hervorgehoben werden: Identifikation eines Pumpenausfalls (also Warn-/Diagnosefunktion in einer Störungssituation), wobei die MET situationsbedingt zeitweise (z.B. einen Tag) auch in der Luft, also nicht von Fluid umströmt/umgeben, angeordnet sein/bleiben kann, ohne Schaden zu nehmen genommen. Dies zeigt auch die Robustheit der hier beschriebenen biofilmbasierten Sensortechnologie auf.

Somit kann die Implementierung der Art und Weise der Auswertung einerseits insbesondere durch Kombination mit einer Online-Messtechnik einer jeweiligen Anlage erfolgen, andererseits besteht unabhängig davon die Möglichkeit einer vergleichsweise einfachen Unterscheidung von Ereignissen wie z.B. Spülstößen und Streusalzeintrag insbesondere unter Bezugnahme auf die Leitfähigkeit. Auch durch Einbezug einer (anwendungsspezifischen) Aufenthaltszeit (des zu untersuchenden Fluids), bei Abwasseranlagen z.B. in der so genannten Vorklärung, kann die hier beschriebene Korrelation von Messdaten und wenigstens einem Parameter erleichtert bzw. in der Genauigkeit verbessert werden.

Die vorliegende Erfindung ermöglicht dabei auch eine vergleichsweise starke Korrelation von Biosensordaten zu Online-Messdaten z.B. einer Kläranlage (z.B. betreffend Volumenstrom, Leitfähigkeit, Temperatur). Dabei ist die hier beschriebene Parameterbestimmungsroutine (insbesondere einschließlich einer/der Modulation) vorteilhaft einfach auch unter realen Betriebsbedingungen anwendbar, grenzt sich also von Laborversuchen mit vordefinierten Randbedingungen ab, indem eine Diagnose im Realkläranlagenbetrieb ermöglicht wird. Das bioelektrochemische Signal zeichnet sich dabei durch einen sehr glatten und stabilen Kurvenverlauf aus, was gute Voraussetzungen für die darauf basierende Datenanalyse schafft. Auch was den mittels der hier beschriebenen Technologie produzierten Katholyt betrifft, kann festgestellt werden, dass dieser vorteilhaft die in der Theorie avisierten Eigenschaften aufweist (sehr klare Flüssigkeit, pH-Wert insbesondere im Bereich von 10, Leitfähigkeit insbesondere im Bereich von ca. 4,4 mS/cm), wohingegen z.B. Abwasser eine mittlere Leitfähigkeit im Bereich von 0,8 mS/cm aufweist. Der gebildete Katholyt hat somit die gewünschte bakterizide Wirkung, d.h., eine Biofilmbildung auf der Kathode kann effektiv im Sinne eines Selbstschutzmechanismus verhindert werden.

Die vorliegende Erfindung ist dabei auch im Kontext von drei Ebenen bzw. Entwicklungs-Plattformen zu sehen: -die keramische MBZ als solche, bzw. die MET (anders ausgedrückt: der Biosensor);
- Embedded System, nämlich Hardware- (Mikrocontroller, AD-Wandler, und dergleichen) und Softwarekomponenten (Firmware);
- ein über eine grafische Benutzerschnittstelle (insbesondere in der Art eines Dashboard zur Visualisierung von Daten) zugängliches System mit Plattformfunktionalität;
Freilich ist die für zuverlässige Langzeitstabilität und möglichst auch Schock-Beständigkeit (vergleiche hierzu auch die erwähnten Spülstöße) adäquate technische Ausgestaltung der messtechnischen Vorrichtung als solcher dabei das notwendige Kriterium, um weitere positive Effekte auch auf den anderen beiden Ebenen erzielen zu können.

Ergänzend kann erwähnt werden, dass die hier beschriebenen messtechnischen Komponenten auf einfache Weise derart implementiert bzw. angewandt werden können, dass auch vergleichsweise gravierende Umwelteinflüsse, wie z.B. Starkregen, Schnee, Frost oder dergleichen, die Funktionsfähigkeit bzw. Funktionsweise der hier beschriebenen messtechnischen Komponenten nicht beeinträchtigen. Vielmehr kann deren Implementierung bei hohem Sicherheitslevel auch im Zusammenhang einer Warnfunktion erfolgen. Denn es hat sich gezeigt, dass die exoelektrogenen Bakterien (obgleich anaerob) auch eine gewisse Zeit dem Luftsauerstoff ausgesetzt sein können, ohne dabei Schaden zu nehmen (dank robustem Biofilm).

Die zuvor genannte Aufgabe wird auch gelöst durch ein Verfahren zum Steuern/Regeln wenigstens einer Stellgröße eines Wasseraufbereitungsprozesses basierend auf Messdaten bzw. Prozessparametern bereitgestellt mittels eines messtechnischen Verfahrens gemäß der vorliegenden Offenbarung. Dies ermöglicht basierend auf den zuvor genannten Vorteilen insbesondere auch eine sehr effiziente Steuerung/Regelung, insbesondere da die messtechnisch ermittelten Parameter zeitlich besonders reaktiv/schnell bereitgestellt werden können.

Die zuvor genannte Aufgabe wird auch gelöst durch eine messtechnische Vorrichtung eingerichtet zum Generieren von wenigstens einem energetischen/elektrochemischen Parameter aus über einen Auswertungszeitraum von mindestens 30 Tagen erhobenen Messdaten basierend auf bioelektrochemischen Prozessen, aufweisend wenigstens eine messtechnische mikrobielle elektrochemische Technologieeinheit (MET) mit einem kathodischen Bereich und mit wenigstens einer zwischen kathodischem Bereich und zu untersuchendem Fluid angeordneten Keramik, wobei die MET eingerichtet ist zum Korrelieren wenigstens eines Messwerts mit wenigstens einem Prozessparameter (P_{SB}) basierend auf verarbeiteten Messdaten; insbesondere messtechnische Vorrichtung gemäß der vorliegenden Offenbarung; wobei die messtechnische Vorrichtung hergestellt ist durch Einfassen der Keramik zwischen einer mit Druck nach radial außen implementierten inneren Stützstruktur und einer mit Zugspannung nach radial innen implementierten äußeren Stützstruktur, insbesondere mit der äußeren Stützstruktur in Funktion als Stromkollektor, wahlweise mit zumindest einer der inneren und äußeren Stützstrukturen als integraler Bestandteil von Kathode und/oder Anode, wobei die Keramik vorteilhaft in Ausgestaltung als zumindest abschnittsweise zylindrischer Körper, insbesondere Rohr, mit geometrisch zumindest annähernd zylindrischem Aufbau zwischen Anode und Kathode bereitgestellt ist/wird, wobei durch das Einfassen bevorzugt eine dreikomponentige Membran-Elektroden-Baugruppe mit Keramik und Elektroden jeweils in vollumfänglicher mantelflächiger Anlage gebildet wird. Dies liefert nicht zuletzt eine einfache, robuste, kostengünstige Ausgestaltung, die nicht zuletzt auch für eine Vielzahl von Messpunkten jeweils implementiert werden kann, ohne dass die Kosten bzw. der Aufwand des gesamten Messsystems nachteilig stark ansteigen.

Es ist zu verstehen, dass sich die hier beschriebene messtechnische Vorrichtung dank robustem einfachem Aufbau und dank unkomplizierter Bauform problemlos auf anwendungsspezifische Gegebenheiten anpassen lässt, so dass sich insbesondere folgende Vorteile auf einfache Weise realisieren lassen können:
- gute Dichtigkeit, minimierte Leckagen;
- stark verringerte Komplexität, insbesondere basierend auf dem konstruktiven Konzept eines Keramikzylinders mit (wenigstens) zwei Elektroden;
- Verzicht auf Gasdiffusionsschicht und auf PTFE-Materialien, wodurch ein sehr umweltverträglicher Aufbau realisiert werden kann;
- Verwendung kostengünstiger Materialien beispielsweise auch im Kontext der Elektroden (z.B. Aktivkohlefaserfilz);
- einfacher Zugang zur Kathode, leichter Austausch bzw. leichte Regeneration der Kathode, z.B. durch Säurebehandlung (falls zweckdienlich), z.B. zur Vorbeugung von potentiellen Salzablagerungen;
- weitere Maximierung der erfindungsgemäßen Langzeitstabilität (insbesondere auch hinsichtlich Feuchte an der Kathode, bakterizider Umgebung, minimierter Salzablagerung).

Zusammenfassendend können drei besonders vorteilhafte Aspekte der hier beschriebenen messtechnischen Vorrichtung hervorgehoben werden:
- einfacher robuster konstruktiver Aufbau begünstigt die Anwendung auf Kläranlagen und in anderen vergleichbar aggressiven Umgebungen, bei vorteilhaft einfacher Wartung (beispielsweise erst nach mehreren Monaten);
- besonders vorteilhaft hohe Stabilität für wartungsarmen Langzeitbetrieb, insbesondere auch im Kontext eines zylinderförmigen Keramik-Chassis;
- kostentechnisch vergleichsweise leichte Realisierung dank günstiger Materialen;

Die zuvor genannte Aufgabe wird auch gelöst durch ein Computerprogrammprodukt umfassend Befehle, die bei Ausführung des Computerprogrammproduktes auf einem Computer oder einer messtechnischen Vorrichtung oder einem messtechnischen System diese/n/s dazu veranlassen, Schritte zum Steuern/Regeln eines Verfahren nach einem der vorhergehenden Verfahrensansprüche auf dem Computer auszuführen, insbesondere Computerprogrammprodukt eingerichtet zum Generieren wenigstens eines energetischen/elektrochemischen Parameters aus Messdaten erhoben über einen Auswertungszeitraum von mindestens 30 Tagen mittels wenigstens einer wenigstens eine zwischen kathodischem Bereich und zu untersuchendem Fluid angeordnete und wirksame Keramik aufweisenden messtechnischen mikrobiellen elektrochemischen Technologieeinheit, wobei wenigstens ein Messwert mit wenigstens einem Prozessparameter (P_{SB}) korreliert wird, insbesondere Messdaten aus zellinternen Widerstandskomponenten und/oder Spannungs-, Kapazitäts-, Strom-, Leistungs-, und/oder elektrischen Ladungsmessdaten; insbesondere basierend auf Messdaten erfasst/ermittelt mittels einer messtechnischen Vorrichtung gemäß der vorliegenden Offenbarung und/oder mittels eines Verfahrens gemäß der vorliegenden Offenbarung. Dies ermöglicht basierend auf den zuvor genannten Vorteilen auch eine anwendungsspezifische Nutzbarmachung von Zielgrößen bzw. Prozessparametern, sei es basierend auf Messdaten ermittelt mittels einer einzelnen messtechnischen Vorrichtung, sei es basierend auf Messdaten ermittelt mittels einer Vielzahl messtechnischen Vorrichtungen bezüglich einer Vielzahl von Messpunkten. Beispielsweise sind/werden folgende Maßnahmen über eine drahtlos-Kommunikations-Lösung wie z.B. eine Cloud implementiert: Frontend (Benutzerschnittstelle bzw. die für einen Nutzer sichtbare und vom Nutzer zu bedienende Seite einer Software oder Anwendung) und Backend (Server und Logik), und/oder Bereitstellung einer Applikation (App) oder Website zur Datenvisualisierung.

Beispielsweise wird basierend auf den hier beschriebenen Komponenten ein System mit Plattformfunktionalität bereitgestellt, welches eingerichtet ist, die hier beschriebene Datenkorrelation und -auswertung und -wiedergabe insbesondere über eine grafische Benutzerschnittstelle zu realisieren (z.B. in der Art eines Dashboards), zum Bereitstellen wenigstens einer mobil nutzbaren Monitoring-Funktion für wenigstens einen Nutzer, insbesondere derart dass ein Nutzer mit einem Endgerät über eine (Klär-)Anlage laufen kann und jeweils messpunktspezifisch, z.B. während des Vorbeilaufens an den einzelnen Becken oder Sensoren bzw. messtechnischen Vorrichtungen die jeweiligen Daten angezeigt bekommt, beispielsweise näherungs-/positionsbezogen. Die Plattform kann dabei z.B. durch eine Cloud-artige informationstechnische Infrastruktur bzw. Architektur bereitgestellt sein/werden. Der Fachmann kann ausgehend von der vorliegenden Offenbarung vorgeben, welche Systemkomponenten dabei lokal an einer jeweiligen Messstelle, und/oder serverseitig und/oder softwarebasiert über ein Computerprogramm(produkt) zu implementieren sind.

Die zuvor genannte Aufgabe wird auch gelöst durch ein System mit Plattformfunktionalität, eingerichtet zur Datenkorrelation und -auswertung und -wiedergabe insbesondere über eine grafische Benutzerschnittstelle, zum Bereitstellen wenigstens einer mobil nutzbaren Monitoring-Funktion für wenigstens einen Nutzer, insbesondere näherungs-/positionsbezogen jeweils betreffend eine einzelne von einer Vielzahl von Messstellen, an welchen Messstellen jeweils wenigstens eine messtechnische Vorrichtung gemäß der vorliegenden Offenbarung zum Generieren von wenigstens einem energetischen/elektrochemischen Parameter aus Messdaten basierend auf bioelektrochemischen Prozessen implementiert ist. Dies kann auch die Nutzung der hier beschriebenen Technologie unmittelbar im Feld erleichtern, insbesondere durch einen jeweiligen Anlagenbetreiber, und/oder im Rahmen von jeweils das zu untersuchende Fluid betreffenden (prozessualen) Monitoring-Maßnahmen und/oder Steuerungs-/Regelungs-Maßnahmen insbesondere zur (Ab-)Wasseraufbereitung. Das System mit Plattformfunktionalität kann dabei auch ein/das hier beschriebene messtechnische System umfassen oder zumindest damit in Kommunikation stehen.

Die zuvor genannte Aufgabe wird auch gelöst durch Verwendung einer Membran-Elektroden-Baugruppe in einer messtechnischen mikrobiellen elektrochemischen Technologieeinheit, MET, einer messtechnischen Vorrichtung zum Generieren von wenigstens einem energetischen/elektrochemischen (Zell-)Parameter beim Erheben von Messdaten über eine Zeitdauer (Auswertungszeitraum) von mindestens 30 Tagen, z.B. bei einmal täglichem Erheben von Messdaten, insbesondere beim Trainieren wenigstens eines KI-Modells basierend auf über wenigstens 30 Tage erhobenen wenigstens 30 Messdatenpunkten, wobei eine/die wenigstens eine Membrankomponente der Membran-Elektroden-Baugruppe durch eine Keramik gebildet ist, wobei eine/die Kathode der Membran-Elektroden-Baugruppe zumindest teilweise durch ein Aktivkohle-Graphit-Leitruss-Gemisch oder Aktivkohlefaserfilz oder granulierte Aktivkohle oder Graphitfilz gebildet ist, wobei eine/die Anode der Membran-Elektroden-Baugruppe zumindest teilweise durch ein kohlenstoffbasiertes Kohlefaservlies (bzw. Kohlenstoffschleier) oder Graphitfilz oder Aktivkohlefaserfilz gebildet ist, insbesondere mit Anode und Kathode jeweils in vollumfänglicher mantelflächiger Anlage an der Keramikinnen- bzw. Keramikaußenmantelfläche, wobei wenigstens ein Messwert mit wenigstens einem (organischen/anorganischen) Prozessparameter (P_{SB}) basierend auf den Messdaten korreliert wird, wobei der wenigstens eine energetische/elektrochemische (Zell-)Parameters aus den über einen Auswertungszeitraum von mindestens 30 Tagen erhobenen Messdaten generiert wird, insbesondere aus Messdaten betreffend zellinterne Widerstandskomponenten und/oder aus Spannungs-, Kapazitäts-, Strom-, Leistungs-, und/oder elektrischen Ladungsmessdaten, insbesondere mittels einer zuvor weiter oben beschriebenen messtechnischen Vorrichtung, insbesondere basierend auf einem zuvor weiter oben beschriebenen messtechnischen Verfahren. Hierdurch lassen sich zuvor genannte Vorteile realisieren, insbesondere in Hinblick auf im Wesentlichen durch vorrichtungstechnisch herbeigeführte Optimierungen zur Realisierung einer guten Langzeitstabilität in Kombination mit einer hohen Datengüte. Die Kathode kann wahlweise auch einen Stromkollektor (z.B. Metallgewebe oder Kohlefaservlies) und/oder eine Gasdiffusionsschicht (z.B. PTFE) umfassen.

Es ist zu verstehen, dass speziell auf Kläranlagen bzw. bei Wasseraufbereitungsprozessen CSB und/oder BSB bisher nicht täglich gemessen werden (können), sondern z.B. nur wöchentlich. Auch bei diesem möglicherweise beizubehaltenden Rhythmus kann die vorliegende Erfindung vorteilhaft implementiert werden. Vorteilhaft ist demnach eine Offline-Kalibrierung z.B. in einem Labor vorgesehen, insbesondere in Kombination mit Messungen vor Ort (in situ) an der Kläranlage, gegebenenfalls erweitert um zusätzliche Messkampagnen.

Auch ist zu verstehen, dass die Funktionsweise der hier beschriebenen MET vorteilhaft mittels einer Referenzelektrode gegengeprüft oder weiter optimiert werden kann, insbesondere indem Anode und/oder Kathode einzeln untersucht werden. Auf diese Weise kann auch ein potentiell die Messung beeinträchtigender Effekt (z.B. Ablagerungen) vergleichsweise leicht erkannt und bei der Auswertung z.B. in Hinblick auf eine größere Langzeit-Stabilität vergleichsweise leicht berücksichtigt werden. Beispielsweise erfolgt simultan eine Messung mittels wenigstens einer Referenzelektrode insbesondere in unmittelbarer Nähe der (jeweiligen) Anode.

Gemäß einem Ausführungsbeispiel ist die messtechnische Vorrichtung eingerichtet für einen Aufbau der MET als Zwei-Elektroden-System, welches eingerichtet ist, zu einem Drei-Elektroden-System erweitert zu sein/werden, insbesondere mit einer/der dritten Elektrode in einer Implementierung als Referenzelektrode. Dies begünstigt nicht zuletzt eine weitere Optimierung und messtechnische Sicherheit insbesondere auch bei Langzeitbetrieb. Das Referenzpotential der Elektroden wird bevorzugt über eine Parallelschaltung gemessen. Dabei kann das Potential zwischen Referenzelektrode und Elektrode gemessen werden. Es handelt sich um eine Spannungsmessung, hier bei Bezugnahme auf Elektroden als Potentialmessung bezeichnet.

Anders ausgedrückt: Die MET kann als (um wenigstens eine Elektrode erweiterbares) Zwei-Elektroden-System oder wahlweise auch direkt als Drei-Elektroden-System ausgestaltet sein, mit der dritten Elektrode in Implementierung als Referenzelektrode.

Dabei ist zu verstehen, dass der hier beschriebene vorrichtungstechnische Aufbau durch eine Referenzelektrode vorteilhaft nicht beeinträchtigt wird, vielmehr lediglich dadurch ergänzt werden. Beispielsweise wird standardmäßig ein Zwei-Elektroden-System betrachtet (MET als Ganzes, beide Elektroden, also Anode und Kathode, in Kombination miteinander). Durch eine optional zusätzlich implementierte Referenzelektrode insbesondere in einer Implementierung der messtechnischen Vorrichtung als Drei-Elektroden-System kann z.B. neben der MET bzw. neben der eigentlichen Membran-Elektroden-Baugruppe im Abwasser eine Messung erfolgen (vorteilhaft nahe an der Anode). Durch zusätzliche Potentialmessungen der Anode und der Kathode können die Elektroden individuell untersucht werden, wodurch ein die Messung potentiell beeinflussender Einfluss der jeweils anderen Elektrode ausgeblendet werden kann. In Kombination mit einer Referenzelektrode kann insbesondere bei Fokus auf die Anode (Bakterien im elektroaktiven Biofilm) eine besonders selektive Auswertung durchgeführt werden. Eine Parameterbestimmungsroutine mag dabei vergleichbar zu jener eines Zwei-Elektroden-System sein/bleiben, bei spezifischer Betrachtung von Anode oder Kathode. Gemäß der vorliegenden Erfindung wird die Implementierung einer Referenzelektrode als optional vorgesehen, insbesondere da im Betrieb darauf zu achten ist, dass die Referenzelektrode nicht verschmutzt (wohingegen Anode und Kathode dieser strengen Anforderung nicht unterliegen), was nicht für alle Anwendungen auf einfache Weise sichergestellt werden mag. Zudem mag der Aufbau der Referenzelektrode relativ bruchempfindlich sein (insbesondere aufgrund von Glaskörper), was ebenfalls einige Anwendungsarten ausschließen mag.

Die zuvor genannte Aufgabe wird auch gelöst durch Verwendung einer messtechnischen mikrobiellen elektrochemischen Technologieeinheit, MET, einer messtechnischen Vorrichtung zum Generieren von wenigstens einem energetischen/elektrochemischen Parameter beim Erheben von Messdaten über einen Auswertungszeitraum (Zeitdauer) von mindestens 30 Tagen, wobei die messtechnische mikrobielle elektrochemische Technologieeinheit wenigstens eine zwischen kathodischem Bereich und zu untersuchendem Fluid angeordnete und wirksame Keramik, insbesondere Silikatkeramik, insbesondere Ton-Keramik aufweist, wobei wenigstens ein Messwert mit wenigstens einem Prozessparameter (P_{SB}) basierend auf den erhobenen Messdaten korreliert wird, insbesondere betreffend den chemischen/biologischen Sauerstoffbedarf (CSB/BSB) und/oder eine Kohlenstoffverbrauchsrate und/oder Stofffraktionen flüchtiger Fettsäuren, wobei das Generieren des wenigstens einen energetischen/elektrochemischen (Zell-)Parameters aus Messdaten betreffend zellinterne Widerstandskomponenten und/oder Spannungs-, Kapazitäts-, Strom-, Leistungs-, und/oder elektrischen Ladungsmessdaten erfolgt, wobei das Generieren des wenigstens einen energetischen/elektrochemischen (Zell-)Parameters basierend auf Modulation erfolgt, insbesondere Pulsmodulation des externen elektrischen Lastwiderstandes; wobei die messtechnische mikrobielle elektrochemische Technologieeinheit verwendet wird bei der Implementierung wenigstens einer Sensorik aus der folgenden Gruppe: Bodensensorik, Gewässersensorik (Sensorik für ruhende Gewässer und/oder Fließgewässersensorik), Abwassersensorik im Allgemeinen, Aquaristiksensorik, Kanalsensorik, Sensorik im Zulauf stromauf von Kläranlagen oder einzelner Prozessstufen (insbesondere anaeroben Prozessstufen), Sensorik im Ablauf stromab von Kläranlagen insbesondere im Bereich so genannter Vorfluter oder stromab einzelner Prozessstufen, Sensorik in wenigstens einer prozessualen Belebungsstufe eines Wasseraufbereitungsprozesses, Sensorik zur Detektion photosynthetischer Prozesse, Sensorik für Industrie- und Pflanzenkläranlagen, Grundwassersensorik; insbesondere mittels einer zuvor weiter oben beschriebenen messtechnischen Vorrichtung, insbesondere basierend auf einem zuvor weiter oben beschriebenen messtechnischen Verfahren. Hierdurch lassen sich zuvor genannte Vorteile realisieren, insbesondere in Hinblick auf eine besonders anwendungsspezifisch ausgerichtete Art und Weise der Implementierung bzw. Korrelation. Beispielsweise die Implementierung der hier beschriebenen messtechnischen Vorrichtungen und Verfahren für wenigstens eine prozessuale Belebungsstufe eines Wasseraufbereitungsprozesses liefert auch hohes Energieeinsparpotential, insbesondere da die ermittelbaren Informationen über den Aufbereitungsprozess in Kombination mit Steuerungs-/Regelungsmaßnahmen eine beträchtliche Energieeinsparung versprechen, insbesondere da Belebungsstufen durchaus mehr als 50% des Gesamtenergiebedarfs einer Aufbereitungsanlage ausmachen können. In diesem Kontext kann auch ein Energieverbrauchsmonitoring bzw. eine damit einher gehende Visualisierung von Energieeinsparpotentialen implementiert sein, insbesondere in Verbindung mit den hier beschriebenen Messpunkten.

Dabei ist als Grundwassersensorik insbesondere eine Sensorik zu verstehen, mittels welcher Stand und/oder Qualität und/oder Druck und/oder chemische Grundwasserparameter erfassbar sind, insbesondere auch Nitrat. Da der CSB im Grundwasser üblicherweise sehr gering ist, wird die hier beschriebene MET für eine derartige Implementierung vorteilhaft mit wenigstens einer MEZ ausgestattet (MET bestehend aus oder umfassend wenigstens eine MBZ und wenigstens eine mikrobieller Elektrolysezelle, MEZ).

Zusammenfassung: Mikrobielle (messtechnische) Brennstoffzellen (MBZ) unterlagen bisher großen kostentechnischen Herausforderungen und großen technischen Hürden hinsichtlich Langzeitstabilität, beispielsweise aufgrund von so genannten Scaling-Effekten insbesondere auch bei (Ab-)Wasseraufbereitungsprozessen. Eine belastbare dauerhafte Auswertung insbesondere des chemischen und/oder biologischen Sauerstoffbedarfs (CSB/BSB) würde jedoch große Vorteile auch im Kontext mit Anlagen- und Prozesssteuerung/-regelung in Aussicht stellen. Die vorliegende Erfindung überwindet die bisherigen Nachteile, insbesondere indem einerseits ein vorrichtungstechnischer Aufbau mit sehr guter Langzeitstabilität bereitgestellt wird, und indem andererseits messtechnische Auswerte- und Korrelationsmaßnahmen aufgezeigt werden, welche die Datengrundlage, die Datengüte und auch das computergestützte Auswerten insbesondere mittels selbstlernender Algorithmen verbessern. Insbesondere stellt die vorliegende Erfindung eine messtechnische Vorrichtung mit den folgenden Merkmalen bereit: messtechnische mikrobielle elektrochemische Technologieeinheit mit einem kathodischen Bereich, Logikeinheit mit wenigstens einem Mikrocontroller, zwischen kathodischem Bereich und zu untersuchendem Fluid angeordnete und wirksame Keramik mit Separatorfunktion zwischen den Elektroden (Anode, Kathode), wobei die messtechnische Vorrichtung mittels der Keramik und den Elektroden eingerichtet ist zum Generieren des wenigstens einen energetischen/elektrochemischen Parameters aus über einen Auswertungszeitraum von mindestens 30 Tagen erhobenen Messdaten. Die vorliegende Erfindung steht auch im Kontext mit einem entsprechenden messtechnischen Verfahren zum Generieren wenigstens eines energetischen/elektrochemischen Parameters aus Messdaten erhoben mittels wenigstens einer solchen messtechnischen mikrobiellen elektrochemischen Technologieeinheit basierend auf bioelektrochemischen Prozessen, bevorzugt unter Einbezug von Modulation und/oder selbstlernenden Algorithmen, sowie in Kontext mit Implementierungen von derartigen messtechnischen Vorrichtung bzw. Verfahren im Kontext prozessualer Steuerungs-/Regelungsmaßnahmen, z.B. bei der Wasseraufbereitung. Die vorliegende Erfindung betrifft auch ein entsprechend ausgestaltetes Computerprogrammprodukt sowie Verwendungen von Membran-Elektroden-Baugruppen in messtechnischen mikrobiellen elektrochemischen Technologieeinheiten solcher messtechnischen Vorrichtungen, wobei eine/die wenigstens eine Membrankomponente der Membran-Elektroden-Baugruppe durch eine/die Keramik gebildet ist, wobei eine/die Kathode der Membran-Elektroden-Baugruppe bevorzugt durch ein Aktivkohle-Graphit-Leitruss-Gemisch oder Aktivkohlefaserfilz oder granulierte Aktivkohle oder Graphitfilz gebildet ist, wobei eine/die Anode der Membran-Elektroden-Baugruppe bevorzugt zumindest teilweise durch ein kohlenstoffbasiertes Aktivkohlefaserfilz oder Kohlefaservlies oder Graphitfilz gebildet ist.

### KURZE BESCHREIBUNG DER FIGUREN

In den nachfolgenden Zeichnungsfiguren wird die Erfindung noch näher beschrieben, wobei für Bezugszeichen, die nicht explizit in einer jeweiligen Zeichnungsfigur beschrieben werden, auf die anderen Zeichnungsfiguren verwiesen wird. Es zeigen jeweils in schematischer bzw. qualitativer Darstellung:
**Figur** 1 in geschnittener Seitenansicht den Aufbau einer messtechnischen mikrobiellen elektrochemischen Technologieeinheit (MET) einer messtechnischen Vorrichtung gemäß Ausführungsbeispielen;
**Figur** 2 in geschnittener Seitenansicht den Aufbau einer Membran-Elektroden-Baugruppe einer MET einer messtechnischen Vorrichtung gemäß Ausführungsbeispielen;
**Figuren 3A, 3B,** 3C in zwei perspektivischen Ansichten und in einer geschnittenen Ansicht jeweils Komponenten einer messtechnischen Vorrichtung gemäß Ausführungsbeispielen;
**Figur 4A** eine Visualisierung der mittels einer messtechnischen Vorrichtung bzw. eines messtechnischen Verfahrens jeweils gemäß Ausführungsbeispielen erzielbaren Langzeitstabilität bzw. Datengüte, unter Bezugnahme auf Spannungs-, Strom- und Leistungsdichteverläufen über die Zeit, bzw. unter Bezugnahme auf Polarisation- und Leistungsdichtekurven;
**Figur 4B** einen weitere Materialtest mit Blick auf ACFF-Material, vergleichbar zu jenem gemäß Fig. 4A, für eine messtechnische Vorrichtung bzw. ein messtechnisches Verfahrens jeweils gemäß Ausführungsbeispielen, insbesondere zur Verifizierung der erzielbare Langzeitstabilität bzw. Datengüte, insbesondere basierend auf einer Membran-Elektroden-Baugruppe mit Aktivkohle-Graphit-Leitruss-Gemisch, unter Bezugnahme auf einen Vergleich von Polarisations- und Leistungsdichtekurven zweier Materialien;
**Figuren 5A, 5B, 5C** eine Darstellung von Zellspannungs-, Widerstands- bzw. elektrischen Ladungsverläufen über die Zeit (bzw. von Systemantworten bei frequentiellem Verbinden und Trennen), in Hinblick auf eine messtechnische Auswertung bzw. ein messtechnisches Verfahren jeweils gemäß Ausführungsbeispielen;
**Figur 6A** einen elektrotechnischen Aufbau eines vereinfachten äquivalenten elektrischen Schaltkreises für messtechnische Vorrichtungen und Verfahren gemäß Ausführungsbeispielen;
**Figuren 6B, 6C** einen Vergleich gemessener und simulierter Daten, gemäß Ausführungsbeispielen;
**Figuren 7A, 7B, 7C** eine Gegenüberstellung des zeitlichen Verlaufs elektrochemischer Parameter zur Verifikation einer messtechnischen Echtzeitbestimmung gemäß Ausführungsbeispielen;
**Figur 8** Schritte eines messtechnischen Verfahrens gemäß Ausführungsbeispielen sowie optional zwischengelagerter und/oder nachgelagerter Maßnahmen;
**Figur 9** einen bilanziellen Zusammenhang für den CSB betreffend die nicht-inerten Anteile, unter Bezugnahme auf eine so genannte BSB-Respirationsmessung zur Ermittlung der Reaktionskonstante (kBSB) und der biologisch abbaubaren CSB-Fraktion, zur Implementierung im Kontext messtechnischer Vorrichtungen und Verfahren gemäß Ausführungsbeispielen der vorliegenden Erfindung, insbesondere zwecks Realisierung einer messtechnischen bzw. analytischen Fraktionierung des CSB (vorteilhaft Echtzeitfraktionierung);
**Figuren 10A, 10B** eine weitere Ausgestaltung eines strukturellen Aufbaus einer messtechnischen Vorrichtung gemäß Ausführungsbeispielen;
**Figur 11** ein System mit Plattformfunktionalität, umfassend wenigstens eine messtechnische Vorrichtung gemäß Ausführungsbeispielen, in Kommunikation mit einer Anlage für beispielsweise Wasseraufbereitung;
**Figur 12** eine Veranschaulichung eines Trendverhaltens der mikrobiellen Aktivität bei einem mittels der messtechnischen Vorrichtung durchgeführten Feldversuch speziell im Kontext des Erfassens der CCR, unter vergleichender Bezugnahme auf Messwerte einer UV-Spektrometersonde und einer Laboranalyse (2h Mischprobe);
**Figur 13** eine Veranschaulichung sensorischer Eigenschaften der MET bei einem mittels der messtechnischen Vorrichtung durchgeführten Feldversuch speziell beim Erfassen des CSB, bei graphischer Gegenüberstellung der Messwerte der kalibrierten MET (basierend auf einem Random-Forest-Regressionsmodell) mit Referenzmessungen aus einer 2h Mischprobe im Labor sowie den Messungen einer UV-Spektrometersonde;
**Figuren 14A, 14B****,** **14C, 14D** jeweils eine Ganglinie der mikrobiellen Aktivität, der Kohlenstoffverbrauchsrate, des zellinternen Widerstands und der kumulierten Ladung, jeweils bei Verwendung einer messtechnische Vorrichtung gemäß Ausführungsbeispielen;
**Figur 15** eine Korrelationsmatrix (Pearson) für mittels der hier beschriebenen messtechnischen Vorrichtung auswertbare Korrelationen bzw. Parameter;
**Figuren 16A****,** **16B, 16C** jeweils einen Vergleich zwischen Simulation und Messung mittels einer messtechnischen Vorrichtung gemäß Ausführungsbeispielen, insbesondere im Kontext einer Kalibrierung der messtechnischen Vorrichtung (Random-Forest-Regressionsmodell), wobei eine Gegenüberstellung von beobachteten und vorhergesagten durchflussproprotionalen Tagesmischproben für den chemischen Sauerstoffbedarf (CSB), den gesamten gebundenen Stickstoff (TNb) und Gesamtphosphor (Pgesamt) erfolgt;
**Figur 17** eine Tabelle zu Charakteristika betreffend die Beschaffenheit der Keramik einer messtechnischen Vorrichtung gemäß Ausführungsbeispielen;
**Figuren 18A, 18B** eine weitere Ausgestaltung eines strukturellen Aufbaus einer messtechnischen Vorrichtung gemäß Ausführungsbeispielen.

### DETAILLIERTE BESCHREIBUNG DER FIGUREN

Die Erfindung wird zunächst unter allgemeiner Bezugnahme auf alle Bezugsziffern und Figuren erläutert. Besonderheiten oder Einzelaspekte oder in der jeweiligen Figur gut sichtbare/darstellbare Aspekte der vorliegenden Erfindung werden individuell im Zusammenhang mit der jeweiligen Figur thematisiert.

Bereitgestellt wird eine messtechnische Vorrichtung 10 bzw. ein entsprechendes messtechnisches Verfahren zum Generieren von energetischen/elektrochemischen Parametern basierend auf Messdaten betreffend ein zu untersuchendes Fluid F, beispielsweise (Ab-)Wässer enthaltend organische Abfälle 1 oder dergleichen, wobei eine messtechnische mikrobielle elektrochemische Technologieeinheit (MET) 11 mit einem kathodischen Bereich 11.1, 12b (abgeschottet/separiert von zu untersuchendem Fluid) bereitgestellt wird, bei welcher eine Membran-Elektroden-Baugruppe 12 einen anodischen Bereich 12a (in Kontakt mit zu untersuchendem Fluid) bzw. eine Anode 12.1 und ein kathodisches Kompartiment 12b bzw. eine Kathode 12.3 durch eine Keramik 12.2, insbesondere Silikatkeramik, insbesondere Ton-Keramik, separiert, vorteilhaft bei zumindest annähernd rohrzylindrischer Grundform der Keramik mit einer Keramikaußenmantelfläche 12.21, an welcher die eine Elektrode zur Anlage kommt, und einer Keramikinnenmantelfläche 12.23, an welcher die andere Elektrode zur Anlage kommt. Eine/die Membran bzw. die Membrankomponente 12.7 der Membran-Elektroden-Baugruppe 12 kann demnach mittels der Keramik bereitgestellt bzw. sichergestellt werden, wobei die Keramik eine/die Separator-/Membranfunktion erfüllt. Optional umfasst die messtechnische Vorrichtung 10 Kommunikationsmodul 17 für uni- oder bidirektionale Drahtlos-Kommunikation (Daten-Transmission), z.B. für energieverbrauchsoptimierte Nahfeld-Kommunikation und/oder für Mobilfunk-Kommunikation. Letztere Art der Kommunikation mag insbesondere im Kontext von Anlagen bzw. Prozessen, die zur kritischen Infrastruktur zu zählen sind, als besonders robuste Kommunikationsart erachtet werden.

Es ist zu verstehen, dass sich im Rahmen der erfindungsgemäßen Untersuchungen gezeigt hat, dass eine Keramik mit einem vergleichsweise hohen SiO2-Anteil eine besonders vorteilhafte Charakteristik erwarten lässt, wobei der gewählte SiO2-Anteil auch im Kontext mit einer jeweils gewählten Porosität und/oder Dicke stehen mag. Beispiel: eine vergleichsweise hohe Porosität (z.B. im Bereich von 25% bis 35%) mag eine Steigerung der Leistung mit zunehmender Dicke der Keramik ermöglichen, wohingegen eine vergleichsweise niedrige Porosität (z.B. im Bereich von weniger als 10% bis 15%) mit steigender Dicke zu einer Leistungsabnahme führen mag.

Wenigstens eine solche messtechnische Vorrichtung 10 kann Bestandteil eines messtechnischen Systems 20 mit einer Datenbank 21 und einer Recheneinheit 22 sein, wobei das messtechnische System 20 anwendungsspezifisch implementierte/implementierbare Sensorik 30 umfassen kann, insbesondere wenigstens eine Sensorikkomponente aus der folgenden Gruppe: Bodensensorik 31, Gewässersensorik 32, Abwassersensorik 33, Aquaristiksensorik 34, Kanalsensorik 35, Sensorik 36a im Zulauf stromauf von Kläranlagen oder einzelner Prozessstufen, Sensorik 36b stromab von Kläranlagen (z.B. Vorfluter oder stromab einzelner Prozessstufen, insbesondere auch im Zulauf zur Belebungsstufe oder im Ablauf des Vorklärbeckens), Sensorik 37 in wenigstens einer prozessualen Belebungsstufe, Sensorik 38 zur Detektion photosynthetischer Prozesse, Sensorik 39 für Industrie- und Pflanzenkläranlagen, Grundwassersensorik. Insbesondere sorgt die Sensorikkomponente für die Erfassung von Umweltdaten wie z.B. der Temperatur. Die jeweilige Sensorikkomponente mag je nach Art und Weise der vorrichtungstechnischen Implementierung auch eine messtechnische Vorrichtung umfassen oder damit in messtechnischer Verbindung stehen. Wahlweise kann die messtechnische Vorrichtung 10, das messtechnische System 20 und/oder die jeweilige Sensorikkomponente 30 mit einem Kommunikationsmodul ausgestattet sein. Die anwendungsspezifisch jeweils optimale vorrichtungstechnische Konfiguration hinsichtlich Baugruppen 10, 20, 30 und deren Komponenten kann vom Fachmann implementiert werden.

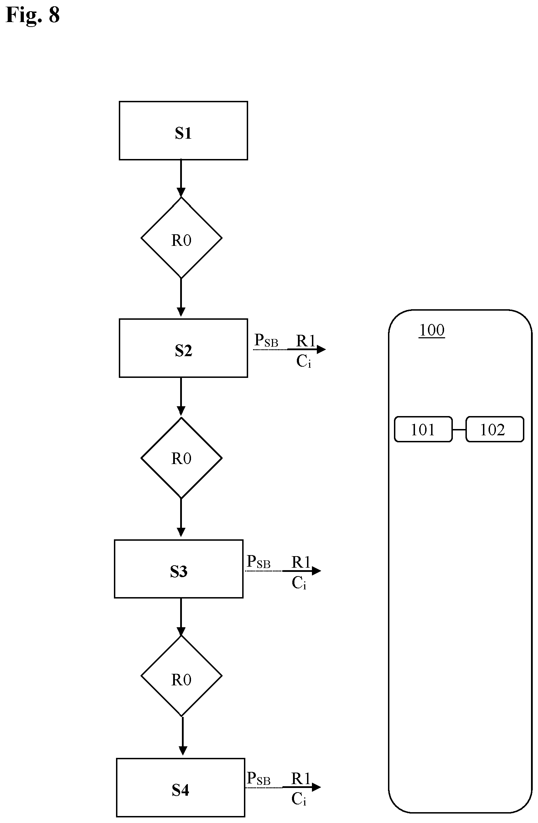

Beispielsweise ist/wird das hier beschriebene messtechnische Verfahren im Rahmen eines Monitoring oder auch einer Steuerung/Regelung einer (Ab-)Wasseraufbereitungsanlage 100 implementiert (Fig. 8), wobei die generierten Daten bzw. Prozessparameter oder Stellgrößen über wenigstens ein Kommunikationsmodul 102 transmittiert und z.B. an eine Steuerungs-/Regelungseinheit 101 der Anlage geliefert werden.

Im Fokus der hier beschriebenen Analytik mag der biologische/chemische Sauerstoffbedarf BSB/CSB stehen (speziell auch der biodegradierbare CSB-Parameter, BCOD bzw. C_CSB, abb), insbesondere bei Wasseraufbereitungsprozessen, gleichwohl mag die hier beschriebene Korrelation auch im Zusammenhang mit der Analyse der Kohlenstoffverbrauchsrate CCR und/oder der Analyse hinsichtlich Stofffraktionen flüchtiger Fettsäuren implementiert sein/werden.

Im Folgenden werden Besonderheiten der Erfindung unter Bezugnahmen auf einzelne Figuren bzw. Ausführungsbeispiele erläutert, wobei die Keramik(komponente) 12.2 auch zusammen mit dem Bezugszeichen 12.7 dargestellt wird, nämlich dann bzw. dort, wenn/wo die Keramik zumindest im Wesentlichen in einer Funktion als Membrankomponente 12.7 verbaut ist. Die Keramik 12.2 kann darüber hinaus auch z.B. Abschnitte des Reaktorkörpers bzw. Chassis bilden, in welchem Bereich die Keramik nicht notwendigerweise eine Separator-/Membranfunktion erfüllt.

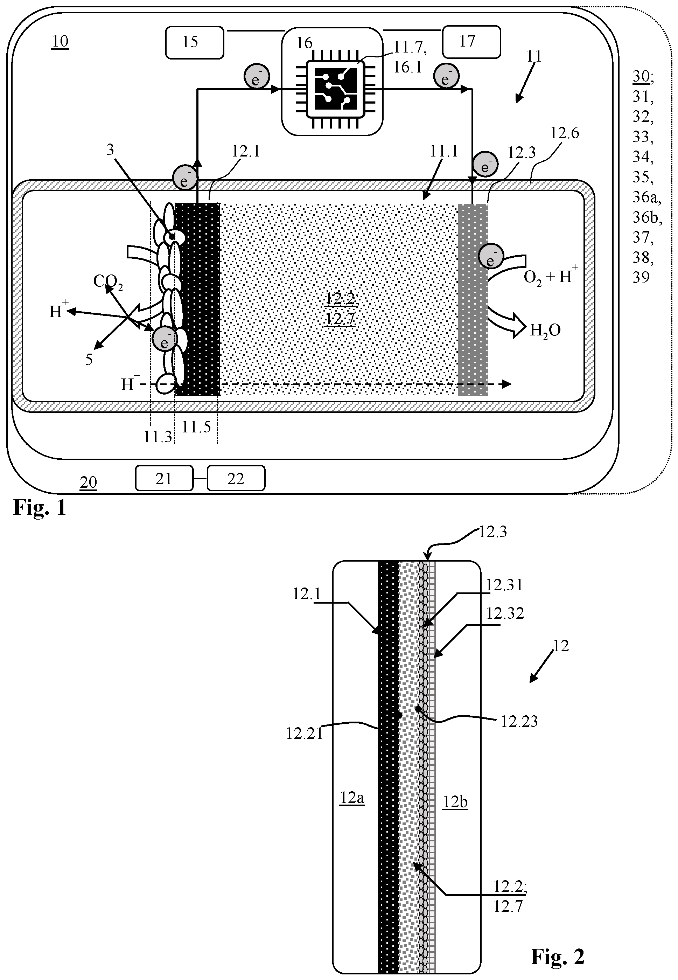

In **Fig. 1** ist ein grundsätzlicher Aufbau der MET 11 dargestellt. Die Keramik-Membrankomponente 12.2, 12.7 ist in mantelflächiger Anlage zwischen den Elektroden 12.1, 12.3 verbaut. Das Chassis 12. 6 (bzw. der Reaktorkörper) ist hier in separater Anordnung von der Keramik 12.2 skizziert; wahlweise kann die Keramik 12.2 in das Chassis 12.6 übergehen bzw. das Chassis 12.6 zumindest teilweise bilden, insbesondere im Bodenbereich zwischen den Elektroden. Es ist zu verstehen, dass die Komponente 12.6 hier lediglich schematisch die Funktionsweise wiedergibt (in der Art eines Rahmens), d.h., die Komponente 12.6 kann in konstruktiver Hinsicht auch andersartig ausgeführt sein. An der Anode der MET 11 kommen Mikroben 3 an einem/einer Biorezeptor(ebene) 11.3 zur Anlage und werden durch biochemische Prozesse insbesondere in H⁺ und CO₂ und kleinere Moleküle 5 zersetzt, wobei der Bereich der Anode auch als Transducer 11.5 fungiert. Anders ausgedrückt: Sobald die Anode in Kontakt mit Abwasser kommt, bildet sich ein Biofilm, der unter anderen exoelektrogene Bakterien enthält. Die Bakterien metabolisieren abbaubare organische Abwasserinhaltsstoffe (Analyt) zu Elektronen, CO2, Protonen (H+) und kleineren Molekülen. Der anodische Biofilm fungiert dabei als Biorezeptor. Die Anodenelektrode ist somit eine Art sensorischer Transducer mit der Funktion, eine Umwandlung in ein messbares elektrisches Signal zu besorgen, d.h., ein Signal betreffend Strom, Spannung, Leistung. Über ein Mikroelektronikmodul 11.7 (bevorzugt einschließlich Software/Firmware und Softsensorfunktion) mit Mikrocontroller 16.1 kann eine/die Auswertung der Messdaten erfolgen. Das Mikroelektronikmodul 11.7 kann Bestandteil einer Logikeinheit 16 sein, die wahlweise auch an eine Energiespeichereinheit 15 angebunden ist.

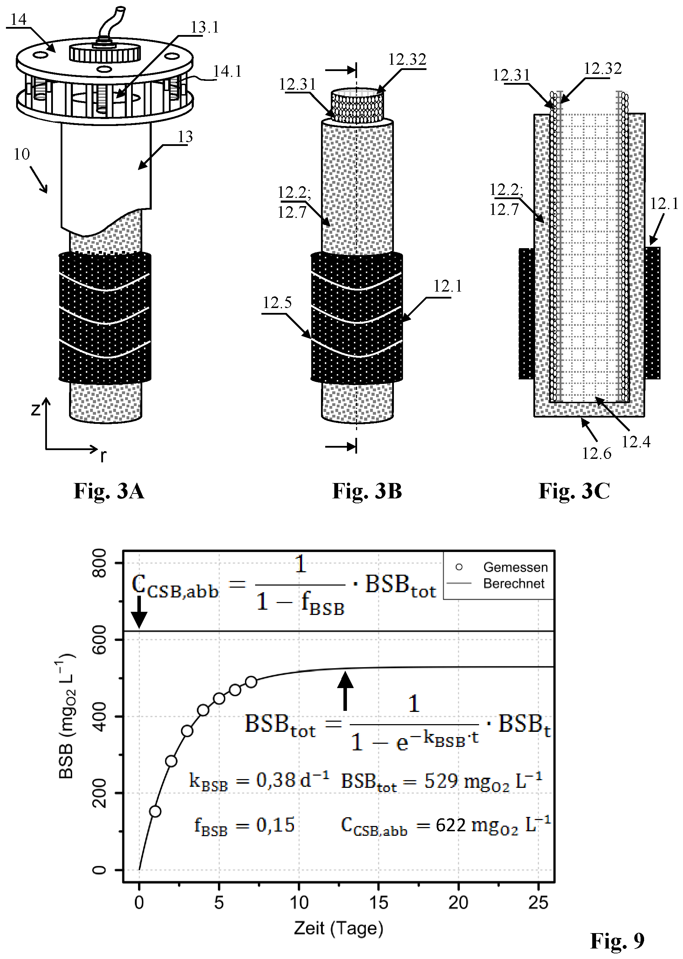

In den folgenden Figuren 2 und 3 werden Komponenten der Membran-Elektroden-Baugruppe näher beschrieben, insbesondere Katalysatorschicht 12.31, Stromkollektor 12.32 (insbesondere mit Metallgewebe), Stützstruktur 12.4, Verdrahtung bzw. Armierung 12.5 (insbesondere Edelstahldrähte), Chassis bzw. Reaktorkörper 12.6 (optional ebenfalls aus Keramik ausgeführt, zumindest teilweise).

In **Fig. 2** ist der grundsätzliche Schichtaufbau einer/der Membran-Elektroden-Baugruppe einer hier beschriebenen MET (11) bzw. messtechnischen Vorrichtung (10) veranschaulicht, weitgehend unabhängig von einer spezifisch gewählten/wählbaren geometrischen Ausgestaltung der Keramikkomponente 12.2 (insbesondere auch mit oder ohne Chassis-Beteiligung). Im anodischen Bereich 12a liegt das zu untersuchende Fluid an (also außen), und das kathodische Kompartiment 12b wird durch die Keramik 12.2 abgeschottet bzw. separiert. Dabei ist die Anode 12.1 vorteilhaft aus wenigstens einer Komponente aus der folgenden Gruppe gebildet: kohlenstoffbasiertes Kohlefaservlies (bzw. Kohlenstoffschleier), Graphitfilz, Aktivkohlefaserfilz. Dabei ist die Kathode 12.3 vorteilhaft zweischichtig gebildet, nämlich durch eine die Keramik 12.2 mantelflächig kontaktierende Katalysatorschicht 12.31 (bevorzugt aus wenigstens einer Komponente aus der folgenden Gruppe: Aktivkohle-Graphit-Leitruss-Gemisch, Aktivkohlefaserfilz, granulierte Aktivkohle, Graphitfilz) und eine radial außen davon angeordnete Stromkollektorschicht 12.32. Dabei kann dieser Aufbau auch wie folgt beschrieben werden: Im von der Keramik definierten Innenraum wird die Kathode angeordnet; wird beispielsweise Aktivkohlefaserfilz als Katalysator verwendet, so wird dieser an der Innenseite der Keramik angeordnet, und daraufhin wird darauf die Stromkollektorschicht z.B. in Ausgestaltung als Edelstahlgewebe gelegt. Bei Bezugnahme auf die Keramik bedeutet "innen" vorteilhaft direkt an der Innenmantelfläche (betreffend die Anordnung des Filz), und "außen" bedeutet für den Stromkollektor die radial entgegengesetzte Richtung. Es ist zu verstehen, dass der Stromkollektor insbesondere bei Verwendung von Aktivkohlefaserfilz wahlweise entfallen kann. Beispielsweise sind die Elektroden so aufgebaut, dass das Aktivkohle-Graphit-Leitruss-Gemisch auf/in das Edelstahlgewebe gepresst ist; insbesondere bei derartiger Konfiguration muss keine zusätzliche radial angeordnete Stromkollektorschicht vorgesehen sein. Die Elektroden 12.1, 12.3 liegen jeweils an einer Keramikaußenmantelfläche 12.21 bzw. Keramikinnenmantelfläche 12.23 der Keramikmembrankomponente 12.2 an, z.B. mit einem flächigen Anteil von mindestens 60% oder mindestens 70% (oder auch noch höherer Mindestschwellwert) der gesamten messtechnisch nutzbaren Mantelfläche der jeweiligen Elektrode. Dabei können die vorrichtungstechnischen Randbedingungen, insbesondere die Relativposition der Komponenten der Keramik-Elektroden-Baugruppe bzw. der kontaktierende Flächenanteil, hinsichtlich möglichst maximal konstanter Betriebsrandbedingungen vergleichsweise konstant gehalten werden, z.B. durch eine Zug-/Druckvorspannungskraftmaßnahme, insbesondere zwecks Maximierung der Datengüte und etwaigen KI-gestützten Auswertungen auch bei Langzeit-Betrieb.

In den **Fig. 3** ist ein Ausführungsbeispiel mit zumindest im Wesentlichen zylindrisch ausgestalteter Membran-Elektroden-Baugruppe bzw. Keramik-Elektroden-Baugruppe 12 gezeigt, wobei bei diesem Ausführungsbeispiel auch eine hülsenartig z.B. bis zum Bereich der Anode ragende Einhausung 13 mit Lufteinlass 13.1 und eine Abdeckung 14 (insbesondere als Schutz gegen Regen bzw. Witterungseinflüsse ausgeführt) mit Befestigungsmitteln 14.1 (insbesondere Schrauben in Kombination mit Distanzhülsen) vorgesehen sind. Dabei ist zu verstehen, dass einzelne zumindest der strukturellen Komponenten der messtechnischen Vorrichtung auch als 3D-gedruckte Komponenten ausgeführt sein können, beispielsweise ein Sensorkörper (ohne die MBZ als solche) in Kombination mit einem Schirm zum Schutz der Kathode vor Umwelteinflüssen (Niederschlag, UV-Strahlung, und dergleichen); beispielsweise können seitlich vorgesehene Luftlöcher zur kontinuierlichen Versorgung der Kathode mit Sauerstoff in Kombination mit oben am Schirm vorgesehener Kabeldurchführung (für Kabel zur Auswerteeinheit bzw. zu Mikrocontroller und Mikroelektronik) in 3D-gedruckter Form ausgestaltet sein. In **Fig. 3A** ist die messtechnische Vorrichtung 10 mit Einhausung 13 und Deckel 14 gezeigt, wobei die Einhausung 13 abgeschnitten veranschaulicht ist, insbesondere um den Blick auf die Anode freizugeben. Es ist zu verstehen, dass die funktionalen Bestandteile Lufteinlass 13.1 und Abdeckung 14 auch auf andere konstruktive Art und Weise eingebunden werden können als in den Figuren dargestellt, z.B. bei einer in additiv gefertigter, insbesondere 3D-Druck-gefertigter Ausführung, also ohne Verschraubungen oder dergleichen Verbindungs-/Befestigungsmittel. Anders ausgedrückt: Der strukturelle Aufbau des Sensorkörpers kann anwendungsspezifisch angepasst werden, z.B. auch indem mehrere der gezeigten Bestandteile zu einstückig-integralen Komponenten zusammengefasst werden. Der Fachmann kann ausgehend von der vorliegenden Offenbarung konstruktive Variationen vornehmen, ohne dass die hier beschriebene Funktionsweise einer neuen Konzipierung bedarf. Die Einhausung 13 bzw. deren Abschnitte, die mit den weiter innen liegenden Komponenten zusammenwirken, kann hier auch als Klemmbereich bezeichnet werden; in diesem Bereich kann der Übergang zwischen Keramikbauteil und Sensorkörper konstruktiv realisiert sein. Beispielsweise wird eine Kabeldurchführung genutzt, um die Keramik einzuspannen. Wahlweise kann die Keramik mit einem Gewinde ausgestattet sein, so dass mittels einer Verschraubung ein kraft-/formschlüssiger Übergang zum Reaktorkörper sichergestellt werden kann. In **Fig. 3B** wird speziell der in diesem Ausführungsbeispiel zylindrische Aufbau dargestellt. In **Fig. 3C** ist einerseits der topfartige Aufbau der Keramik 12.2 umfassend Membran 12.7 und Chassiskomponente 12.6 dargestellt, andererseits der zylindrische Mantelflächenaufbau der Elektroden 12.1, 12.3. In den Fig. 3 kennzeichnen r; z die radiale Richtung und die Hochachse bzw. lotrechte Richtung, entsprechend einer beispielhaften Ausrichtung der Membran-Elektroden-Baugruppe im zu untersuchenden Fluid.

Unter Bezugnahme auf die Fig. 3 können auch folgende vorrichtungstechnische Aspekte erläutert werden: die messtechnische Vorrichtung 10 weist wenigstens eine Öffnung zum kathodischen Bereich für Luft-/Sauerstoffaustausch auf, so dass die Funktionsweise der MET sichergestellt ist, wobei Sauerstoffkonzentrationsschwankungen insbesondere im kathodischen Bereich weitgehend vernachlässigbar sind, insbesondere da gezeigt werden kann, dass die MET bis hinunter zu ca. fünf Vol.- % Sauerstoff funktionsfähig bleibt ohne spürbare Qualitätseinbußen (erwartungsgemäß wird sich im kathodischen Bereich je nach konstruktiver Ausgestaltung eine Sauerstoffkonzentration im Bereich von mindestens 15% einstellen). Es ist zu verstehen, dass bereits der gelöste Sauerstoff im Katholyt ausreichend ist, damit die Sauerstoffreduktionsreaktion stattfinden kann, d.h. <9 mg/L (Sättigungskonzentration bei einem O2-Partialdruck von 0,21atm. bei 20°C). Dies ermöglicht auch Variationen hinsichtlich Realisierung der messtechnischen Vorrichtung umfassend eine Flüssigkatholyt-MBZ anstelle einer Luftkathoden-MBZ.

Es ist zu verstehen, dass für eine optimierte Funktionsweise ein möglichst vollständig flächiges Anliegen von Anode und Kathode an der entsprechenden Keramikmantelfläche vorteilhaft ist. Hierzu sieht die vorliegende Erfindung insbesondere vor, eine mit Druck nach (radial) außen implementierte innere Stützstruktur innen von der Kathode zu verbauen und eine mit Zugspannung nach (radial) innen implementierte äußere Stützstruktur (insbesondere als Armierung bzw. Verdrahtung ausgestaltet) außen von der Anode zu verbauen, wobei die äußere Stützstruktur vorteilhaft auch die Funktion eines Stromkollektors erfüllt. Je nach konstruktiver Implementierung können derartige Stützstrukturen jedoch auch als integraler Bestandteil von Kathode und/oder Anode ausgeführt sein.

Der Fachmann erkennt basierend auf der vorliegenden Offenbarung, dass ein möglichst hoher kontaktierender Mantelflächenanteil wünschenswert ist, und dass dieser z.B. vorrichtungstechnisch unterstützt durch Zug-/Druckkräfte verbessert werden kann, oder auch durch leichte Übermaß-"Passungen". Wahlweise kann auch ein Keramikgrundkörper zwecks Erzielung besserer Toleranzwerte nachbearbeitet werden, z.B. indem Innen- und/oder Außenmantelfläche hinsichtlich zylindrischer Rundheit nachbearbeitet werden, z.B. abgeschliffen werden. Es ist zu verstehen, dass es im Kontext der vorliegenden Erfindung zwecks Verbesserung der Langzeitstabilität von Interesse ist, die vorrichtungstechnischen Randbedingungen möglichst konstant zu halten, und dies betrifft auch die Relativposition der Komponenten der Keramik-Elektroden-Baugruppe. Anders ausgedrückt: Es ist hinsichtlich möglichst maximal konstanter Betriebsrandbedingungen zu vermeiden, dass sich die Relativposition der jeweilige Elektrode relativ zur Keramik mit der Zeit verändert, bzw. dass der kontaktierende Mantelflächenanteil schwankt.

Die in **Fig. 3C** angedeutete Bereitstellung einer/der Chassiskomponente 12.6 mittels der Keramik 12.2 ist als optional anzusehen. Vorteil: gute Steifigkeit und gute Dichtigkeit, insbesondere bei rohrartiger/zylindrischer Ausgestaltung der Keramikbrücke 12.2 zwischen Anode und Kathode. Freilich kann die Keramik auch leicht konisch ausgeführt sein/werden.

Insbesondere im Kontext mit den in den Figuren gezeigten Ausführungsbeispielen kann ergänzend auch folgendes funktionelles Beispiel erläutert werden, insbesondere mit Blick auf die erfindungsgemäße Langzeitstabilität, mit speziell hierbei getroffener Material-/Chemikalienauswahl: Anode: Anode mit Kohlenstoff-/Karbonschleier (20g/m2);
Kathode: Aktivkohle;
Membran(komponente) und wahlweise auch Chassis: Keramik, insbesondere ausgehend von einem Tontopfgrundkörper;
Chemikalienauswahl: Ammoniumacetat (C2H7NO2) als Stickstoff- und Kohlenstoffquelle, und Dinatriumhydrogenphosphat x2H2O (Na2HPO4 - 2 H2O) als Puffer und Phosphorquelle;
Betrieb: Batch-Betrieb unter Verwendung desselben Anolyten (siehe unten Tabelle [1]) Zugabe-/Fütterungszyklus: 2x pro Woche;
Als Batch-Betriebszyklus ist hier insbesondere der zyklische Substrat-Tausch zwecks Einstellen vordefinierter Versuchsbedingungen zu verstehen.

**Tabelle [1] Anolyt-Zusammensetzung**

| **NH₄-N** | **Acetat** | **PO₄-P** |
|---|---|---|
| 727 mgN/L | 3274 mg/L (3493 mgCOD/L) | 873 mgP/L |

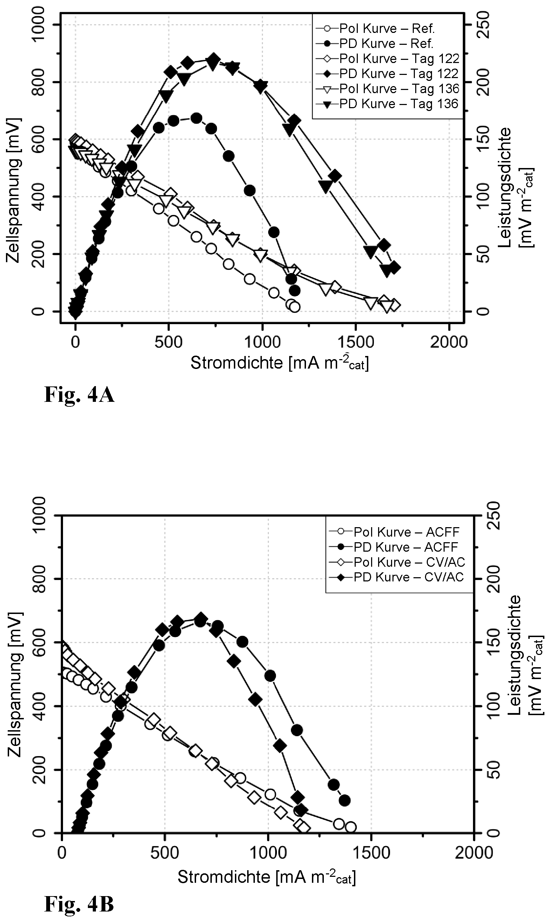

In den **Fig. 4** ist werden Ergebnisse von Langzeitversuchen für unterschiedliche Membran-Elektroden-Baugruppen visualisiert, wobei die Zellspannung (Ordinate links) und die Leistungsdichte (rechts) über der Stromdichte (Abszisse) aufgetragen sind. Randnotiz: Die in den Fig. 4 gezeigten Kurven können basierend auf der so genannten Varying Circuit Resistance (VCR) -Technik generiert werden.

In **Fig. 4A** ist beispielhaft ein Verlauf der Leistungsdichte veranschaulicht, wobei sich ein dauerhaft stabiler Prozess insbesondere auch hinsichtlich Katholyt-Haushalt einstellt. Dabei steht "PD" gemäß der Legende in Fig. 4A für die Leistungsdichte(-Kurven), und "PoI" steht für die Polarisation bzw. Polarisationskurven.

Aus Fig. 4A ist ersichtlich, dass ein Abfall der Leistungsdichte nicht auftritt. Vielmehr scheint die Leistung zu steigen, was auf eine gesteigerte Biofilmaktivität zurückgeführt werden kann. Fig. 4 liefert weitere Belege für die hier beschriebene Langzeitstabilität, hier unter beispielhafter Bezugnahme auf 136 Tage Dauerbetrieb (wobei zu verstehen ist, dass sich ein nachweisbar vergleichbarer Kurvenverlauf auch über eine Dauer von über 200 Tagen realisieren lässt): Insbesondere durch die keramische Komponente, vorteilhaft verbaut als Membran, kann direkter Abwasserkontakt der Kathode vermieden werden. Der gebildete Katholyt kann ein ständiges Feuchthalten der Kathode sicherstellen, in Kombination mit kontinuierlichem Abtransport von Salzen. Das im Stand der Technik bisher aufgetretene Scaling-Phänomen kann signifikant reduziert bzw. weitgehend vermieden werden. Fig. 4 untermauert dabei auch die Funktionsfähigkeit von in vorrichtungstechnischer Hinsicht vergleichsweise kostengünstig implementierter MBZ bzw. MET. Verglichen mit Labor-MBZ (ausgestattet mit vergleichsweise kostenintensiven Graphitfaserbürsten-Anoden und GasdiffusionsKathoden) ist ein signifikanter Leistungsunterschied nicht erkennbar. Der interne Zellwiderstand (RInt) lässt sich anhand der Polarisationskurve ermitteln. Dieser sinkt sukzessiv von 122 Ohm (REF) auf 118 Ohm (122d), 94 Ohm (136d) und letztlich auf 85 Ohm (in diesem Beispiel nach 206 Tagen). Auch dies ist eine Bestätigung der hier beschriebenen Langzeitstabilität, insbesondere da Salzablagerungen zu einer signifikanten Erhöhung des internen Widerstands führen würden.

In Fig. 4B werden zwei spezifische Materialkombinationen für die Membran-Elektroden-Baugruppe näher untersucht. Insbesondere ist dargestellt, dass die hier beschriebenen Materialien der Membran-Elektroden-Baugruppe der MET allgemein vorteilhaft für die hier beschriebenen Anwendungen der MET bzw. der messtechnischen Vorrichtung implementiert werden können, insbesondere die Materialkombination Keramik (Separator) und Aktivkohle-Graphit-Leitruss-Gemisch (für wenigstens eine Elektrode, insbesondere für den Katalysator). Es ist ersichtlich, dass Aktivkohlefaserfilz, ACFF, (verwendet als Anode und Kathode) vergleichbare Leistungen liefern kann wie Kohlenstoffschleier zur Verwendung als Anode und Aktivkohle-Graphit-Leitruss-Gemisch zur Verwendung als Kathode. Leistungsdichten >150 [mW/m2_{Cat}] können problemlos sichergestellt werden.

Dabei steht in Fig. 4B das hier so genutzte Kürzel ACFF für "Activated Carbon Fibre Felt", also für Aktivkohlefaserfilz, und das Kürzel CV für "Carbon Veil", also für Kohlenstoffschleier, und das Kürzel AC für Aktivkohle-Graphit-Leitruss-Gemisch.

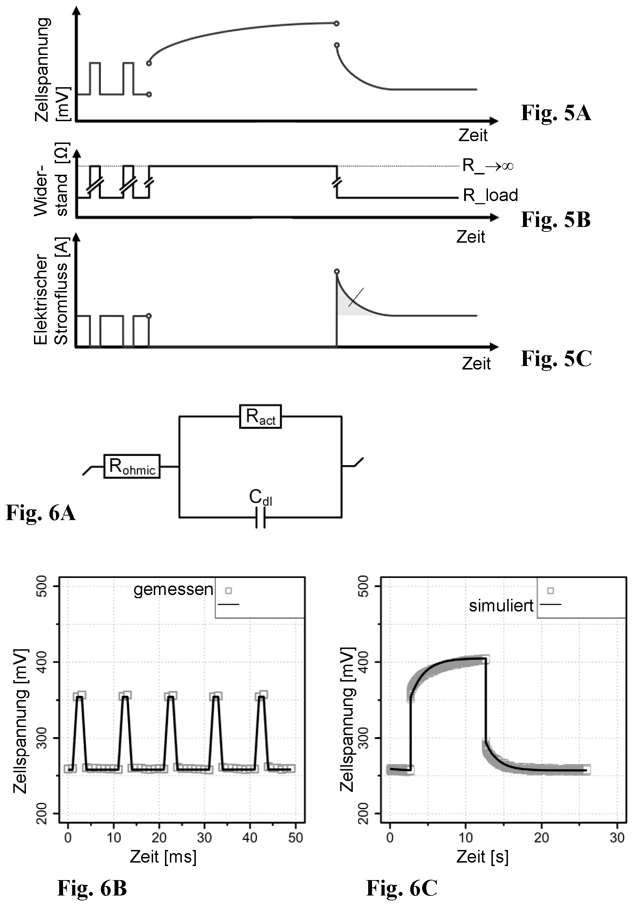

In den **Fig. 5** ist der Kontext einer im Rahmen der vorliegenden Erfindung implementierbaren Routine zur unterbrechungsfreien Parameterschätzung visualisiert, durch schematische Darstellung einer Systemantwort (Strom- und Spannungsänderung) verursacht durch frequentiertes/frequentielles Ab- und Zuschalten (Verbinden / Trennen) des externen elektrischen Lastwiderstandes (R-PWM), bei hohen und niedrigen Frequenzen. Die Auswertung des Spannungsprofils, insbesondere resultierend aus dem modulierten Lastwiderstand, kann zur Bestimmung der internen Zellwiderstände, der Kapazität sowie der Leerlaufspannung führen. In **Fig. 5A** ist der Verlauf der Zellspannung wiedergegeben. In **Fig. 5B** ist der Verlauf des Widerstands wiedergegeben; die obere gepunktete Linie steht für den Level R_{→∞} (Stromkreis unterbrochen, d.h., es liegt ein theoretisch unendlich großer Widerstand an), und die untere Linie für den Level des Lastwiderstands R_{load}. In **Fig. 5C** ist der Verlauf des elektrischen Stromflusses wiedergegeben; die gespeicherte Ladung entspricht der durch die Bezugs-Linie markierten Fläche unter der Kurve; die Ermittlung der kumulierten elektrischen Ladung kann demnach unter Bezugnahme auf denjenigen Flächeninhalt erfolgen, welcher dem aufsummierten Strom über die Zeit der Entladung des Kondensators (bzw. des Biofilms) entspricht (dargestellt durch die in Fig. 5C entsprechend hervorgehobene Fläche). Im Kontext der Betrachtung gemäß Fig. 5 kann ein vereinfachter Randle-Kreislauf (bzw. Schaltkreis) angenommen bzw. zugrunde gelegt werden. Damit kann eine modellbasierte Parameterbestimmungsroutine realisiert werden, insbesondere indem das angenommene Schaltkreismodell analytisch gelöst wird und mit Hilfe der Spannungsprofile die entsprechenden Parameter ermittelt werden. Der Vollständigkeit halber ist auch an dieser Stelle zu erwähnen, dass auch die Möglichkeit besteht, den Konzentrationswiderstand zu ermitteln.

In den **Fig. 6** ist eine Verifikation von Simulation und Messung im Kontext einer durch gezieltes Öffnen und Schließen des externen elektrischen Stromkreises generierten Systemantwort veranschaulicht. In **Fig. 6A** ist ein vereinfachter äquivalenter elektrischer Schaltkreis dargestellt (Schaltkreismodells einer MET), wobei R_{act} für den Aktivierungswiderstand steht, und C_{dl} für Doppelschichtkapazität (engl.: double-layer capacitance) steht, und R_{ohmic} für den ohmschen Widerstand steht.

In **Fig. 6B** ist eine Systemantwort (Änderung des Spannungsprofils) bei vergleichsweise hohen (Schalt-)Frequenzen gezeigt, wobei in **Fig. 6C** eine Systemantwort bei vergleichsweise niedrigen Frequenzen gezeigt ist (gemessene und simulierte Systemantwort bzw. Spannungsänderung verursacht durch Ab- und Zuschalten des externen elektrischen Lastwiderstandes bei hohen und niedrigen Frequenzen). Aus diesen Spannungsprofilen kann basierend auf dem äquivalenten elektrischen Schaltkreis eine Parameterermittlung erfolgen (insbesondere Rohmic, Ract, Cdl, elektrische Ladung, Leerlaufspannung). In den Fig. 6B, 6C entspricht die durchgehende Linie dem simulierten Verlauf, und die mit Rechtecken gekennzeichneten Punkte (bzw. bei zahlreichen Punkten einer entsprechenden breiten Linie) entsprechen gemessenen Daten(punkten). Aus Fig. 6 ist ersichtlich, dass die Systemantwort (hier: dargestellt durch die Zellspannung) durch gezieltes Öffnen und Schließen des externen elektrischen Stromkreises bei hohen Frequenzen (100Hz, 80% DC) und niedrigen Frequenzen (0,02Hz, 80% DC) ausgelöst sein kann. Die Auswertung des Spannungsprofils ermöglicht eine vergleichsweise exakte Ermittlung elektrochemischer Parameter. Hierfür kann ein vereinfachter äquivalenter elektrischer Schaltkreis (vereinfachter Randles Circuit, kurz: ECM) genutzt werden, insbesondere im Kontext einer mathematischen Beschreibung des generierten Spannungsprofils. Insbesondere basierend auf einer analytischen Lösung des Modells lassen sich elektrochemische Parameter ermitteln, beispielsweise der ohmsche Widerstand, der Aktivierungswiderstand, die Leerlaufspannung, die Zellkapazität, die Stromdichte, die Leistungsdichte, die elektrische Ladung. Überprüft werden kann die Güte der Parameterbestimmungsroutine und des ECMs insbesondere basierend auf einem Vergleich zwischen simuliertem und beobachtetem Spannungsprofil. Hier kann eine vergleichsweise niedrige RMSE (root mean square error) von nur 4,94mV erreicht werden (rel. RMSE=1.7%), d.h., der hier beschriebene vergleichsweise stark vereinfachte Modellansatz erweist sich als adäquat, um sowohl die schnellen als auch die langsamen elektrischen Dynamiken mit guter messtechnischer/datentechnischer Belastbarkeit abzubilden. Auch einem Vergleich mit gängigen elektrochemischen Methoden hält diese Parameterbestimmungsroutine stand (insbesondere der elektrochemischen Impedanzspektroskopie (EIS) und der so genannten "current interruption" (CI)-Methode; diese beiden Methoden sind jedoch an das Vorhandensein eines Potentiostaten gebunden, welcher außerordentlich kostenintensiv ist und auch keine Untersuchung im laufenden Betrieb ermöglicht); auch in solcher Hinsicht kann dieser Ansatz verifiziert werden.

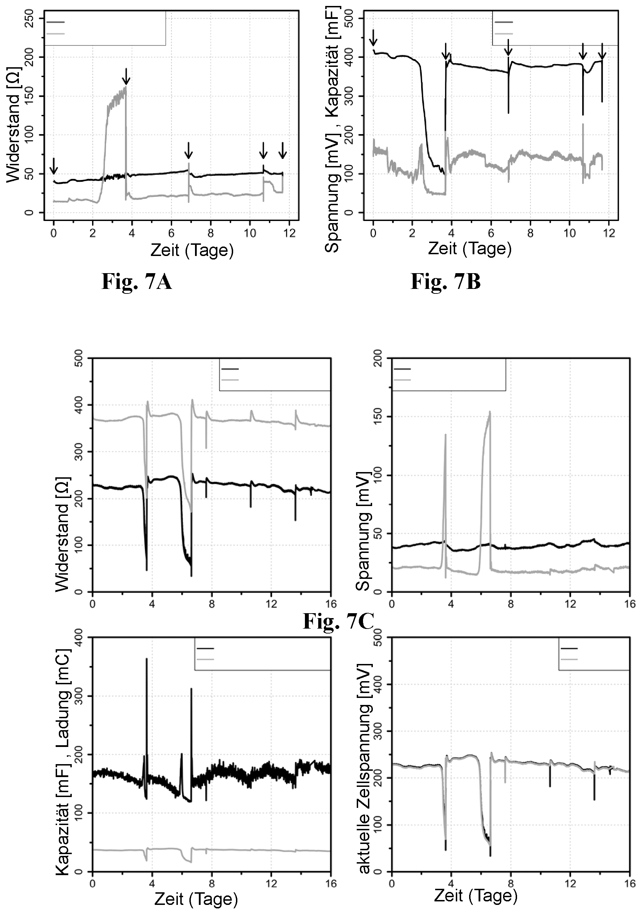

In den **Fig. 7** erfolgt eine Visualisierung einer Echtzeitbestimmung elektrochemischer Parameter sowie einer Substratabhängigkeit, insbesondere durch Bezugnahme auf die zeitliche Entwicklung elektrochemischer Parameter einer im Batch-Modus betriebenen MET und durch Vergleich der gemessenen und (basierend auf dem äquivalenten elektrischen Schaltkreismodell) simulierten Zellspannung. Der zeitliche Verlauf elektrochemischer Parameter während eines Batch-Zyklus ist vom Substratverbrauch (abnehmende COD-Konzentration) abhängig. Der Aktivierungswiderstand, der in direkten Zusammenhang mit der metabolischen Aktivität der Bakterien steht, steigt nach ca. zweieinhalb Tagen an (von 15 Ohm auf >150 Ohm), siehe **Fig. 7A****.** Im Gegensatz dazu zeigt der ohmsche Widerstand nur geringfügige Änderungen, was auf eine nur geringfügige Änderung der Leitfähigkeit zurückgeführt werden kann (wobei die Abnahme der Leitfähigkeit auf eine abnehmende Acetatkonzentration zurückzuführen ist). Analog zum Aktivierungswiderstand, nur reziprok, verhält es sich mit der Leerlaufspannung. Diese fällt nach zweitägigem konstantem Verlauf von 400mV auf 100mV ab. Dabei zeigt der Verlauf der Zellkapazität während des Batch-Zyklus Schwankungen, die direkt mit der Biofilmdichte verknüpft sind und Schwankungen in der mikrobiellen Aktivität widerspiegeln. Der fortlaufend fallende Trend der Kapazität lässt durch die Abhängigkeit der Kapazität vom Potential bzw. der ebenfalls abnehmenden Stromdichte erklären. In **Fig. 7A** ist der Widerstand über der Zeit dargestellt, wobei die dunkle vergleichsweise konstant verlaufende Linie den Verlauf des ohmschen Widerstands wiedergibt, und die hellere vergleichsweise unstetige Linie gibt den Verlauf des Aktivierungswiderstands wieder. In **Fig. 7B** sind Spannung und Kapazität über der Zeit dargestellt, wobei die dunkle weiter oben verlaufende Linie den Verlauf der Spannung des offenen Kreises wiedergibt, und die hellere weiter unten verlaufende Linie gibt den Verlauf der Doppelschichtkapazität wieder. Die nach unten weisenden Pfeile an den Kurvenverläufen der Fig. 7A, 7B zeigen die Zeitpunkte einer Neubefüllung mit Substrat an (Batch-Betrieb).

Zur Doppelschichtkapazität ist dabei zu verstehen, dass in MBZs die Umwandlung von chemischer Energie in elektrische Energie das Ergebnis intrinsischer elektrostatischer (kapazitiver) und irreversibler elektrochemischer (faradayscher) Prozesse ist. Der faradaysche Strom hängt mit der Freisetzung von Elektronen an der Anode während der anaeroben Oxidation von organischen Stoffen durch EABs zusammen. Im offenen Stromkreis sind die akkumulierten Elektronen für die Bildung der elektrischen Doppelschicht (EDL) an der Anoden/Elektrolyt-Grenzfläche verantwortlich, wo die negativen Oberflächenladungen (e-) durch im Abwasser vorhandene Gegenionen (Kationen) ausgeglichen werden. Der gleiche Prozess, jedoch mit umgekehrter Polarität, findet an der Kathode statt. Die EDL wirkt dabei wie ein Kondensator, in welchem Strom gespeichert werden kann, und ist für den kapazitiven Strom verantwortlich. Wenn der Stromkreis geschlossen ist, liefert die Anode gleichzeitig den kapazitiven Strom (aus der EDL-Bildung freigesetzte Elektronen) und den faradischen Strom, d.h. den von EABs erzeugten Strom. Der erzeugte elektrische Gesamtstrom entspricht demnach der Summe aus kapazitivem und faradayschem Strom.

In **Fig. 7C** erfolgt die Visualisierung der Echtzeitbestimmung elektrochemischer Parameter sowie der Substratabhängigkeit auch unter Bezugnahme auf die elektrische Ladung (in Ergänzung zu den in Fig. 7B gezeigten Messungen), bei Gegenüberstellung gemessener und simulierter Zellspannung. In Fig. 7C im Graph oben links ist der Verlauf von aktueller (momentaner) Zellspannung (relativ dunklere Linie) und Spannung des offenen Kreises (relativ hellere Linie) dargestellt; im Graph oben rechts ist der Verlauf von ohmschem Widerstand (relativ dunklere Linie) und Aktivierungswiderstand (relativ hellere Linie) dargestellt; im Graph unten links ist der Verlauf von Doppelschichtkapazität (relativ dunklere Linie) und kumulierter elektrischer Ladung (relativ hellere Linie) dargestellt; im Graph unten rechts ist ein Vergleich zwischen Messwerten (relativ dunklere Linie) und Simulation (relativ hellere Linie) dargestellt. Die gemessene Zellspannung wird der simulierten Zellspannung gegenübergestellt. Dabei können sowohl die ermittelten elektrochemischen Parameter als auch das angenommene Schaltkreismodell verifiziert werden; kann die gemessene Spannung gut nachgerechnet werden, so ist davon auszugehen, dass sowohl das Modell als auch die Parameterbestimmungsroutine adäquat implementiert wurden. Fig. 7C zeigt auch, dass der angenommene vereinfachte äquivalente elektrische Schaltkreis (ECM) für den Kontext der vorliegenden Implementierung geeignet ist, und dass die Parameterbestimmungsroutine korrekte Ergebnisse liefert (die gemessene Zellspannung kann basierend auf dem ECM und den ermittelten Parametern vergleichsweise exakt modelliert werden). Speziell beim gezeigten Beispiel liegt die RMSE sogar bei nur 3,3mV.

In **Fig. 8** ist ein Verfahren zum Generieren wenigstens eines energetischen/elektrochemischen Parameters aus Messdaten erhoben mittels wenigstens einer messtechnischen mikrobiellen elektrochemischen Technologieeinheit (MET) basierend auf bioelektrochemischen Prozessen veranschaulicht, beispielsweise mit den Schritten: Schritt **S1** Datenerfassung; Schritt S2 Datenauswertung; Schritt **S3** Modulation; Schritt **S4** Datenanalyse insbesondere basierend auf ML-Algorithmen; Schritt S1 umfasst insbesondere das Erheben von Messdaten mittels wenigstens einer MET basierend auf bioelektrochemischen Prozessen; Schritt S2 umfasst insbesondere das Korrelieren wenigstens eines Messwerts mit wenigstens einem Prozessparameter; dabei kann ein Generieren wenigstens eines energetischen/elektrochemischen Parameters aus den über einen Auswertungszeitraum von mindestens 30 Tagen erhobenen Messdaten erfolgen, insbesondere Messdaten aus zellinternen Widerstandskomponenten und/oder Spannungs-, Kapazitäts-, Strom-, Leistungs-, und/oder elektrischen Ladungsmessdaten. Wahlweise sind auch die Schritte S3 und/oder S4 implementiert.

Es ist zu verstehen, dass dem Schritt S1 ein initialer Kalibrierungsschritt vorausgehen kann, insbesondere im Sinne einer Grundkalibrierung, wobei für das Kalibrieren wenigstens ein ML-Algorithmus herangezogen werden kann. Dieser oder weitere Kalibrierungsschritte können auch zu anderen Zeitpunkten bzw. Verfahrensschritten ergänzend durchgeführt werden.

Die in Fig. 8 angedeuteten rautenförmigen Felder "R0" zwischen den einzelnen Schritten veranschaulichen mögliche anwendungsspezifische Anpassungs- oder Implementierungsmaßnahmen für den Fachmann (zwischengelagerte Maßnahmen), insbesondere zwischen den Schritten S1 und S2, beispielsweise indem wenigstens ein ML-Algorithmus angepasst und/oder erweitert wird (oder allgemein Auswahl des jeweiligen Prozessparameters, Festlegen von Korrelationspartnern). Zudem können nach Schritt S2 und/oder nach Schritt S3 und/oder nach Schritt S4 anwendungsspezifische Anpassungs- oder Implementierungsmaßnahmen vorgesehen sein (nachgelagerte Maßnahmen "R1"), z.B. das Generieren bzw. das Übermitteln von Stellgrößen Cᵢ eines zu steuernden/regelnden Prozesse, insbesondere eines Wasseraufbereitungsprozesses, und/oder anderweitigen Steuerungs-/Regelungsparametern.

Mit anderen Worten kann gemäß Schritt S1 eine Datenerfassung bzw. Bioindikation erfolgen, wobei ein bioelektrisches Signal erzeugt wird (Biostrom). Gemäß Schritt S2 kann daraufhin die Ermittlung der mikrobiellen Aktivität erfolgen, wobei aus Schritt S2 eine Stellgröße/Regelgröße für z.B. Belüfteraggregate oder auch Probenahmeeinrichtungen genutzt werden kann (z.B. im Rahmen einer ereignisbezogenen bzw. Event-getriebenen Probenahme). Somit kann gemäß R0 in einer Phase zwischen Schritt S1 und S2 eine Implementierungsmaßnahme betreffend eine Umrechnung von Strom-Messwerten auf den Metabolismus der Mikroorganismen vorgesehen sein. Gemäß Schritt S3 kann eine Modulation erfolgen, insbesondere indem ergänzend zu den energetischen Parametern (Biostrom, Spannung, etc.) elektrochemische Parameter ermittelt werden. Somit kann gemäß R0 in einer Phase zwischen Schritt S2 und S3 eine als Parameterbestimmungsroutine (bzw. mathematisches Modell) ausgebildete Softsensorik implementiert sein. Zusätzliche Regelgrößen (R1) bzw. P_SB bzw. PSB sind dann nicht notwendigerweise vorgesehen. Somit kann gemäß R0 in einer Phase zwischen Schritt S3 und S4 eine Datenanalyse insbesondere unter Bezugnahme auf wenigstens einen ML-Algorithmus vorgesehen sein. Beispielsweise wird ein ANN vortrainiert (kalibriert), und auf dieser kalibrierten Basis werden Prozessparameter wie CSB, BSB, TN, NH4-N, etc. ermittelt. Diese können dann z.B. im Rahmen eines Steuerungs-/Regelungsprozesses z.B. zur Vorgabe der Art und Weise einer Belüftung oder auch zur Steuerung einer Probenahmeeinrichtung genutzt werden.

Es ist zu verstehen, dass der in Fig. 8 angedeutete Datenoutput P_{SB} allgemein für Daten betreffend wenigstens einen Parameter steht, insbesondere Daten zum BSB, CSB, BCOD, zur CCR und/oder weiteren Parametern umfassen kann.

In **Fig. 9** wird auf den Umstand Bezug genommen, dass eine Korrelation von Messwerten in Hinblick auf eine Bestimmung des CSB auch im Kontext von BSB (bzw. BOD biological oxygen demand), Kohlenstoffverbrauchsrate und Stofffraktionen flüchtiger Fettsäuren stehen mag, insbesondere basierend auf leicht konkretisierbaren Abhängigkeiten, beispielsweise auch auf bilanzielle Weise. Der im Deutschen so bezeichnete C_{CSB,abb} und im Englischen so genannte "biodegradable COD" (BCOD), entsprechend dem CSB abzüglich allein der nicht-inerten Anteile, kann insbesondere basierend auf einem/dem beispielhaften in Fig. 9 veranschaulichten Zusammenhang (BSB-Respirationsmessung im Kontext der Absorption von CO2) ermittelt werden, wobei die dargestellten Punkte bzw. Kreise die Messpunkte veranschaulichen und wobei die Kurve den daraus ermittelten Verlauf veranschaulicht. Auf der Abszisse ist die Zeiteinheit für den BSB1, BSB5, usw.usf. angegeben. Die Parameter K_{BSB} (bzw. k_{BOD}) und f_{BSB} (bzw. f_{BOD}) bezeichnen dabei die so genannte "first order rate constant" und den so genannten Wegsterbe- bzw. Korrektur-Faktor (im Bereich 0,1 bis 0,2). Als BSBₜₒₜ (bzw. BODₜₒₜ) wird der extrapolierte BOD bezeichnet, berechenbar durch Einbezug der Zeit-Variablen bzw. Zeit-Abhängigkeit (vergleiche BSBₜ in Gleichung in Fig. 9). In Klammern sind hier die im Englischsprachigen geläufigen Abkürzungen wiedergegeben. Dabei unterteilt sich der COD bzw. CSB (insbesondere bei Abwässern) in die Haupt-Fraktionen BCOD und "non-biodegradable COD", und diese beiden Haupt-CSB-Fraktionen unterteilen sich in löslichen COD und partikulären COD, und der lösliche BCOD unterteilt sich weiter in VFA einerseits und komplexe Bestandteile andererseits. Darauf basierend kann die Fraktionierung zur analytischen Auswertung vollzogen werden, insbesondere auch in Echtzeit. Die erfindungsgemäße möglichst detaillierte Aufschlüsselung bzw. Fraktionierung (insbesondere Echtzeit-Fraktionierung) des CSB kann daher auch eine besonders hohe Datengüte und hohe Belastbarkeit etwaiger aus der messtechnischen Analyse gewonnener (Stell-)Parameter sicherstellen. Aus Fig. 9 geht demnach auch hervor, dass die hier beschriebene Messtechnik auf elegante Weise eine vergleichsweise einfache Ermittlung des BCOD ermöglichen kann, welcher üblicherweise bisher wegen zu hohen Aufwandes nicht ermittelt wurde, zumindest im Feld bzw. von Anlagenbetreibern (im Feld wird allenfalls noch der BSB5 ermittelt). Der COD bzw. CSB allgemein kann auf chemische Weise ermittelt werden.

Erwähnenswert ist, dass Sensor und Parameterauswahl basierend auf dem Konzept der vorliegenden Erfindung auch nach einer Art Baukastenprinzip angepasst werden können, beispielsweise wie folgt:
1) messtechnische Vorrichtung in Grundausführung (Basissensor) zur Überwachung mikrobieller Aktivität (im Sinne einer ersten Ausbaustufe);
2) Basissensor in Kombination mit anwendungsspezifisch implementierten/implementierbaren Abwasserqualitätsparametern (im Sinne einer zweiten Ausbaustufe), wobei durch Softsensorik auf CSB, BSB5, TN, NH4-N, etc. rückgeschlossen werden kann (mit TN entsprechend dem Gesamtstickstoffgehalt in der untersuchten Probe bzw. im Abwasser);
3) Basissensor in Kombination mit implementierten Abwasserqualitätsparametern, zuzüglich physikalischer Messsensorik (Temperatur, pH, etc.), im Sinne einer dritten Ausbaustufe; dabei können z.B. auch zusätzlich Umwelteinflüsse Berücksichtigung finden (Stichwort: Multiparametersonde), insbesondere zwecks Steigerung der Genauigkeit der Messung;

Im Kontext der Fig. 9 ist an dieser Stelle auch nochmal der so genannte Ertragskoeffizient Y (bzw. Ertragsrate) zu erwähnen, über welchen unter Bezugnahme auf Elektronenfluss und Elektronenbilanzen oder, für praktische Zwecke, unter Bezugnahme auf CSB-Bilanzen (mit dem CSB als Maß für die verfügbaren Elektronen) zwischen entnommenem Substrat, erzeugter Biomasse und verbrauchtem gelöstem Sauerstoff bilanziert werden kann. Der "wahre" Ertragskoeffizient, der üblicherweise in CSB-Einheiten ausgedrückt wird, beschreibt dabei die Menge an Biomasse, die pro Einheit des abgebauten Substrats gebildet wird. Unter Bezugnahme auf den Ertragskoeffizienten können der Anteil der durch die Biosynthese aufgenommenen Elektronen und der Anteil, der mit dem Elektronenakzeptor gekoppelt ist (für Energiereaktionen verwendet), ermittelt werden.

Es ist zu verstehen, dass die in Fig. 9 gezeigten Zusammenhänge insbesondere auch im Rahmen einer (Grund-)Kalibrierung vor dem bzw. beim Bereitstellen einer hier beschriebenen messtechnischen Vorrichtung genutzt werden können, insbesondere zum Bereitstellen einer/der Funktionalität des Basissensors (messtechnische Vorrichtung mit Grundausstattung, eingerichtet für weitere anwendungsspezifische Implementierung).

Im Rahmen der vorliegenden Offenbarung wird der Spannung bzw. Zellspannung üblicherweise das Symbol U zugeordnet, bei MBZs auch U_MBZ, und die Stromdichte wird üblicherweise mit dem Symbol j bezeichnet. Zusätzlich wird üblicherweise die Angabe gemacht, auf welche Elektrodenfläche die Stromdichte bezogen wird (üblicherweise auf die Kathode, da diese limitierend ist). Dies führt dann zur folgenden Bezeichnung: j_Kat.

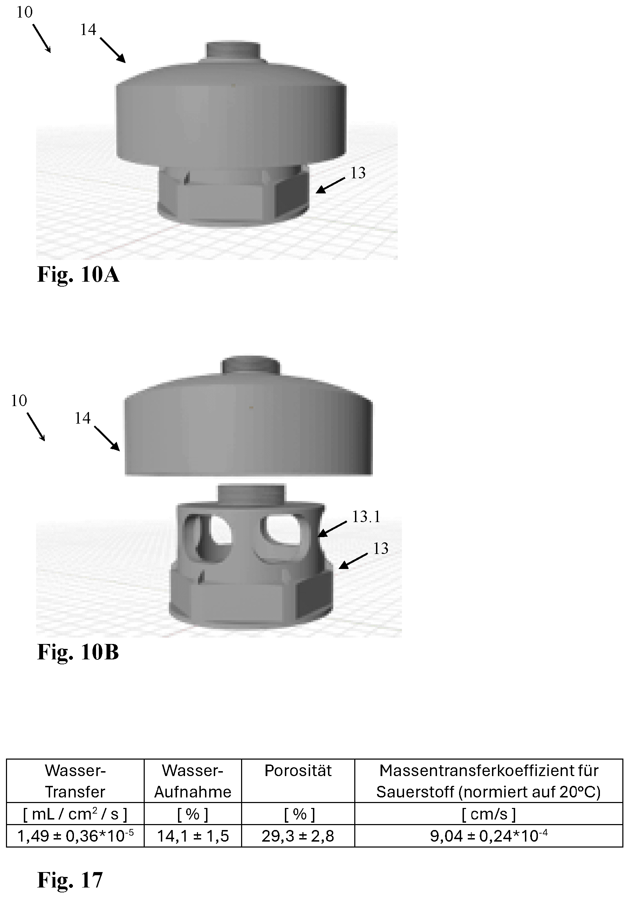

In den **Figuren 10A** und **10B** ist ein weiteres Ausführungsbeispiel für einen vorteilhafte strukturellen Aufbau der messtechnischen Vorrichtung 10 gezeigt, insbesondere für Anwendungen im Feld, bei welchen die Vorrichtung 10 in ein Becken, in einen Kanal, in ein Gewässer oder dergleichen eingebracht werden können soll. In **Fig. 10A** ist ein geschlossener Zustand mit auf der Einhausung 13 montiertem Deckel (bzw. Schutzschirm) 14 gezeigt, z.B. mit verschraubtem Deckel. **Fig. 10B** zeigt in Explosionsdarstellung einen/den demontierten Schutzschirm 14 und die sich darunter befindlichen Lufteinlässe 13.1.

Es ist zu verstehen, dass die in den Fig. 3 und 10 gezeigten Ausführungsformen der messtechnischen Vorrichtung 10 auch mit einer Abtriebssicherung ausgestattet sein können, also mit Mitteln zur Vermeidung einer örtlichen Verlagerung der messtechnischen Vorrichtung 10 im Feld. Beispielsweise weist ein Deckel (bzw. eine Auftriebskörperkomponenten) der messtechnischen Vorrichtung 10 eine Bohrung auf, welche mit einem Stab kuppelt, wobei der Stab bei durch die Bohrung gesteckter Anordnung eine Verankerung der bestimmungsgemäß aufschwimmenden messtechnischen Vorrichtung 10 sicherstellen kann, insbesondere am Untergrund/Boden. Die messtechnische Vorrichtung 10 kann dabei entlang des Stabs dem Wasserpegel folgend hoch und runter pendeln, ohne die bestimmungsgemäße Position zu verlassen. Dabei kann die messtechnische Vorrichtung 10 mittels des Deckels oder auch mittels zusätzlicher Schwimmkörperelemente als auf dem Wasser aufschwimmende Sensorik ausgestaltet sein. Beispielsweise kann unterhalb vom Deckel ein zusätzlicher ringartiger Schwimmkörper vorgesehen sein (z.B. im Durchmesser ca. 25cm bis 30cm), mittels welchem ein Aufschwimmen in der gewünschten relativen Höhenposition zur Wasseroberfläche sichergestellt werden kann.

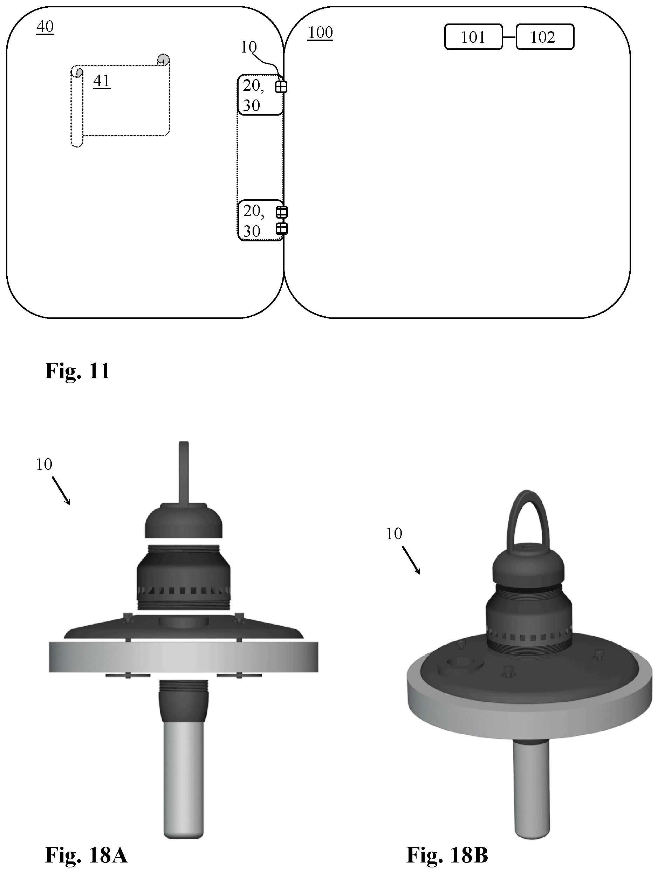

In **Fig. 11** ist eine/die messtechnische Vorrichtung 10 (wenigstens eine messtechnische Vorrichtung 10) als Bestandteil wenigstens eines/des messtechnischen Systems 20 gezeigt, welches in ein System 40 mit Plattformfunktionalität mit Zugang zu einer grafischen Benutzerschnittstelle 41 (oder diese umfassend) insbesondere zur Auswertung und Visualisierung von im Kontext mit der hier beschriebenen Messtechnologie generierten Daten implementiert ist. Die Plattform bzw. ein Dashboard oder dergleichen Visualisierungstool kann dabei auch über ein beliebiges Endgerät für einen autorisierten Nutzer zugänglich sein, insbesondere über das hier beschriebene Computerprogramm(produkt) bzw. die entsprechende Benutzerschnittstelle (Mensch-Maschine-Schnittstelle bzw. Human Machine Interface, HMI). Auf diese Weise kann z.B. eine (Ab-)Wasseraufbereitungsanlage 100 über das System 40 im Kontext mit durch eine jeweilige messtechnische Vorrichtung 10 oder ein übergeordnetes messtechnisches Systems 20 angesteuert werden, z.B. indem mit dessen Steuerungs-/Regelungseinheit 101 kommuniziert wird, z.B. über ein Kommunikationsmodul 102. Gleichermaßen kann auch ein Datenempfang erfolgen (bilaterale Kommunikation), z.B. betreffend anlagenspezifische Online-Messdaten wie Volumenstrom, Leitfähigkeit, Temperatur.

Bei den in den Figuren 2 und 3 und 10 und 11 gezeigten vorrichtungstechnischen Komponenten der messtechnischen Vorrichtung sind beispielsweise (mikro)elektronische Komponenten oder Datenverarbeitungs-Komponenten nicht im Einzelnen dargestellt. Diesbezüglich wird auf die Beschreibung zu den weiteren Ausführungsbeispielen verwiesen, insbesondere auch auf Figur 1.

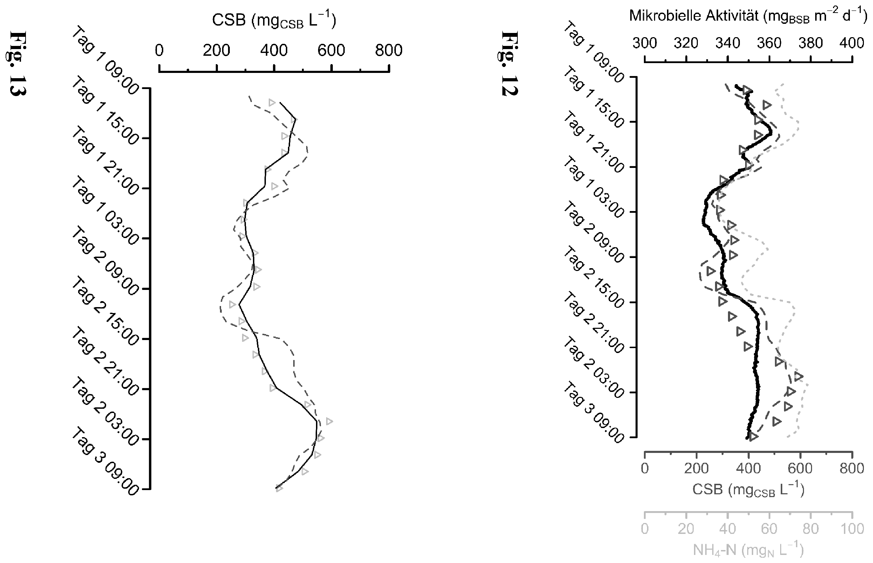

In **Fig. 12** ist eine Bewertung der Trendsensorik der messtechnischen Vorrichtung ersichtlich, basierend auf einem Vergleich zwischen dem Rohsignal (CCR, Kohlenstoffverbrauchsrate) der messtechnischen Vorrichtung, 2h-Mischproben (Laboranalytik betreffend den CSB) und den Messwerten einer UV-Spektrometersonde (für CSB-Äquivalent) und der Ammoniumstickstoffkonzentration (NH4-N, nasschemisch), im realen Kläranlagenbetrieb. Es ist ersichtlich, dass das bioelektrochemische Antwortsignal (CCR) auch ohne Kalibrierung hohe Korrelationen zu den erhobenen Online- und Labormessungen zeigt: Die Korrelationskoeffizienten (Pearson) betragen: Rxy = 0.66 (2h-Mischprobe), Rxy = 0.84 (UV-Sonde). Dabei wird hauptsächlich der biologisch abbaubare CSB gemessen, also nicht der absolute CSB (Inertanteile daraus indirekt über Korrelation). Für den Zeitpunkt Tag 2, ca. 14 Uhr zeigt der Verlauf eine zusätzliche Schlammwassereinleitung, welche vermutlich temporär die Konzentration an VFAs erhöht. Dadurch ist der Anstieg des mittels der messtechnischen Vorrichtung gemessenen Sensorsignals zu erklären, bei gleichzeitig wenig verändertem Gesamt-CSB. Erwähnenswert ist, dass der ohmsche Widerstand der messtechnischen Vorrichtung einen außerordentlich hohen Pearson-Korrelationskoeffizienten von -0.83 aufweist. Randnotiz: Schlammwasser hat, verglichen mit Rohabwasser, eine vergleichsweise hohe Leitfähigkeit aufgrund von Ammoniumionen und VFAs. In Fig. 12 ist auf der Ordinate rechter Hand ist neben dem CSB auch das NH4-N Äquivalent dargestellt, auf einer Skala von 0 bis 100 [mg_{N}L⁻¹]. Die gepunktete Kurve betrifft die nasschemische Messung (NH4-N), der durch Dreiecke gekennzeichnete Verlauf betrifft die 2h-Mischprobe, die durchgehende Linie betrifft die Messwerte der MET, und die Strichlinie betrifft die UV-Spektrometersonde.

In Fig. 12 werden R2-Werte (Bestimmtheitsmaß) bezüglich Vergleichsmessungen mit einer UV-Sonde bzw. Labormessungen präsentiert. Es ist jedoch zu verstehen, dass auch Vergleichsmessungen unter mehreren simultan eingesetzten messtechnischen Vorrichtungen bzw. METen aufzeigen, dass die erhobenen Daten eine hohe Güte aufweisen. Insbesondere haben Vergleichsmessungen bei mindestens dreifacher messtechnischer Redundanz der hier beschriebenen Sensorik im selben örtlichen Bereich einer Kläranlage gezeigt, dass außerordentlich gleiche und reproduzierbare Ganglinien insbesondere der CCR (Kohlenstoffverbrauchsrate) generiert werden können, insbesondere bei sehr hohen Pearson-Korrelationskoeffizienten im Bereich zwischen 0,91 bis 0,98 (Dauer des Vergleichsversuchs z.B. mindestens 40 Tage). Diese bioelektrochemischen Antwortsignale sind im Trend als nahezu identisch einzustufen, wodurch Reproduzierbarkeit der Messsignale belegt ist. Die Vergleichbarkeit der messtechnischen Signale unter mehreren der hier beschriebenen messtechnischen Vorrichtungen bzw. METen kann demnach auch im Feld nachgewiesen werden, bei Korrelationskoeffizienten >0,9.

In **Fig. 13** ist eine Bewertung der messtechnischen Vorrichtung hinsichtlich CSB-Sensorik-Funktionalität ersichtlich. Es erfolgte eine Kalibrierung der messtechnischen Vorrichtung basierend auf einem so genannten Random-Forests-Regressionsmodell, und der Vergleich bzw. die Plausibilitätsprüfung wurde anhand von 2h-Mischproben durchgeführt. Eingangsgrößen: CCR, ohmsche Widerstand, Aktivierungswiderstand, Leerlaufspannung, Doppelschicht-Kapazität und Abwassertemperatur. Die Random-Forests-Regression wurde mit so genanntem Random Search Hyperparameter-Tuning sowie mit so genannter 5-Fold Cross-Validation implementiert. Ergebnis: Bestimmtheitsmaß R2=0,965 ; RMSE: 20,03 mgCSB/L ; relRMSE: 5,1% .

Dabei betrifft der durch Dreiecke gekennzeichnete Verlauf die 2h-Mischprobe, und die durchgehende Linie betrifft die Messwerte der MET, und die Strichlinie betrifft die UV-Spektrometersonde.

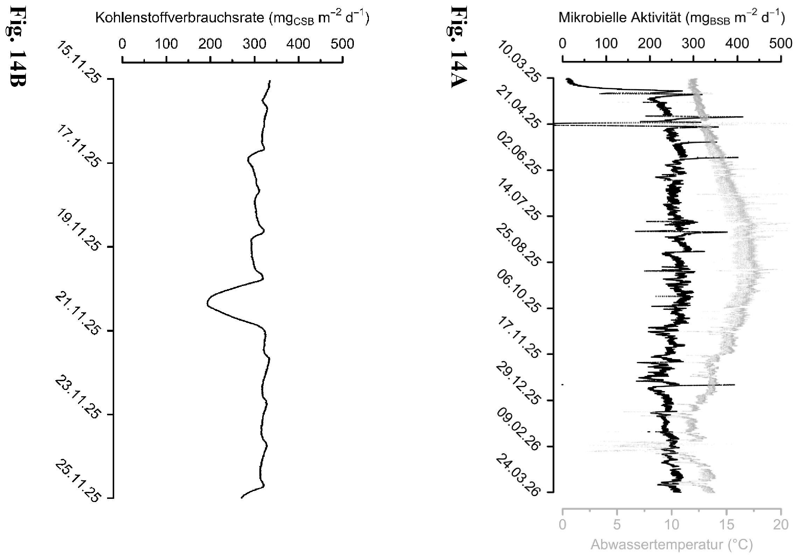

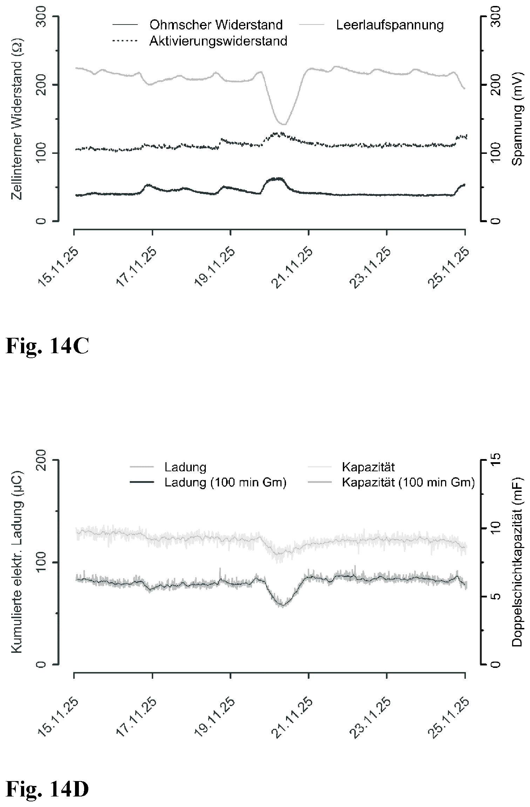

In den **Figuren 14** wird die messtechnische Leistungsfähigkeit der hier beschriebenen messtechnischen Vorrichtung insbesondere für einen Einsatz im realen Kläranlagenbetrieb weiter belegt, unter Bezugnahme auf Ganglinien der mikrobiellen Aktivität über einen Zeitraum von 379 Tagen im realen Kläranlagenbetrieb, insbesondere zur Beurteilung der Langzeitstabilität der messtechnischen Vorrichtung, wobei jeweils eine Ganglinie der Kohlenstoffverbrauchsrate (Maß für die mikrobielle Aktivität), der zellinternen Widerstandskomponenten (ohmscher Widerstand und Aktivierungswiderstand), der Leerlaufspannung, der elektrischen Ladung und der Doppelschichtkapazität im realen Kläranlagenbetrieb und die Auswirkungen eines Regenereignisses (am 20.11.2025.) dokumentiert sind.

In **Fig. 14A** ist eine Ganglinie der mikrobiellen Aktivität [mg_{BSB} m⁻² d⁻¹] über einen Zeitraum von ca. einem Jahr gezeigt, wobei auch die Abwassertemperatur erfasst wurde. Deutlich zu erkennen ist, dass kein Abfall der messtechnischen Leistungsfähigkeit erfolgt. Die MET bzw. die gesamte messtechnische Vorrichtung musste über den gesamten Zeitraum (379d) nicht einmal gewartet werden. Dies belegt die Langzeitstabilität und damit auch die Eignung für exakte bzw. belastbare Datentrainings. Auf der Ordinate rechter Hand ist die Abwassertemperatur in [°C] auf einer linearen Skala von 0 bis 20 dargestellt. Weiterer Beleg für die Langzeitstabilität ist der nahezu konstante ohmsche Widerstand, welcher als Indikator für Materialalterung herangezogen werden kann. Dieser schwankt lediglich aufgrund von Änderungen der Abwasserzusammensetzung (Niederschlag, Tag/Nacht-Zyklen) zwischen 90 und 150 Ohm; auch ein ansteigender Trend ist nicht zu erkennen.

In **Fig. 14B** ist eine Ganglinie der Kohlenstoffverbrauchsrate [mg_{CSB} m⁻² d⁻¹] bei Trocken- und Regenwetter gezeigt. Deutlich zu erkennen sind die Tagesgänge bei Trockenwetter. Der starke Abfall zum Mess-Zeitpunkt 20.11.25 (x-Achse) lässt sich auf ein Regenevent zurückführen.

In **Fig. 14C** ist eine Ganglinie des zellinternen Widerstandes [Ω] (unterste Kurve) unter Bezugnahme auf die Spannung [mV] gezeigt; zudem ist der Verlauf der Leerlaufspannung (oberste Kurve) und des Aktivierungswiderstands (mittlere Kurve, gepunktete Linie) gezeigt.

In **Fig. 14D** ist eine Ganglinie der kumulierten elektrischen Ladung [µC] unter Bezugnahme auf die Doppelschichtkapazität [mF] gezeigt; dabei gibt die obere Kurve die Kapazität bzw. die Kapazität (100min. Gm) wieder, und die untere Kurve gibt die Ladung bzw. die Ladung (100min. Gm) wieder; dabei bedeutet "Gm" gleitender Mittelwert. Diese repräsentativen Ganglinien der elektrochemischen Parameter bei Trocken- und Regenwetter (vergleiche jene zur CCR in Fig. 14B) verdeutlichen Parameteränderungen bei Regenevents: Steigt der ohmscher Widerstand, so sinkt die Leitfähigkeit; steigt der Aktivierungswiderstand, so sinkt die mikrobielle Aktivität; sinkt die Leerlaufspannung, so erfolgt eine Abnahme des Anodenpotentials (weniger Substrat vorhanden); sinken Kapazität und Ladung, so werden im Biofilm weniger Elektronen gespeichert, da weniger Substrat verstoffwechselt wird.

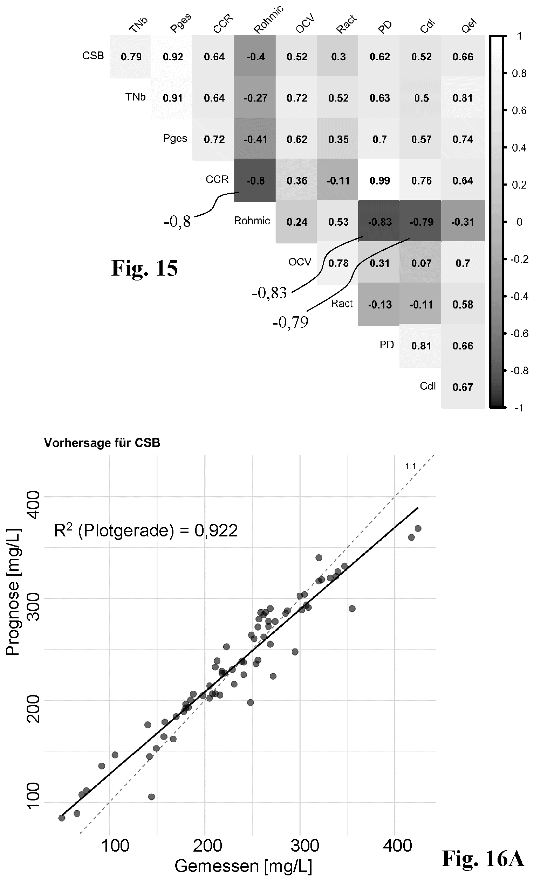

In **Fig. 15** ist erkennbar, dass im Zusammenhang mit dem hier beschriebenen Ansatz hohe Korrelationen zwischen den erhobenen energetischen und elektrochemischen Biosensordaten und ausgewählten Abwasserqualitätsparametern (CSB, TNb, Pgesamt) vorhanden sind, wobei in der Korrelationsmatrix so genannte Pearson-Korrelationskoeffizienten wiedergegeben sind. Hier wurden die Korrelationen (Pearson, n=73) zwischen den energetischen und elektrochemischen Parametern der hier beschriebenen messtechnischen Vorrichtung und den durchflussproprotionalen Tagesmischproben für den chemischen Sauerstoffbedarf (CSB), den gesamten gebundenen Stickstoff (TNb) und Gesamtphosphor (Pgesamt) bestimmt, indem zwischen durchflussproportionalen 24h-Proben (n=73) einer Kläranlage mit den Tagesmittelwerten der mittels der messtechnischen Vorrichtung erfassten Messdaten verglichen wurde. Ab einem Pearson-Korrelationskoeffizient >0,5 kann von hohen Korrelationen gesprochen werden. Dabei ist zu verstehen, dass nach Erstellen einer derartigen Korrelationsmatrix bei der weiteren nachgelagerten Auswertung bevorzugt mit der jeweils besonders guten Korrelation (je Spalte) gearbeitet werden kann. "PD" steht dabei für "power density" bzw. für die Leistungsdichte(-Kurve), und C_{dl} für Doppelschichtkapazität (engl.: double-layer capacitance), und OCV für die Leerlaufspannung, und "TNb" für den gesamten gebundenen Stickstoff, und "Qel" für die elektrische Ladung, und Ract für den Aktivierungswiderstand. Die drei auf besonders dunklem Hintergrund wiedergegebenen Korrelations-Werte (-0,8 und -0,83 und -0,79) sind hier der besseren Sichtbarkeit separat bezeichnet.

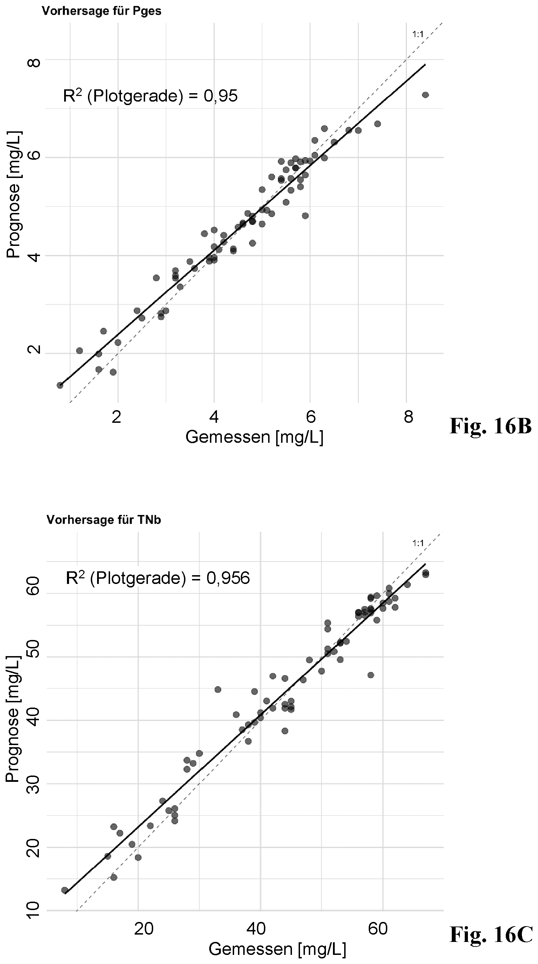

In den **Figuren 16** wird der Aspekt einer Kalibrierung weiter thematisiert, insbesondere unter Bezugnahme auf eine bestimmte Technik des Trainierens von KI-Modellen. Dabei zeigt die jeweilige Hochachse (Ordinate) die prognostizierten Werte, und die Abszisse nimmt Bezug auf die gemessenen Werte/Daten. Speziell wurde für das Training ein so genannter Supervised Learning-Ansatz verfolgt, insbesondere unter Anwendung eines so genannten Random Decision Forests-Regressionsansatzes. Als Eingangsdaten sind die Messdaten der messtechnischen Vorrichtung vorgesehen: CCR, Rohmic, OCV, Ract, PD, Cdl, Qel; als Zielgrößen sind vorgesehen: CSB, TNb, und Pges. In **Fig. 16A** ist eine Gegenüberstellung zwischen beobachteten und vorhergesagten (Random-Forest-Regressionsmodell) durchflussproprotionalen Tagesmischproben für den chemischen Sauerstoffbedarf (CSB) gezeigt. **Fig. 16B** zeigt eine solche Gegenüberstellung für den gesamten gebundenen Stickstoff (TNb). **Fig. 16C** zeigt eine solche Gegenüberstellung für den Gsamtphosphor (Pgesamt). Der relative RMSE für CSB, TNb und Pges liegt bei 10,0%, 7,5% und 8,2%.

In **Fig. 17** ist mit Tabelle [2] ein Ausführungsbeispiel für die Beschaffenheit der Keramik der messtechnischen Vorrichtung gezeigt, unter Bezugnahme auf Transportparameter für Wasser und Sauerstoff, die in Abhängigkeit von der Porosität und der Wasseraufnahme gelistet sind, wobei jeweils der Mittelwert und die Standardabweichung angegeben sind:

**Tabelle [2]**

| **Wasserstransport** | **Wasseraufnahme** | **Porosität** | **Massentransferkoeffizient für Sauerstoff** (normiert auf 20°C) |
|---|---|---|---|
| [mL / cm² / s] | [ % ] | [ % ] | [ cm/s ] |
| 1,49 ± 0,36*10⁻⁵ | 14,0 ± 1,5 | 29,3 ± 2,8 | 9,04 ± 0,24*10⁻⁴ |

Der Sauerstoffeintrag ist in diesem Ausführungsbeispiel vergleichbar mit jenem bei Gasdiffusionskathoden mit PTFE als Gasdiffusionsschicht.

Es ist zu verstehen, dass auch die Porengröße einen Einfluss auf die messtechnischen oder zumindest betrieblichen Charakteristika der Keramik haben kann. Es hat sich im Rahmen der vorliegenden Untersuchungen gezeigt, dass vorteilhaft ein heterogenes Porennetzwerk mit Porengrößen sowohl im Makroporenbereich (>50nm) als auch im Mesoporenbereich (2nm bis 50nm) vorliegt, also mit einem ausgeprägt hohen prozentualen Anteil im Übergangsbereich von Meso- zu Makroporen. Insbesondere liegt der überwiegende Anteil der Poren der Keramik in einem oberen Größenspektrum der Mesoporen bis hinunter zu ca. 30µm, insbesondere bis hinauf zu ca. 85nm im unteren Größenspektrum der Makroporen. Ein Mikroporenanteil kann, wenn überhaupt vorhanden, vergleichsweise klein sein. Der Fachmann kann ausgehend von der vorliegenden Offenbarung Untersuchungen zur Porengröße in Korrelation mit den hier aufgeführten Parametern für den Stofftransport durchführen, um eine für eine jeweilige Anwendung jeweils optimale Beschaffenheit der Keramik vorzugeben.

In den **Figuren 18** wird eine mit einem unterhalb des Deckels vorgesehenen Schwimmkörper ausgestattete messtechnische Vorrichtung 10 gezeigt, welche mit einer Abtriebssicherung ausgestattet ist. Vorteilhaft ist die Abtriebssicherung durch eine Bohrung in Deckel und Schwimmkörper in Kombination mit einem dadurch gesteckten Stab (hier nicht gezeigt) implementiert, zur Verankerung der messtechnische Vorrichtung 10, z.B. auch in einem Zulaufbereich und/oder in einem Belebungsbecken einer biologischen Reinigungsstufe. In **Fig. 18A** ist der Aufbau in leichter Explosionsdarstellung gezeigt; es wird darin auch deutlich, dass dank des Schwimmkörpers auf exakte Weise eine (Mindest-)Eintauchtiefe für den keramischen Teil der MET definiert werden kann, z.B. mindestens 5cm. In **Fig. 18B** ist in perspektivischer Draufsicht die Bohrung sichtbar, durch welche ein Stab zur Verankerung (nicht gezeigt) gesteckt werden kann.

Es ist zu verstehen, dass die in den Figuren beschriebenen Ausführungsformen der messtechnischen Vorrichtung 10 vorrichtungstechnisch bevorzugt derart ausgestaltet sind, dass die Keramik bzw. die Anode mit einer Eintauchtiefe von mindestens 3cm, bevorzugt mindestens 5cm bis mindestens 10cm im zu untersuchenden Fluid eingetaucht wird. Dies kann die messtechnische Genauigkeit weiter verbessern, insbesondere da im Oberflächenbereich (insbesondere bei weniger als 3cm zur Oberfläche) eine andere/höhere Sauerstoffkonzentration vorliegen mag, und da bis in eine Tiefe von 10cm mit einem anormalen Oxidationsverhalten zu rechnen sein könnte. Anders ausgedrückt: Ab Eintauchtiefen von ca. 10cm und mehr können derlei Nebeneffekte weitgehend ausgeschlossen werden und die messtechnische Güte weiter verbessert werden. Der Fachmann kann die Keramik bzw. den MBZ-Bereich derart relativ zum Deckel bzw. zu einem dazwischen vorgesehenen Schwimmkörper anordnen, dass diese bevorzugte Mindest-Eintauchtiefe sichergestellt ist.

### Bezugszeichenliste

- 1: organische Abfälle oder dergleichen
- 3: Mikroben
- 5: Moleküle
- 10: messtechnische Vorrichtung
- 11: messtechnische mikrobielle elektrochemische Technologieeinheit (MET)
- 11.1: kathodischer Bereich
- 11.3: Biorezeptor(ebene)
- 11.5: Transducer
- 11.7: Mikroelektronikmodul
- 12: Membran-Elektroden-Baugruppe
- 12a: anodischer Bereich
- 12b: kathodisches Kompartiment
- 12.1: Anode
- 12.2: Keramik, insbesondere zum Abschotten des kathodischen Bereichs
- 12.21: Keramikaußenmantelfläche
- 12.23: Keramikinnenmantelfläche
- 12.3: Kathode
- 12.31: Katalysatorschicht
- 12.32: Stromkollektor, insbesondere mit Metallgewebe
- 12.4: Stützstruktur (innere Stützstruktur)
- 12.5: Verdrahtung/Armierung, insbesondere Edelstahldrähte (äußere Stützstruktur)
- 12.6: Chassis, Reaktorkörper
- 12.7: Membran bzw. Membrankomponente
- 13: Einhausung
- 13.1: Lufteinlass
- 14: Deckel bzw. Abdeckung
- 14.1: Befestigungsmittel, insbesondere Schrauben in Kombination mit Distanzhülsen
- 15: Energiespeichereinheit
- 16: Logikeinheit
- 16.1: Mikrocontroller
- 17: Kommunikationsmodul
- 20: messtechnisches System
- 21: Datenbank
- 22: Recheneinheit
- 30: Sensorik umfassend wenigstens eine messtechnische Vorrichtung
- 31: Bodensensorik
- 32: Gewässersensorik
- 33: Abwassersensorik im Allgemeinen
- 34: Aquaristiksensorik
- 35: Kanalsensorik
- 36a: Sensorik im Zulauf stromauf von Kläranlagen oder einzelner Prozessstufen
- 36b: Sensorik stromab von Kläranlagen
- 37: Sensorik in wenigstens einer prozessualen Belebungsstufe
- 38: Sensorik zur Detektion photosynthetischer Prozesse
- 39: Sensorik für Industrie- und Pflanzenkläranlagen, Grundwassersensorik
- 40: System mit Plattformfunktionalität
- 41: grafische Benutzerschnittstelle
- 100: (Ab-)Wasseraufbereitungsanlage
- 101: Steuerungs-/Regelungseinheit
- 102: Kommunikationsmodul
- BSB/CSB: biologischer/chemischer Sauerstoffbedarf
- F: zu untersuchendes Fluid
- P_{SB}: Prozessparameter insbesondere für den BSB/CSB
- R0: Regelung/Anpassung (verfahrenstechnisch zwischengelagerte Maßnahme)
- R1: verfahrenstechnisch nachgelagerte Maßnahme
- S1: Schritt S1 Datenerfassung
- S2: Schritt S2 Datenauswertung
- S3: Schritt S3 Modulation
- S4: Schritt S4 Datenanalyse insbesondere basierend auf wenigstens einem ML-Algorithmus
- Cᵢ: Stellgröße eines zu steuernden/regelnden Prozesse
- PD: Leistungsdichte(-Kurve)
- PoI: Polarisation bzw. Polarisationkurve
- CCR: Kohlenstoffverbrauchsrate
- BCOD: biodegradierbarer CSB-Parameter (auch bezeichnet als: C_CSB, abb)
- Y: Ertragskoeffizient, Ertragsrate
- r; z: radiale Richtung; Hochachse oder lotrechte Richtung

## Patentansprüche

1. Messtechnische Vorrichtung (10) eingerichtet zum Generieren von wenigstens einem energetischen/elektrochemischen Parameter aus Messdaten basierend auf bioelektrochemischen Prozessen, aufweisend wenigstens eine messtechnische mikrobielle elektrochemische Technologieeinheit (11) mit einem kathodischen Bereich (11.1) abgeschottet von zu untersuchendem Fluid, und aufweisend eine Logikeinheit (16) mit wenigstens einem Mikrocontroller (16.1) eingerichtet zum Verarbeiten von über einen Auswertungszeitraum erhobenen Messdaten, wobei die messtechnische Vorrichtung (10) eingerichtet ist zum Korrelieren wenigstens eines Messwerts mit wenigstens einem Prozessparameter (P_{SB}) basierend auf den verarbeiteten Messdaten, insbesondere mit einem Prozessparameter (P_{SB}) für den chemischen/biologischen Sauerstoffbedarf (CSB/BSB) und/oder für eine Kohlenstoffverbrauchsrate und/oder für Stofffraktionen flüchtiger Fettsäuren; **dadurch gekennzeichnet, dass** die messtechnische Vorrichtung (10) wenigstens eine zwischen kathodischem Bereich (11.1) und zu untersuchendem Fluid angeordnete und wirksame Keramik (12.2) als Bestandteil der messtechnischen mikrobiellen elektrochemischen Technologieeinheit (11) aufweist, welche den kathodischen Bereich (11.1) vom zu untersuchenden Fluid separiert, wobei die messtechnische Vorrichtung (10) mittels der wenigstens einen Keramik (12.2) eingerichtet ist zum Generieren des wenigstens einen energetischen/elektrochemischen Parameters aus über einen Auswertungszeitraum von mindestens 30 Tagen erhobenen Messdaten, insbesondere Messdaten aus zellinternen Widerstandskomponenten und/oder Spannungs-, Kapazitäts-, Strom-, Leistungs-, und/oder elektrischen Ladungsmessdaten.

2. Messtechnische Vorrichtung nach dem vorhergehenden Anspruch, wobei die messtechnische mikrobielle elektrochemische Technologieeinheit eine Membran-Elektroden-Baugruppe aufweist, bei welcher die Keramik als Membran in radial zugkraftvorgespannter Anordnung mit der Anode vorliegt und/oder bei welcher die Keramik als Membran in radial druckkraftvorgespannter Anordnung mit der Kathode vorliegt, insbesondere derart dass eine Relativbewegung zwischen entsprechender Elektrode und Keramik unterbunden ist, bei vordefiniert gleichbleibendem Mantelflächenkontaktanteil.

3. Messtechnische Vorrichtung nach einem der vorhergehenden Ansprüche, wobei die messtechnische Vorrichtung eingerichtet ist zum Generieren des wenigstens einen energetischen/elektrochemischen Parameters basierend auf Modulation, insbesondere Pulsmodulation des externen elektrischen Lastwiderstandes; und/oder wobei der wenigstens eine Mikrocontroller der messtechnischen Vorrichtung eingerichtet ist, eine Modulation, insbesondere Pulsmodulation ohne PC-Anbindung zu realisieren; und/oder wobei die messtechnische Vorrichtung ein Kommunikationsmodul eingerichtet zur drahtlosen Übermittlung von generierten Zellparametern aufweist; und/oder wobei die messtechnische Vorrichtung eingerichtet ist, die aus den erhobenen Messdaten, insbesondere Messdaten aus zellinternen Widerstandskomponenten und/oder Spannungs-, Kapazitäts-, Strom-, Leistungs-, und/oder elektrischen Ladungsmessdaten, basierend auf Modulation, insbesondere Pulsmodulation des externen elektrischen Lastwiderstandes generierten energetischen/elektrischen Parameter basierend auf wenigstens einem KI-Modell auszuwerten, insbesondere mittels eines/des in die messtechnische Vorrichtung integrierten Mikrocontrollers ohne PC-Anbindung.

4. Messtechnische Vorrichtung nach einem der vorhergehenden Ansprüche, wobei die messtechnische Vorrichtung eingerichtet ist zum Korrelieren wenigstens eines Messwerts mit einem Prozessparameter (P_{SB}) für einen/den chemischen Sauerstoffbedarf (CSB), insbesondere eines zu optimierenden Wasseraufbereitungsprozesses; und/oder wobei die messtechnische Vorrichtung eingerichtet ist zum Korrelieren wenigstens eines Messwerts mit einem Prozessparameter (P_{SB}), insbesondere CSB, unter Bezugnahme auf den biologischen Sauerstoffbedarf (BSB), insbesondere eines zu optimierenden Wasseraufbereitungsprozesses; und/oder wobei die messtechnische Vorrichtung eingerichtet ist zum Korrelieren wenigstens eines Messwerts mit einem Prozessparameter (P_{SB}), insbesondere CSB, unter Bezugnahme auf Stofffraktionen flüchtiger Fettsäuren, VFA.

5. Messtechnische Vorrichtung nach einem der vorhergehenden Ansprüche, wobei die Logikeinheit eingerichtet ist zum Auswerten von Messdaten basierend auf maschinellem Lernen, wobei Zellparameter zum Bestimmen des chemischen und/oder biologischen Sauerstoffbedarfs und/oder der Kohlenstoffverbrauchsrate und/oder von Stofffraktionen flüchtiger Fettsäuren ausgewertet werden, welche bevorzugt basierend auf Modulation, insbesondere Pulsmodulation des externen elektrischen Lastwiderstandes von aus über einen Auswertungszeitraum von mindestens 30 Tagen erhobenen Messdaten betreffend zellinterne Widerstandskomponenten und/oder Spannungs-, Kapazitäts-, Strom-, Leistungs-, und/oder elektrischen Ladungsmessdaten generiert sind/wurden.

6. Wasseraufbereitungsanlage (100), aufweisend wenigstens eine messtechnische Vorrichtung (10) nach einem der vorhergehenden Ansprüche, oder zumindest eingerichtet, in datenkommunikativer Verbindung mit dieser zustehen, und aufweisend eine Steuerungs-/Regelungseinheit (101) eingerichtet zum Steuern/Regeln eines Wasseraufbereitungsprozesses in Abhängigkeit von mittels der messtechnischen Vorrichtung (10) bevorzugt drahtlos über wenigstens ein Kommunikationsmodul (102) bereitgestellten Messdaten oder Prozessparametern.

7. Messtechnisches System (20) umfassend wenigstens eine messtechnische Vorrichtung (10) nach einem der Ansprüche 1 bis 5, aufweisend wenigstens eine Datenbank (21) und wenigstens eine Recheneinheit (22) eingerichtet zum Auswerten von Messdaten basierend auf maschinellem Lernen, wobei Zellparameter zum Bestimmen des chemischen und/oder biologischen Sauerstoffbedarfs ausgewertet werden, welche basierend auf Modulation, insbesondere Pulsmodulation des externen elektrischen Lastwiderstandes von aus über einen Auswertungszeitraum von mindestens 30 Tagen erhobenen Messdaten betreffend zellinterne Widerstandskomponenten und/oder Spannungs-, Kapazitäts-, Strom-, Leistungs-, und/oder elektrischen Ladungsmessdaten generiert sind/wurden.

8. Verfahren zum Generieren wenigstens eines energetischen/elektrochemischen Parameters aus Messdaten erhoben mittels wenigstens einer messtechnischen mikrobiellen elektrochemischen Technologieeinheit (11) basierend auf bioelektrochemischen Prozessen, wobei wenigstens ein Messwert mit wenigstens einem Prozessparameter (P_{SB}) korreliert wird, insbesondere mit einem Prozessparameter (P_{SB}) für den chemischen/biologischen Sauerstoffbedarf (CSB/BSB) und/oder für eine Kohlenstoffverbrauchsrate und/oder für Stofffraktionen flüchtiger Fettsäuren, insbesondere Acetate; wobei die Messdaten mittels der wenigstens einer messtechnischen mikrobiellen elektrochemischen Technologieeinheit (11) aufweisend wenigstens eine zwischen kathodischem Bereich (11.1) und zu untersuchendem Fluid angeordnete und wirksame Keramik (12.2) erhoben werden, wobei das Generieren des wenigstens einen energetischen/elektrochemischen Parameters aus Messdaten erhoben über einen Auswertungszeitraum von mindestens 30 Tagen erfolgt, insbesondere Messdaten aus zellinternen Widerstandskomponenten und/oder Spannungs-, Kapazitäts-, Strom-, Leistungs-, und/oder elektrischen Ladungsmessdaten.

9. Verfahren nach einem der vorhergehenden Verfahrensansprüche, wobei das Generieren des wenigstens einen energetischen/elektrochemischen Parameters eine Modulation umfasst, insbesondere eine Pulsmodulation des externen elektrischen Lastwiderstandes; und/oder wobei eine/die Modulation, insbesondere Pulsmodulation ohne PC-Anbindung vorgenommen wird, nämlich lokal an einem/dem entsprechenden Messpunkt; und/oder wobei die erhobenen Messdaten, insbesondere Messdaten aus zellinternen Widerstandskomponenten und/oder Spannungs-, Kapazitäts-, Strom-, Leistungs-, und/oder elektrischen Ladungsmessdaten, basierend auf wenigstens einem KI-Modell ausgewertet werden.

10. Verfahren nach einem der vorhergehenden Verfahrensansprüche, wobei wenigstens ein Messwert mit einem Prozessparameter (P_{SB}) für einen/den chemischen Sauerstoffbedarf (CSB) korreliert wird; und/oder wobei wenigstens ein Messwert mit einem Prozessparameter (P_{SB}), insbesondere CSB, basierend auf dem biologischen Sauerstoffbedarf (BSB) korreliert wird; und/oder wenigstens ein Messwert mit einem Prozessparameter (P_{SB}), insbesondere CSB, basierend auf Stofffraktionen flüchtiger Fettsäuren, VFA, korreliert wird; und/oder wobei die Kohlenstoffverbrauchsrate (CCR) ermittelt wird, wobei die Kohlenstoffverbrauchsrate als insbesondere im Rahmen einer Steuerung/Regelung nutzbarer Prozessparameter ausgegeben wird und/oder weiter ausgewertet wird zum Ermitteln eines Prozessparameters (P_{SB}) für den chemischen Sauerstoffbedarf (CSB) oder des CSB selbst; und/oder wobei die Kohlenstoffverbrauchsrate basierend auf mittels der messtechnischen mikrobiellen elektrochemischen Technologieeinheit (11) der messtechnischen Vorrichtung (10) generiertem Biostrom ermittelt wird, insbesondere unter Bezugnahme auf ein Elektronen-/Stromäquivalent des CSB und der Ertragsrate 0,243.

11. Verfahren nach einem der vorhergehenden Verfahrensansprüche, wobei das Auswerten von Messdaten basierend auf maschinellem Lernen erfolgt, wobei Zellparameter zum Bestimmen des chemischen und/oder biologischen Sauerstoffbedarfs und/oder der Kohlenstoffverbrauchsrate und/oder von Stofffraktionen flüchtiger Fettsäuren ausgewertet werden, welche bevorzugt basierend auf Modulation, insbesondere Pulsmodulation des externen elektrischen Lastwiderstandes von aus über einen Auswertungszeitraum von mindestens 30 Tagen erhobenen Messdaten betreffend zellinterne Widerstandskomponenten und/oder Spannungs-, Kapazitäts-, Strom-, Leistungs-, und/oder elektrischen Ladungsmessdaten generiert sind/wurden.

12. Verfahren zum Steuern/Regeln wenigstens einer Stellgröße eines Wasseraufbereitungsprozesses basierend auf Messdaten oder Prozessparametern bereitgestellt mittels eines Verfahrens nach einem der vorhergehenden Verfahrensansprüche.

13. Messtechnische Vorrichtung (10) eingerichtet zum Generieren von wenigstens einem energetischen/elektrochemischen Parameter aus über einen Auswertungszeitraum von mindestens 30 Tagen erhobenen Messdaten basierend auf bioelektrochemischen Prozessen, aufweisend wenigstens eine messtechnische mikrobielle elektrochemische Technologieeinheit (11) mit einem kathodischen Bereich (11.1) und mit wenigstens einer zwischen kathodischem Bereich (11.1) und zu untersuchendem Fluid angeordneten Keramik (12.2) und eingerichtet zum Korrelieren wenigstens eines Messwerts mit wenigstens einem Prozessparameter (P_{SB}) basierend auf verarbeiteten Messdaten; insbesondere messtechnische Vorrichtung nach einem der Ansprüche 1 bis 5; wobei die messtechnische Vorrichtung hergestellt ist durch Einfassen der Keramik zwischen einer mit Druck nach radial außen implementierten inneren Stützstruktur und einer mit Zugspannung nach radial innen implementierten äußeren Stützstruktur, insbesondere mit der äußeren Stützstruktur in Funktion als Stromkollektor, wahlweise mit zumindest einer der inneren und äußeren Stützstrukturen als integraler Bestandteil von Kathode und/oder Anode, wobei die Keramik vorteilhaft in Ausgestaltung als zumindest abschnittsweise zylindrischer Körper, insbesondere Rohr, mit geometrisch zumindest annähernd zylindrischem Aufbau zwischen Anode und Kathode bereitgestellt ist/wird, wobei durch das Einfassen bevorzugt eine dreikomponentige Membran-Elektroden-Baugruppe mit Keramik und Elektroden jeweils in vollumfänglicher mantelflächiger Anlage gebildet wird.

14. Computerprogrammprodukt umfassend Befehle, die bei Ausführung des Computerprogrammproduktes auf einem Computer oder einer messtechnischen Vorrichtung oder einem messtechnischen System diese/n/s dazu veranlassen, Schritte zum Steuern/Regeln eines Verfahren nach einem der vorhergehenden Verfahrensansprüche auf dem Computer auszuführen, insbesondere Computerprogrammprodukt eingerichtet zum Generieren wenigstens eines energetischen/elektrochemischen Parameters aus Messdaten erhoben über einen Auswertungszeitraum von mindestens 30 Tagen mittels wenigstens einer wenigstens eine zwischen kathodischem Bereich (11.1) und zu untersuchendem Fluid angeordnete und wirksame Keramik (12.2) aufweisenden messtechnischen mikrobiellen elektrochemischen Technologieeinheit (11), wobei wenigstens ein Messwert mit wenigstens einem Prozessparameter (P_{SB}) korreliert wird, insbesondere Messdaten aus zellinternen Widerstandskomponenten und/oder Spannungs-, Kapazitäts-, Strom-, Leistungs-, und/oder elektrischen Ladungsmessdaten; insbesondere basierend auf Messdaten erfasst/ermittelt mittels einer messtechnischen Vorrichtung (10) nach einem der Ansprüche 1 bis 5 und/oder mittels eines Verfahrens nach einem der vorhergehenden Verfahrensansprüche.

15. Verwendung einer messtechnischen mikrobiellen elektrochemischen Technologieeinheit (11) einer messtechnischen Vorrichtung (10) zum Generieren von wenigstens einem energetischen/elektrochemischen Parameter beim Erheben von Messdaten über einen Auswertungszeitraum von mindestens 30 Tagen, wobei die messtechnische mikrobielle elektrochemische Technologieeinheit (11) wenigstens eine zwischen kathodischem Bereich (12b) und zu untersuchendem Fluid angeordnete und wirksame Keramik (12.2) aufweist, wobei wenigstens ein Messwert mit wenigstens einem Prozessparameter (P_{SB}) basierend auf den erhobenen Messdaten korreliert wird, insbesondere betreffend den chemischen/biologischen Sauerstoffbedarf (CSB/BSB) und/oder eine Kohlenstoffverbrauchsrate und/oder Stofffraktionen flüchtiger Fettsäuren, wobei das Generieren des wenigstens einen energetischen/elektrochemischen Parameters aus Messdaten betreffend zellinterne Widerstandskomponenten und/oder Spannungs-, Kapazitäts-, Strom-, Leistungs-, und/oder elektrischen Ladungsmessdaten erfolgt, wobei das Generieren des wenigstens einen energetischen/elektrochemischen Parameters basierend auf Modulation erfolgt, insbesondere Pulsmodulation des externen elektrischen Lastwiderstandes; wobei die messtechnische mikrobielle elektrochemische Technologieeinheit (11) verwendet wird bei der Implementierung wenigstens einer Sensorik aus der folgenden Gruppe: Bodensensorik, Gewässersensorik, Abwassersensorik im Allgemeinen, Aquaristiksensorik, Kanalsensorik, Sensorik im Zulauf stromauf von Kläranlagen oder einzelner Prozessstufen, Sensorik im Ablauf stromab von Kläranlagen insbesondere im Bereich so genannter Vorfluter oder stromab einzelner Prozessstufen, Sensorik in wenigstens einer prozessualen Belebungsstufe eines Wasseraufbereitungsprozesses, Sensorik zur Detektion photosynthetischer Prozesse, Sensorik für Industrie- und Pflanzenkläranlagen, Grundwassersensorik.
